# EUROPEAN PATENT APPLICATION

(11) **EP 2 322 548 A1**
(43) Date of publication of application: **18.05.2011**
(21) Application number: 10013139.0
(22) Date of filing: 16.12.2003
(51) Int. Cl.: C07K 14/705, G01N 33/50, A01K 67/00, A01K 67/027

(54) **NPC1L1 (NPC) and methods of use thereof**

(30) Priority: 17.07.2003 US 621758; 22.08.2003 US 646301; 16.09.2003 US 663208
(62) Divisional of application: 03818238.2
(71) Applicant: Schering Corporation, Kenilworth, NJ 07033-0530 (US)
(72) Inventor: Altmann, Scott W., Fanwood, NJ 07023 (US); Murgolo, Nicholas J., 07946 Millington, NJ (US); Wang, Lu Quan, East Brunswick, NJ 08816 (US); Graziano, Michael P., Scotch Plains, NJ 07076 (US)
(74) Representative: UEXKÜLL & STOLBERG

(57) **Abstract**

The present invention provides human, rat and mouse NPC1L1 polypeptides and polynucleotides encoding the polypeptides. Also provided are methods for detecting agonists and antagonists of NPC1L1. Inhibitors of NPC1L1 can be used for inhibiting intestinal cholesterol absorption in a subject.

## Description

This application is a continuation-in-part of U.S. Patent Application No. 10/663,208; filed September 16, 2003 which is a continuation-in-part of U.S. Patent Application No. 10/646,301; filed August 22, 2003 which is a continuation-in-part of U.S. Patent Application No. 10/621,758; filed July 17, 2003 which claims the benefit of U.S. Provisional Patent Application No. 60/397,442; filed July 19, 2002 each of which is herein incorporated by reference in its entirety.

### FIELD OF THE INVENTION

The present invention includes NPC1L1 polypeptides and polynucleotides which encode the polypeptides along with methods of use thereof.

### BACKGROUND OF THE INVENTION

A factor leading to development of vascular disease, a leading cause of death in industrialized nations, is elevated serum cholesterol. It is estimated that 19% of Americans between the ages of 20 and 74 years of age have high serum cholesterol. The most prevalent form of vascular disease is arteriosclerosis, a condition associated with the thickening and hardening of the arterial wall. Arteriosclerosis of the large vessels is referred to as atherosclerosis. Atherosclerosis is the predominant underlying factor in vascular disorders such as coronary artery disease, aortic aneurysm, arterial disease of the lower extremities and cerebrovascular disease.

Cholesteryl esters are a major component of atherosclerotic lesions and the major storage form of cholesterol in arterial wall cells. Formation of cholesteryl esters is also a step in the intestinal absorption of dietary cholesterol. Thus, inhibition of cholesteryl ester formation and reduction of serum cholesterol can inhibit the progression of atherosclerotic lesion formation, decrease the accumulation of cholesteryl esters in the arterial wall, and block the intestinal absorption of dietary cholesterol.

The regulation of whole-body cholesterol homeostasis in mammals and animals involves the regulation of intestinal cholesterol absorption, cellular cholesterol trafficking, dietary cholesterol and modulation of cholesterol biosynthesis, bile acid biosynthesis, steroid biosynthesis and the catabolism of the cholesterol-containing plasma lipoproteins. Regulation of intestinal cholesterol absorption has proven to be an effective means by which to regulate serum cholesterol levels. For example, a cholesterol absorption inhibitor, ezetimibe ( 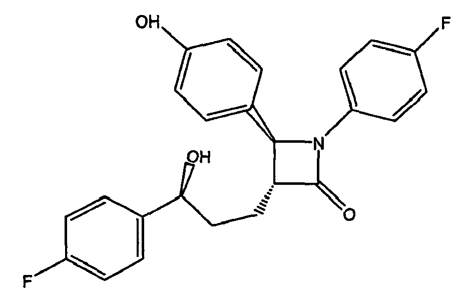 ), has been shown to be effective in this regard. A pharmaceutical composition containing ezetimibe is commercially available from Merck/Schering-Plough Pharmaceuticals, Inc. under the tradename Zetia®. Identification of a gene target through which ezetimibe acts is important to understanding the process of cholesterol absorption and to the development of other, novel absorption inhibitors. The present invention addresses this need by providing a rat and a mouse homologue of human NPC1L1 (also known as NPC3; Genbank Accession No. AF192522; Davies, et al., (2000) Genomics 65(2):137-45 and Ioannou, (2000) Mol. Genet. Metab.71(1-2):175-81), an ezetimibe target.

NPC1L1 is an N-glycosylated protein comprising a YQRL (SEQ ID NO: 38) motif (*i.e*., a *trans*-golgi network to plasma membrane transport signal; see Bos, et al., (1993) EMBO J. 12:2219-2228; Humphrey, et al., (1993) J. Cell. Biol. 120:1123-1135; Ponnambalam, et al., (1994) J. Cell. Biol. 125:253-268 and Rothman, et al., (1996) Science 272:227-234) which exhibits limited tissue distribution and gastrointestinal abundance. Also, the human *NPC1L1* promoter includes a Sterol Regulated Element Binding Protein 1 (SREBP1) binding consensus sequence (Athanikar, et al., (1998) Proc. Natl. Acad. Sci. USA 95:4935-4940; Ericsson, et al., (1996) Proc. Natl. Acad. Sci. USA 93:945-950; Metherall, et al., (1989) J. Biol. Chem. 264:15634-15641; Smith, et al., (1990) J. Biol. Chem. 265:2306-2310; Bennett, et al., (1999) J. Biol. Chem. 274:13025-13032 and Brown, et al., (1997) Cell 89:331-340). NPC1L1 has 42% amino acid sequence homology to human NPC1 (Genbank Accession No. AF002020), a receptor responsible for Niemann-Pick C1 disease (Carstea, et al., (1997) Science 277:228-231). Niemann-Pick C 1 disease is a rare genetic disorder in humans which results in accumulation of low density lipoprotein (LDL)-derived unesterified cholesterol in lysosomes (Pentchev, et al., (1994) Biochim. Biophys. Acta. 1225: 235-243 and Vanier, et al., (1991) Biochim. Biophys. Acta. 1096:328-337). In addition, cholesterol accumulates in the trans-golgi network of *npc1* cells, and relocation of cholesterol, to and from the plasma membrane, is delayed. NPC1 and NPC1L1 each possess 13 transmembrane spanning segments as well as a sterol-sensing domain (SSD). Several other proteins, including HMG-CoA Reductase (HMG-R), Patched (PTC) and Sterol Regulatory Element Binding Protein Cleavage-Activation Protein (SCAP), include an SSD which is involved in sensing cholesterol levels possibly by a mechanism which involves direct cholesterol binding (Gil, et al., (1985) Cell 41:249-258; Kumagai, et al., (1995) J. Biol. Chem. 270:19107-19113 and Hua, et al., (1996) Cell 87:415-426).

### SUMMARY OF THE INVENTION

The present invention includes an isolated polypeptide comprising 42 or more contiguous amino acids from an amino acid sequence selected from SEQ ID NOs: 2 and 12, preferably comprising the amino acid sequence selected from SEQ ID NOs: 2 and 12. The present invention also comprises an isolated polypeptide comprising the amino acid sequence of SEQ ID NO: 4. The invention also includes an isolated polynucleotide encoding a polypeptide of SEQ ID NO: 2, 4 or 12, preferably comprising a nucleotide sequence selected from SEQ ID NOs: 1, 3, 5-10, 11 and 13. A recombinant vector comprising a polynucleotide of the invention is also provided along with a host cell comprising the vector.

The present invention also provides an isolated antibody which specifically binds to or was raised against NPC1L1 (*e.g.,* rat NPC1L1, mouse NPC1L1 or human NPC1L1) or any antigenic fragment thereof, preferably rat NPC1L1, more preferably a polypeptide comprising an amino acid sequence selected from SEQ ID NO: 39-42. Preferably, the antibody is an isolated polyclonal or monoclonal antibody. In one embodiment, the antibody is obtained from a rabbit.

The present invention also includes a method for making an NPC1L1 polypeptide of the invention comprising culturing a host cell of the invention under conditions in which the nucleic acid in the cell which encodes the NPC1L1 polypeptide is expressed. Preferably, the method includes the step of isolating the polypeptide from the culture.

The present invention includes methods for identifying an agonist or antagonist of NPC1L1 comprising (a) contacting a host cell (*e.g.*, chinese hamster ovary (CHO) cell, a J774 cell, a macrophage cell or a Caco2 cell) expressing a polypeptide comprising the amino acid sequence of SEQ ID NO: 2 or SEQ ID NO: 4 or SEQ ID NO: 12 or a functional fragment thereof on a cell surface, in the presence of a known amount of a detectably labeled (*e.g.*, with ³H, ¹⁴C or ¹²⁵I) substituted azetidinone (*e.g.*, ezetimibe), with a sample to be tested for the presence of an NPC1L1 agonist or antagonist; and (b) measuring the amount of detectably labeled substituted azetidinone (*e.g.*, ezetimibe) specifically bound to the polypeptide; wherein an NPC1L1 agonist or antagonist in the sample is identified by measuring substantially reduced binding of the detectably labeled substituted azetidinone (*e.g.*, ezetimibe) to the polypeptide, compared to what would be measured in the absence of such an agonist or antagonist.

Another method for identifying an agonist or antagonist of NPC1L1 is also provided. The method comprises (a) placing, in an aqueous suspension, a plurality of support particles, impregnated with a fluorescer (*e.g.,* yttrium silicate, yttrium oxide, diphenyloxazole and polyvinyltoluene), to which a host cell (*e.g.*, chinese hamster ovary (CHO) cell, a J774 cell, a macrophage cell or a Caco2 cell) expressing a polypeptide comprising the amino acid sequence of SEQ ID NO: 2 or SEQ ID NO: 4 or SEQ ID NO: 12 or a functional fragment thereof on a cell surface are attached; (b) adding, to the suspension, a radiolabeled (*e.g.,* with ³H, ¹⁴C or ¹²⁵I) substituted azetidinone (*e.g.*, ezetimibe) and a sample to be tested for the presence of an antagonist or agonist, wherein the radiolabel emits radiation energy capable of activating the fluorescer upon the binding of the substituted azetidinone (*e.g.*, ezetimibe) to the polypeptide to produce light energy, whereas radiolabeled substituted azetidinone (*e.g.*, ezetimibe) that does not bind to the polypeptide is, generally, too far removed from the support particles to enable the radioactive energy to activate the fluorescer; and (c) measuring the light energy emitted by the fluorescer in the suspension; wherein an NPC1L1 agonist or antagonist in the sample is identified by measuring substantially reduced light energy emission, compared to what would be measured in the absence of such an agonist or antagonist.

Also provided is a method for identifying an agonist or antagonist of NPC1L1 comprising (a) contacting a host cell (*e.g.*, chinese hamster ovary (CHO) cell, a J774 cell, a macrophage cell or a Caco2 cell) expressing an polypeptide comprising an amino acid sequence of SEQ ID NO: 2 or SEQ ID NO: 4 or SEQ ID NO: 12 or a functional fragment thereof on a cell surface with detectably labeled (*e.g.*, with ³H, ¹⁴C or ¹²⁵I) sterol (*e.g.*, cholesterol) or 5α-stanol and with a sample to be tested for the presence of an antagonist or agonist; and (b) measuring the amount of detectably labeled sterol (*e.g.*, cholesterol) or 5α-stanol in the cell; wherein an NPC1L1 antagonist in the sample is identified by measuring substantially reduced detectably labeled sterol (*e.g.*, cholesterol) or 5α-stanol within the host cell, compared to what would be measured in the absence of such an antagonist and wherein an NPC1L1 agonist in the sample is identified by measuring substantially increased detectably labeled sterol (*e.g.*, cholesterol) or 5α-stanol within the host cell, compared to what would be measured in the absence of such an agonist.

The present invention includes methods for inhibiting NPC1L1-mediated intestinal sterol (*e.g.*, cholesterol) or 5α-stanol uptake, in a subject, by administering a substance identified by the screening methods described herein to the subject. Such substances include compounds such as small molecule antagonists of NPC1L1 other than ezetimibe. Also contemplated are methods for antagonizing NPC1L1-mediated sterol (*e.g.*, cholesterol) or 5α-stanol absorption by administering anti-NPC1L1 antibodies. NPC1L1-mediated absorption of sterol (*e.g.*, cholesterol) or 5α-stanol can also be antagonized by any method which reduces expression of NPC1L1 in an organism. For example, NPC1L1 expression can be reduced by introduction of anti-sense *NPC1L1* mRNA into a cell of an organism or by genetic mutation of the *NPC1L1* gene in an organism (*e.g.*, by complete knockout, disruption, truncation or by introduction of one or more point mutations).

Also included in the present invention is a mutant transgenic mammal (*e.g.*, mouse, rat, dog, rabbit, pig, guinea pig, cat, horse), preferably a mouse comprising a homozygous or heterozygous mutation (*e.g.*, disruption, truncation, one or more point mutations, knock out) of endogenous, chromosomal *NPC1L1* wherein, preferably, the mouse does not produce any functional NPC1L1 protein. Preferably, the mutant mouse, lacking functional NPC1L1, exhibits a reduced level of intestinal sterol (*e.g.*, cholesterol) or 5α-stanol absorption and/or a reduced level of serum sterol (*e.g.*, cholesterol) or 5α-stanol and/or a reduced level of liver sterol (*e.g.*, cholesterol) or 5α-stanol as compared to that of a non-mutant mouse comprising functional NPC1L1. Preferably, in the mutant mouse chromosome, the region of *NPC1L1* (SEQ ID NO: 45) deleted is from nucleotide 790 to nucleotide 998. In one embodiment, *NPC1L1* (SEQ ID NO: 11) is deleted from nucleotide 767 to nucleotide 975. Any offspring or progeny of a parent *NPC1L1* mutant mouse (*i.e*., *npc1l1)* of the invention which has inherited an *npc1l1* mutant allele is also part of the present invention.

The scope of the present invention also includes a method for screening a sample for an intestinal sterol (*e.g.*, cholesterol) or 5α-stanol absorption antagonist comprising (a) feeding a sterol (*e.g.*, cholesterol) or 5α-stanol-containing substance (*e.g.*, comprising radiolabeled cholesterol, such as ¹⁴C-cholesterol or ³H-cholesterol) to a first and second mouse comprising a functional *NPC1L1* gene and to a third, mutant mouse lacking a functional *NPC1L1*; (b) administering the sample to the first mouse comprising a functional *NPC1L1* but not to the second mouse; (c) measuring the amount of sterol (*e.g.*, cholesterol) or 5α-stanol absorption in the intestine of said first, second and third mouse (*e.g.*, by measuring serum cholesterol); and (d) comparing the levels of intestinal sterol (*e.g.*, cholesterol) or 5α-stanol absorption in each mouse; wherein the sample is determined to contain the intestinal sterol (*e.g.*, cholesterol) or 5α-stanol absorption antagonist when the level of intestinal sterol (*e.g.*, cholesterol) or 5α-stanol absorption in the first mouse and third mouse are less than the amount of intestinal sterol (*e.g.,* cholesterol) or 5α-stanol absorption in the second mouse.

The present invention also encompasses a kit comprising (a) a substituted azetidinone (*e.g.*, ezetimibe) in a pharmaceutical dosage form (*e.g.*, a pill or tablet comprising 10mg substituted azetidinone (*e.g.*, ezetimibe)); and (b) information, for example in the form of an insert, indicating that NPC1L1 is a target of ezetimibe. The kit may also include simvastatin in a pharmaceutical dosage form (*e.g.*, a pill or tablet comprising 5 mg, 10 mg, 20 mg, 40 mg or 80mg simvastatin). The simvastatin in pharmaceutical dosage form and the ezetimibe in pharmaceutical dosage form can be associated in a single pill or tablet or in separate pills or tablets.

The present invention also provides any isolated mammalian cell (*e.g.*, isolated mouse cell, isolated rat cell or isolated human cell) which lacks a gene which encodes or can produce a functional NPC1L1 polypeptide. The isolated cell of can be isolated from a mutant mouse comprising a homozygous mutation of endogenous, chromosomal *NPC1L1* wherein the mouse does not produce any functional NPC1L1 protein. Further, the mutation can be in a gene, which when un-mutated, encodes an amino acid sequence of SEQ ID NO: 12 (*e.g.*, comprising a nucleotide sequence of SEQ ID NO: 11). The cell can be isolated or derived from duodenum, gall bladder, liver, small intestine or stomach tissue. The cell can be an enterocyte.

### DETAILED DESCRIEPTION OF THE EWENTION

The present invention includes an NPC1L1 polypeptide from rat, human and from mouse along with polynucleotides encoding the respective polypeptides. Preferably, the rat NPC1L1 polypeptide comprises the amino acid sequence set forth in SEQ ID NO: 2, the human NPC1L1 comprises the amino acid sequence set forth in SEQ ID NO: 4 and the mouse NPC1L1 polypeptide comprises the amino acid sequence set forth in SEQ ID NO.12. The rat *NPC1L1* polynucleotide of SEQ ID NO: 1 or 10 encodes the rat NPC1L1 polypeptide. The human *NPC1L1* polynucleotide of SEQ ID NO: 3 encodes the human NPC1L1 polypeptide. The mouse *NPC1L1* polynucleotide of SEQ ID NO:11 or 13 encodes the mouse NPC1L1 polypeptide.

The present invention includes any isolated polynucleotide or isolated polypeptide comprising a nucleotide or amino acid sequence referred to, below, in Table 1.

**Table 1. Polynucleotides and Polypeptides of the Invention.**

| **Polynucleotide or Polypeptide** | **Sequence Identifier** |
|---|---|
| Rat *NPC1L1* polynucleotide | SEQ ID NO: 1 |
| Rat NPC1L1 polypeptide | SEQ ID NO: 2 |
| Human *NPC1L1* polynucleotide | SEQ ID NO: 3 |
| Human NPC1L1 polypeptide | SEQ ID NO: 4 |
| Rat *NPC1L1* expressed sequence tag 603662080F1 (partial sequence) | SEQ ID NO: 5 |
| Rat *NPC1L1* expressed sequence tag 603665037F1 (partial sequence) | SEQ ID NO: 6 |
| Rat *NPC1L1* expressed sequence tag 604034587F1 (partial sequence) | SEQ ID NO: 7 |
| EST 603662080F1 with downstream sequences added | SEQ ID NO: 8 |
| EST 603662080F1 with upstream and downstream sequences added | SEQ ID NO: 9 |
| Back-translated polynucleotide sequence of rat NPC1L1 | SEQ ID NO: 10 |
| Mouse *NPC1L1* polynucleotide | SEQ ID NO: 11 |
| Mouse NPC1L1 polypeptide | SEQ ID NO: 12 |
| Back-translated polynucleotide sequence of mouse NPC1L1 | SEQ ID NO: 13 |
| Back-translated polynucleotide sequence of human NPC1L1 | SEQ ID NO: 51 |

A human NPC1L1 is also disclosed under Genbank Accession Number AF192522. As discussed below, the nucleotide sequence of the rat *NPC1L1* set forth in SEQ ID NO: 1 was obtained from an expressed sequence tag (EST) from a rat jejunum enterocyte cDNA library. SEQ ID NOs: 5-7 include partial nucleotide sequences of three independent cDNA clones. The downstream sequence of the SEQ ID NO: 5 EST (603662080F1) were determined; the sequencing data from these experiments are set forth in SEQ ID NO: 8. The upstream sequences were also determined; these data are set forth in SEQ ID NO: 9.

SEQ ID NOs: 43 and 44 are the nucleotide and amino acid sequence, respectively, of human NPC1L1 which is disclosed under Genbank Accession No.: AF192522 (see Davies, et al., (2000) Genomics 65(2):137-45).

SEQ ID NO: 45 is the nucleotide sequence of a mouse *NPC1L1* which is disclosed under Genbank Accession No. AK078947.

NPC1L1 mediates intestinal sterol (*e.g.*, cholesterol) or 5α-stanol absorption. Inhibition of NPC1L1 in a patient is a useful method for reducing intestinal sterol (*e.g.*, cholesterol) or 5α-stanol absorption and serum sterol (*e.g.*, cholesterol) or 5α-stanol in the patient. Reducing the level of intestinal sterol (*e.g.*, cholesterol) or 5α-stanol absorption and serum sterol (*e.g.,* cholesterol) or 5α-stanol in a patient is a useful way in which to treat or prevent the occurrence of atherosclerosis, particularly diet-induced atherosclerosis.

As used herein, the term "sterol" includes, but is not limited to, cholesterol and phytosterols (including, but not limited to, sitosterol, campesterol, stigmasterol and avenosterol)).

As used herein, the term "5α-stanol" includes, but is not limited to, cholestanol, 5α-campestanol and 5α-sitostanol.

### Molecular Biology

In accordance with the present invention there may be employed conventional molecular biology, microbiology, and recombinant DNA techniques within the skill of the art. Such techniques are explained fully in the literature. See, *e.g.*, Sambrook, Fritsch & Maniatis, Molecular Cloning: A Laboratory Manual, Second Edition (1989) Cold Spring Harbor Laboratory Press, Cold Spring Harbor, New York (herein "Sambrook, *et al.,* 1989"); DNA Cloning: A Practical Approach, Volumes I and II (D.N. Glover ed. 1985); Oligonucleotide Synthesis (M.J. Gait ed. 1984); Nucleic Acid Hybridization (B.D. Hames & S.J. Higgins eds. (1985)); Transcription And Translation (B.D. Hames & S.J. Higgins, eds. (1984)); Animal Cell Culture (R.I. Freshney, ed. (1986)); Immobilized Cells And Enzymes (IRL Press, (1986)); B. Perbal, A Practical Guide To Molecular Cloning (1984); F.M. Ausubel, et al. (eds.), Current Protocols in Molecular Biology, John Wiley & Sons, Inc. (1994).

The back-translated sequences of SEQ ID NO: 10 and of SEQ ID NO: 13 uses the single-letter code shown in Table 1 of Annex C, Appendix 2 of the PCT Administrative Instruction in the Manual of Patent Examination Procedure.

A "polynucleotide", "nucleic acid " or "nucleic acid molecule" may refer to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in single stranded form, doublestranded form or otherwise.

A "polynucleotide sequence", "nucleic acid sequence" or "nucleotide sequence" is a series of nucleotide bases (also called "nucleotides") in a nucleic acid, such as DNA or RNA, and means any chain of two or more nucleotides.

A "coding sequence" or a sequence "encoding" an expression product, such as a RNA, polypeptide, protein, or enzyme, is a nucleotide sequence that, when expressed, results in production of the product.

The term "gene" means a DNA sequence that codes for or corresponds to a particular sequence of ribonucleotides or amino acids which comprise all or part of one or more RNA molecules, proteins or enzymes, and may or may not include regulatory DNA sequences, such as promoter sequences, which determine, for example, the conditions under which the gene is expressed. Genes may be transcribed from DNA to RNA which may or may not be translated into an amino acid sequence.

The present invention includes nucleic acid fragments of any of SEQ ID NOs: 1, 5-11 or 13. A nucleic acid "fragment" includes at least about 30 (*e.g.*, 31, 32, 33, 34), preferably at least about 35 (e.g, 25, 26, 27, 28, 29, 30, 31, 32, 33 or 34), more preferably at least about 45 (*e.g.*, 35, 36, 37, 38, 39, 40, 41, 42, 43 or 44), and most preferably at least about 126 or more contiguous nucleotides (*e.g.*, 130, 131, 132, 133, 134, 135, 136, 137, 138, 139, 140, 150, 160, 170, 180, 190, 200, 300, 400, 500, 1000 or 1200) from any of SEQ ID NOs: 1, 5-11 or 13.

The present invention also includes nucleic acid fragments consisting of at least about 7 (*e.g.*, 9, 12, 17, 19), preferably at least about 20 (*e.g.*, 30, 40, 50, 60), more preferably about 70 (*e.g.*, 80, 90, 95), yet more preferably at least about 100 (*e.g.*, 105, 110, 114) and even more preferably at least about 115 (*e.g.*, 117, 119, 120, 122, 124, 125, 126) contiguous nucleotides from any of SEQ ID NOs: 1, 5-11 or 13.

As used herein, the term "oligonucleotide" refers to a nucleic acid, generally of no more than about 100 nucleotides (*e.g.*, 30, 40, 50, 60, 70, 80, or 90), that may be hybridizable to a genomic DNA molecule, a cDNA molecule, or an mRNA molecule encoding a gene, mRNA, cDNA, or other nucleic acid of interest. Oligonucleotides can be labeled, *e.g.*, by incorporation of ³²P-nucleotides, ³H-nucleotides, ¹⁴C-nucleotides, ³⁵S-nucleotides or nucleotides to which a label, such as biotin, has been covalently conjugated. In one embodiment, a labeled oligonucleotide can be used as a probe to detect the presence of a nucleic acid. In another embodiment, oligonucleotides (one or both of which may be labeled) can be used as PCR primers, either for cloning full length or a fragment of the gene, or to detect the presence of nucleic acids. Generally, oligonucleotides are prepared synthetically, preferably on a nucleic acid synthesizer.

A "protein sequence", "peptide sequence" or "polypeptide sequence" or "amino acid sequence" may refer to a series of two or more amino acids in a protein, peptide or polypeptide.

"Protein", "peptide" or "polypeptide" includes a contiguous string of two or more amino acids. Preferred peptides of the invention include those set forth in any of SEQ ID NOs: 2 or 12 as well as variants and fragments thereof. Such fragments preferably comprise at least about 10 (*eg.,* 11, 12, 13, 14, 15, 16, 17, 18 or 19), more preferably at least about 20 (*e.g.*, 21, 22, 23, 24, 25, 26, 27, 28, 29, 30, 35, 40), and yet more preferably at least about 42 (*e.g.*, 43, 44, 45, 46, 47, 48, 49, 50, 60, 70, 80, 90, 100, 110, 120 or 130) or more contiguous amino acid residues from any of SEQ ID NOs: 2 or 12.

The present invention also includes polypeptides, preferably antigenic polypeptides, consisting of at least about 7 (*e.g.*, 9, 10, 13, 15, 17, 19), preferably at least about 20 (*e.g.,* 22, 24, 26, 28), yet more preferably at least about 30 (*e.g.,* 32, 34, 36, 38) and even more preferably at least about 40 (*e.g.*, 41, 42) contiguous amino acids from any of SEQ ID NOs: 2 or 12.

The polypeptides of the invention can be produced by proteolytic cleavage of an intact peptide, by chemical synthesis or by the application of recombinant DNA technology and are not limited to polypeptides delineated by proteolytic cleavage sites. The polypeptides, either alone or cross-linked or conjugated to a carrier molecule to render them more immunogenic, are useful as antigens to elicit the production of antibodies and fragments thereof. The antibodies can be used, *e.g.*, in immunoassays for immunoaffinity purification or for inhibition of NPC1L1, *etc.*

The terms "isolated polynucleotide" or "isolated polypeptide" include a polynucleotide (*e.g.*, RNA or DNA molecule, or a mixed polymer) or a polypeptide, respectively, which are partially or fully separated from other components that are normally found in cells or in recombinant DNA expression systems. These components include, but are not limited to, cell membranes, cell walls, ribosomes, polymerases, serum components and extraneous genomic sequences.

An isolated polynucleotide or polypeptide will, preferably, be an essentially homogeneous composition of molecules but may contain some heterogeneity.

"Amplification" of DNA as used herein may denote the use ofpolymerase chain reaction (PCR) to increase the concentration of a particular DNA sequence within a mixture of DNA sequences. For a description of PCR see Saiki, et al., Science (1988) 239:487.

The term "host cell" includes any cell of any organism that is selected, modified, transfected, transformed, grown, or used or manipulated in any way, for the production of a substance by the cell, for example the expression or replication, by the cell, of a gene, a DNA or RNA sequence or a protein. Preferred host cells include chinese hamster ovary (CHO) cells, murine macrophage J774 cells or any other macrophage cell line and human intestinal epithelial Caco2 cells.

The nucleotide sequence of a nucleic acid may be determined by any method known in the art (*e.g.*, chemical sequencing or enzymatic sequencing). "Chemical sequencing" of DNA includes methods such as that of Maxam and Gilbert (1977) (Proc. Natl. Acad. Sci. USA 74:560), in which DNA is randomly cleaved using individual base-specific reactions. "Enzymatic sequencing" of DNA includes methods such as that of Sanger (Sanger, et al., (1977) Proc. Natl. Acad. Sci. USA 74:5463).

The nucleic acids herein may be flanked by natural regulatory (expression control) sequences, or may be associated with heterologous sequences, including promoters, internal ribosome entry sites (IRES) and other ribosome binding site sequences, enhancers, response elements, suppressors, signal sequences, polyadenylation sequences, introns, 5'- and 3'- non-coding regions, and the like.

In general, a "promoter" or "promoter sequence" is a DNA regulatory region capable of binding an RNA polymerase in a cell (*e.g.*, directly or through other promoter-bound proteins or substances) and initiating transcription of a coding sequence. A promoter sequence is, in general, bounded at its 3' terminus by the transcription initiation site and extends upstream (5' direction) to include the minimum number of bases or elements necessary to initiate transcription at any level. Within the promoter sequence may be found a transcription initiation site (conveniently defined, for example, by mapping with nuclease S1), as well as protein binding domains (consensus sequences) responsible for the binding of RNA polymerase. The promoter may be operably associated with other expression control sequences, including enhancer and repressor sequences or with a nucleic acid of the invention. Promoters which may be used to control gene expression include, but are not limited to, cytomegalovirus (CMV) promoter (U.S. Patent Nos. 5,385,839 and 5,168,062), the SV40 early promoter region (Benoist, et al., (1981) Nature 290:304-310), the promoter contained in the 3' long terminal repeat of Rous sarcoma virus (Yamamoto, et al., (1980) Cell 22:787-797), the herpes thymidine kinase promoter (Wagner, et al., (1981) Proc. Natl. Acad. Sci. USA 78:1441-1445), the regulatory sequences of the metallothionein gene (Brinster, et al., (1982) Nature 296:39-42); prokaryotic expression vectors such as the β-lactamase promoter (Villa-Komaroff et al., (1978) Proc. Natl. Acad. Sci. USA 75:3727-3731), or the tac promoter (DeBoer, et al., (1983) Proc. Natl. Acad. Sci. USA 80:21-25); see also "Useful proteins from recombinant bacteria" in Scientific American (1980) 242:74-94; and promoter elements from yeast or other fungi such as the *Gal 4* promoter, the *ADC* (alcohol dehydrogenase) promoter, PGK (phosphoglycerol kinase) promoter or the alkaline phosphatase promoter.

A coding sequence is "under the control of", "functionally associated with" or "operably associated with" transcriptional and translational control sequences in a cell when the sequences direct RNA polymerase mediated transcription of the coding sequence into RNA, preferably mRNA, which then may be RNA spliced (if it contains introns) and, optionally, translated into a protein encoded by the coding sequence.

The terms "express" and "expression" mean allowing or causing the information in a gene, RNA or DNA sequence to become manifest; for example, producing a protein by activating the cellular functions involved in transcription and translation of a corresponding gene. A DNA sequence is expressed in or by a cell to form an "expression product" such as an RNA (*e.g.*, mRNA) or a protein. The expression product itself may also be said to be "expressed" by the cell.

The term "transformation" means the introduction of a nucleic acid into a cell. The introduced gene or sequence may be called a "clone". A host cell that receives the introduced DNA or RNA has been "transformed" and is a "transformant" or a "clone." The DNA or RNA introduced to a host cell can come from any source, including cells of the same genus or species as the host cell, or from cells of a different genus or species.

The term "vector" includes a vehicle (*e.g.*, a plasmid) by which a DNA or RNA sequence can be introduced into a host cell, so as to transform the host and, optionally, promote expression and/or replication of the introduced sequence.

Vectors that can be used in this invention include plasmids, viruses, bacteriophage, integratable DNA fragments, and other vehicles that may facilitate introduction of the nucleic acids into the genome of the host. Plasmids are the most commonly used form of vector but all other forms of vectors which serve a similar function and which are, or become, known in the art are suitable for use herein. See, *e.g.,* Pouwels, et al., Cloning Vectors: A Laboratory Manual, 1985 and Supplements, Elsevier, N.Y., and Rodriguez et al. (eds.), Vectors: A Survey of Molecular Cloning Vectors and Their Uses, 1988, Buttersworth, Boston, MA.

The term "expression system" means a host cell and compatible vector which, under suitable conditions, can express a protein or nucleic acid which is carried by the vector and introduced to the host cell. Common expression systems include *E. coli* host cells and plasmid vectors, insect host cells and Baculovirus vectors, and mammalian host cells and vectors.

Expression of nucleic acids encoding the NPC1L1 polypeptides of this invention can be carried out by conventional methods in either prokaryotic or eukaryotic cells. Although *E. coli* host cells are employed most frequently in prokaryotic systems, many other bacteria, such as various strains of *Pseudomonas* and *Bacillus,* are known in the art and can be used as well. Suitable host cells for expressing nucleic acids encoding the NPC1L1 polypeptides include prokaryotes and higher eukaryotes. Prokaryotes include both gram-negative and gram-positive organisms, *e.g., E. coli* and *B. subtilis.* Higher eukaryotes include established tissue culture cell lines from animal cells, both of non-mammalian origin, *e.g.*, insect cells, and birds, and of mammalian origin, *e.g.*, human, primates, and rodents.

Prokaryotic host-vector systems include a wide variety of vectors for many different species. A representative vector for amplifying DNA is pBR322 or many of its derivatives (*e.g.*, pUC18 or 19). Vectors that can be used to express the NPC1L1 polypeptides include, but are not limited to, those containing the *lac* promoter (pUC-series); *trp* promoter (pBR322-trp); *Ipp* promoter (the pIN-series); lambda-pP or pR promoters (pOTS); or hybrid promoters such as *ptac* (pDR540). See Brosius et al., "Expression Vectors Employing Lambda-, trp-, lac-, and Ipp-derived Promoters", in Rodriguez and Denhardt (eds.) Vectors: A Survey of Molecular Cloning Vectors and Their Uses, 1988, Buttersworth, Boston, pp. 205-236. Many polypeptides can be expressed, at high levels, in an E.coli/T7 expression system as disclosed in U.S. Patent Nos. 4,952,496, 5,693,489 and 5,869,320 and in Davanloo, P., et al., (1984) Proc. Natl. Acad. Sci. USA 81: 2035-2039; Studier, F. W., et al., (1986) J. Mol. Biol. 189: 113-130; Rosenberg, A. H., et al., (1987) Gene 56: 125-135; and Dunn, J. J., et al., (1988) Gene 68: 259.

Higher eukaryotic tissue culture cells may also be used for the recombinant production of the NPC1L1 polypeptides of the invention. Although any higher eukaryotic tissue culture cell line might be used, including insect baculovirus expression systems, mammalian cells are preferred. Transformation or transfection and propagation of such cells have become a routine procedure. Examples of useful cell lines include HeLa cells, chinese hamster ovary (CHO) cell lines, J774 cells, Caco2 cells, baby rat kidney (BRK) cell lines, insect cell lines, bird cell lines, and monkey (COS) cell lines. Expression vectors for such cell lines usually include an origin of replication, a promoter, a translation initiation site, RNA splice sites (if genomic DNA is used), a polyadenylation site, and a transcription termination site. These vectors also, usually, contain a selection gene or amplification gene. Suitable expression vectors may be plasmids, viruses, or retroviruses carrying promoters derived, *e.g.*, from such sources as adenovirus, SV40, parvoviruses, vaccinia virus, or cytomegalovirus. Examples of expression vectors include pCR^{®}3.1, pCDNA1, pCD (Okayama, et al., (1985) Mol. Cell Biol. 5:1136), pMClneo Poly-A (Thomas, et al., (1987) Cell 51:503), pREP8, pSVSPORT and derivatives thereof, and baculovirus vectors such as pAC373 or pAC610. One embodiment of the invention includes membrane bound NPC1L1. In this embodiment, NPC1L1 can be expressed in the cell membrane of a eukaryotic cell and the membrane bound protein can be isolated from the cell by conventional methods which are known in the art.

The present invention also includes fusions which include the NPC1L1 polypeptides and *NPC1L1* polynucleotides of the present invention and a second polypeptide or polynucleotide moiety, which may be referred to as a "tag". The fusions of the present invention may comprise any of the polynucleotides or polypeptides set forth in Table 1 or any subsequence or fragment thereof (discussed above). The fused polypeptides of the invention may be conveniently constructed, for example, by insertion of a polynucleotide of the invention or fragment thereof into an expression vector. The fusions of the invention may include tags which facilitate purification or detection. Such tags include glutathione-S-transferase (GST), hexahistidine (His6) tags, maltose binding protein (MBP) tags, haemagglutinin (HA) tags, cellulose binding protein (CBP) tags and myc tags. Detectable tags such as ³²P, ³⁵S, ³H, ^{99m}Tc, ¹²³I, ¹¹¹In, ⁶⁸Ga, ¹⁸F, ¹²⁵I, ¹³¹I, ^{113m}In, ⁷⁶Br, ⁶⁷Ga, ^{99m}Tc, ¹²³I, ¹¹¹In and ⁶⁸Ga may also be used to label the polypeptides and polynucleotides of the invention. Methods for constructing and using such fusions are very conventional and well known in the art.

Modifications (*e.g.*, post-translational modifications) that occur in a polypeptide often will be a function of how it is made. For polypeptides made by expressing a cloned gene in a host, for instance, the nature and extent of the modifications, in large part, will be determined by the host cell's post-translational modification capacity and the modification signals present in the polypeptide amino acid sequence. For instance, as is well known, glycosylation often does not occur in bacterial hosts such as *E. coli.* Accordingly, when glycosylation is desired, a polypeptide can be expressed in a glycosylating host, generally a eukaryotic cell. Insect cells often carry out post-translational glycosylations which are similar to those of mammalian cells. For this reason, insect cell expression systems have been developed to express, efficiently, mammalian proteins having native patterns of glycosylation. An insect cell which may be used in this invention is any cell derived from an organism of the class *Insecta.* Preferably, the insect is *Spodoptera fruigiperda* (Sf9 or Sf21) or *Trichoplusia ni* (High 5). Examples of insect expression systems that can be used with the present invention, for example to produce NPC1L1 polypeptide, include Bac-To-Bac (Invitrogen Corporation, Carlsbad, CA) or Gateway (Invitrogen Corporation, Carlsbad, CA). If desired, deglycosylation enzymes can be used to remove carbohydrates attached during production in eukaryotic expression systems.

Other modifications may also include addition of aliphatic esters or amides to the polypeptide carboxyl terminus. The present invention also includes analogs of the NPC1L1 polypeptides which contain modifications, such as incorporation of unnatural amino acid residues, or phosphorylated amino acid residues such as phosphotyrosine, phosphoserine or phosphothreonine residues. Other potential modifications include sulfonation, biotinylation, or the addition of other moieties. For example, the NPC1L1 polypeptides of the invention may be appended with a polymer which increases the half-life of the peptide in the body of a subj ect. Preferred polymers include polyethylene glycol (PEG) (e.g., PEG with a molecular weight of 2 kDa, 5 kDa, 10 kDa, 12 kDa, 20 kDa, 30 kDa and 40 kDa), dextran and monomethoxypolyethylene glycol (mPEG).

The peptides of the invention may also be cyclized. Specifically, the amino- and carboxy-terminal residues of an NPC1L1 polypeptide or two internal residues of an NPC1L1 polypeptide of the invention can be fused to create a cyclized peptide. Methods for cyclizing peptides are conventional and very well known in the art; for example see Gurrath, et al., (1992) Eur. J. Biochem. 210:911-921.

The present invention contemplates any superficial or slight modification to the amino acid or nucleotide sequences which correspond to the polypeptides of the invention. In particular, the present invention contemplates sequence conservative variants of the nucleic acids which encode the polypeptides of the invention. "Sequence-conservative variants" of a polynucleotide sequence are those in which a change of one or more nucleotides in a given codon results in no alteration in the amino acid encoded at that position. Function-conservative variants of the polypeptides of the invention are also contemplated by the present invention. "Function-conservative variants" are those in which one or more amino acid residues in a protein or enzyme have been changed without altering the overall conformation and function of the polypeptide, including, but, by no means, limited to, replacement of an amino acid with one having similar properties. Amino acids with similar properties are well known in the art. For example, polar/hydrophilic amino acids which may be interchangeable include asparagine, glutamine, serine, cysteine, threonine, lysine, arginine, histidine, aspartic acid and glutamic acid; nonpolar/hydrophobic amino acids which may be interchangeable include glycine, alanine, valine, leucine, isoleucine, proline, tyrosine, phenylalanine, tryptophan and methionine; acidic amino acids which may be interchangeable include aspartic acid and glutamic acid and basic amino acids which may be interchangeable include histidine, lysine and arginine.

The present invention includes polynucleotides encoding rat, human or mouse NPC1L1 and fragments thereof as well as nucleic acids which hybridize to the polynucleotides. Preferably, the nucleic acids hybridize under low stringency conditions, more preferably under moderate stringency conditions and most preferably under high stringency conditions. A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook, *et al., supra*). The conditions of temperature and ionic strength determine the "stringency" of the hybridization. Typical low stringency hybridization conditions are 55°C, 5X SSC, 0.1% SDS, 0.25% milk, and no formamide at 42°C; or 30% formamide, 5X SSC, 0.5% SDS at 42°C. Typical, moderate stringency hybridization conditions are similar to the low stringency conditions except the hybridization is carried out in 40% formamide, with 5X or 6X SSC at 42°C. High stringency hybridization conditions are similar to low stringency conditions except the hybridization conditions are carried out in 50% formamide, 5X or 6X SSC and, optionally, at a higher temperature (*e.g.*, higher than 42°C: 57°C, 59 °C, 60 °C, 62 °C, 63 °C, 65°C or 68 °C). In general, SSC is 0.15M NaC1 and 0.015M Na-citrate. Hybridization requires that the two nucleic acids contain complementary sequences, although, depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the higher the stringency under which the nucleic acids may hybridize. For hybrids of greater than 100 nucleotides in length, equations for calculating the melting temperature have been derived (see Sambrook, *et al., supra,* 9.50-9.51). For hybridization with shorter nucleic acids, *i.e*., oligonucleotides, the position of mismatches becomes more important, and the length of the oligonucleotide determines its specificity (see Sambrook, *et al., supra*).

Also included in the present invention are polynucleotides comprising nucleotide sequences and polypeptides comprising amino acid sequences which are at least about 70% identical, preferably at least about 80% identical, more preferably at least about 90% identical and most preferably at least about 95% identical (*e.g.*, 95%, 96%, 97%, 98%, 99%, 100%) to the reference rat *NPC1L1* nucleotide (*e.g.*, any of SEQ ID NOs: 1 or 5-10) and amino acid sequences (*e.g.*, SEQ ID NO: 2), reference human *NPC1L1* nucleotide (*e.g.*, SEQ ID NO: 3) and amino acid sequences (*e.g.*, SEQ ID NO: 4) or the reference mouse *NPC1L1* nucleotide (*e.g.,* any of SEQ ID NOs: 11 or 13) and amino acids sequences (*e.g.*, SEQ ID NO: 12), when the comparison is performed by a BLAST algorithm wherein the parameters of the algorithm are selected to give the largest match between the respective sequences over the entire length of the respective reference sequences. Polypeptides comprising amino acid sequences which are at least about 70% similar, preferably at least about 80% similar, more preferably at least about 90% similar and most preferably at least about 95% similar (*e.g.*, 95%, 96%, 97%, 98%, 99%, 100%) to the reference rat NPC1L1 amino acid sequence of SEQ ID NO: 2, reference human NPC1L1 amino acid sequence of SEQ ID NO: 4 or the reference mouse NPC1L1 amino acid sequence of SEQ ID NO: 12, when the comparison is performed with a BLAST algorithm wherein the parameters of the algorithm are selected to give the largest match between the respective sequences over the entire length of the respective reference sequences, are also included in the present invention.

Sequence identity refers to exact matches between the nucleotides or amino acids of two sequences which are being compared. Sequence similarity refers to both exact matches between the amino acids of two polypeptides which are being compared in addition to matches between nonidentical, biochemically related amino acids. Biochemically related amino acids which share similar properties and may be interchangeable are discussed above.

The following references regarding the BLAST algorithm are herein incorporated by reference: **BLAST ALGORITHMS:** Altschul, S.F., et al., (1990) J. Mol. Biol. 215:403-410; Gish, W., et al., (1993) Nature Genet. 3:266-272; Madden, T.L., et al., (1996) Meth. Enzymol. 266:131-141; Altschul, S.F., et al., (1997) Nucleic Acids Res. 25:3389-3402; Zhang, J., et al., (1997) Genome Res. 7:649-656; Wootton, J.C., et al., (1993) Comput. Chem. 17:149-163; Hancock, J.M., et al., (1994) Comput. Appl. Biosci. 10:67-70; **ALIGNMENT SCORING SYSTEMS:** Dayhoff, M.O., et al., "A model of evolutionary change in proteins." in Atlas of Protein Sequence and Structure, (1978) vol. 5, suppl. 3. M.O. Dayhoff (ed.), pp. 345-352, Natl. Biomed. Res. Found., Washington, DC; Schwartz, RM., et al., "Matrices for detecting distant relationships." in Atlas of Protein Sequence and Structure, (1978) vol. 5, suppl. 3." M.O. Dayhoff (ed.), pp. 353-358, Natl. Biomed. Res. Found., Washington, DC; Altschul, S.F., (1991) J. Mol. Biol. 219:555-565; States, D.J., et al., (1991) Methods 3:66-70; Henikoff, S., et al., (1992) Proc. Natl. Acad. Sci. USA 89:10915-10919; Altschul, S.F., et al., (1993) J. Mol. Evol. 36:290-300; **ALIGNMENT STATISTICS:** Karlin, S., et al., (1990) Proc. Natl. Acad. Sci. USA 87:2264-2268; Karlin, S., et al., (1993) Proc. Natl. Acad. Sci. USA 90:5873-5877; Dembo, A., et al., (1994) Ann. Prob. 22:2022-2039; and Altschul, S.F. "Evaluating the statistical significance of multiple distinct local alignments." in Theoretical and Computational Methods in Genome Research (S. Suhai, ed.), (1997) pp. 1-14, Plenum, New York.

### Protein Purification

The proteins, polypeptides and antigenic fragments of this invention can be purified by standard methods, including, but not limited to, salt or alcohol precipitation, affinity chromatography (*e.g.*, used in conjunction with a purification tagged NPC1L1 polypeptide as discussed above), preparative disc-gel electrophoresis, isoelectric focusing, high pressure liquid chromatography (HPLC), reversed-phase HPLC, gel filtration, cation and anion exchange and partition chromatography, and countercurrent distribution. Such purification methods are well known in the art and are disclosed, *e.g.*, in *"*Guide to Protein Purification ", Methods in Enzymology, Vol. 182, M. Deutscher, Ed., 1990, Academic Press, New York, NY.

Purification steps can be followed by performance of assays for receptor binding activity as described below. Particularly where an NPC1L1 polypeptide is being isolated from a cellular or tissue source, it is preferable to include one or more inhibitors of proteolytic enzymes in the assay system, such as phenylmethanesulfonyl fluoride (PMSF), Pefabloc SC, pepstatin, leupeptin, chymostatin and EDTA.

### Antibody Molecules

Antigenic (including immunogenic) fragments of the NPC1L1 polypeptides of the invention are within the scope of the present invention (*e.g.*, 42 or more contiguous amino acids from SEQ ID NO: 2, 4 or 12). The antigenic peptides may be useful, *inter alia,* for preparing isolated antibody molecules which recognize NPC1L1. Isolated anti-NPC1L1 antibody molecules are useful NPC1L1 antagonists.

An antigen is any molecule that can bind specifically to an antibody. Some antigens cannot, by themselves, elicit antibody production. Those that can induce antibody production are immunogens.

Preferably, isolated anti-NPC1L1 antibodies recognize an antigenic peptide comprising an amino acid sequence selected from SEQ ID NOs: 39-42 (*e.g.*, an antigen derived from rat NPC1L1). More preferably, the antibody is A0715, A0716, A0717, A0718, A0867, A0868, A1801 or A1802.

The term "antibody molecule " includes, but is not limited to, antibodies and fragments (preferably antigen-binding fragments) thereof. The term includes monoclonal antibodies, polyclonal antibodies, bispecific antibodies, Fab antibody fragments, F(ab)₂ antibody fragments, Fv antibody fragments (*e.g.*, V_{H} or V_{L}), single chain Fv antibody fragments and dsFv antibody fragments. Furthermore, the antibody molecules of the invention may be fully human antibodies, mouse antibodies, rat antibodies, rabbit antibodies, goat antibodies, chicken antibodies, humanized antibodies or chimeric antibodies.

Although it is not always necessary, when NPC1L1 polypeptides are used as antigens to elicit antibody production in an immunologically competent host, smaller antigenic fragments are, preferably, first rendered more immunogenic by cross-linking or concatenation, or by coupling to an immunogenic carrier molecule (*i.e*., a macromolecule having the property of independently eliciting an immunological response in a host animal, such as diptheria toxin or tetanus). Cross-linking or conjugation to a carrier molecule may be required because small polypeptide fragments sometimes act as haptens (molecules which are capable of specifically binding to an antibody but incapable of eliciting antibody production, *i.e*., they are not immunogenic). Conjugation of such fragments to an immunogenic carrier molecule renders them more immunogenic through what is commonly known as the "carrier effect".

Carrier molecules include, *e.g.,* proteins and natural or synthetic polymeric compounds such as polypeptides, polysaccharides, lipopolysaccharides etc. Protein carrier molecules are especially preferred, including, but not limited to, keyhole limpet hemocyanin and mammalian serum proteins such as human or bovine gammaglobulin, human, bovine or rabbit serum albumin, or methylated or other derivatives of such proteins. Other protein carriers will be apparent to those skilled in the art. Preferably, the protein carrier will be foreign to the host animal in which antibodies against the fragments are to be elicited.

Covalent coupling to the carrier molecule can be achieved using methods well known in the art, the exact choice of which will be dictated by the nature of the carrier molecule used. When the immunogenic carrier molecule is a protein, the fragments of the invention can be coupled, *e.g.,* using water-soluble carbodiimides such as dicyclohexylcarbodiimide or glutaraldehyde.

Coupling agents, such as these, can also be used to cross-link the fragments to themselves without the use of a separate carrier molecule. Such cross-linking into aggregates can also increase immunogenicity. Immunogenicity can also be increased by the use of known adjuvants, alone or in combination with coupling or aggregation.

Adjuvants for the vaccination of animals include, but are not limited to, Adjuvant 65 (containing peanut oil, mannide monooleate and aluminum monostearate); Freund's complete or incomplete adjuvant; mineral gels such as aluminum hydroxide, aluminum phosphate and alum; surfactants such as hexadecylamine, octadecylamine, lysolecithin, dimethyldioctadecylammonium bromide, N,N-dioctadecyl-N',N'-bis(2-hydroxymethyl) propanediamine, methoxyhexadecylglycerol and pluronic polyols; polyanions such as pyran, dextran sulfate, poly IC, polyacrylic acid and carbopol; peptides such as muramyl dipeptide, dimethylglycine and tuftsin; and oil emulsions. The polypeptides could also be administered following incorporation into liposomes or other microcarriers.

Information concerning adjuvants and various aspects of immunoassays are disclosed, *e.g.,* in the series by P. Tijssen, Practice and Theory of Enzyme Immunoassays, 3rd Edition, 1987, Elsevier, New York. Other useful references covering methods for preparing polyclonal antisera include Microbiology, 1969, Hoeber Medical Division, Harper and Row; Landsteiner, Specificity of Serological Reactions, 1962, Dover Publications, New York, and Williams, et al., Methods in Immunology and Immunochemistry, Vol. 1, 1967, Academic Press, New York.

The anti-NPC1L1 antibody molecules of the invention preferably recognize human, mouse or rat NPC1L1; however, the present invention includes antibody molecules which recognize NPC1L1 from any species, preferably mammals (*e.g.*, cat, sheep or horse). The present invention also includes complexes comprising an NPC1L1 polypeptide of the invention and an anti-NPC1L1 antibody molecule. Such complexes can be made by simply contacting the antibody molecule with its cognate polypeptide.

Various methods may be used to make the antibody molecules of the invention. Human antibodies can be made, for example, by methods which are similar to those disclosed in U.S. Patent Nos. 5,625,126; 5,877,397; 6,255,458; 6,023,010 and 5,874,299.

Hybridoma cells which produce the monoclonal anti-NPC1L1 antibodies maybe produced by methods which are commonly known in the art. These methods include, but are not limited to, the hybridoma technique originally developed by Kohler, et al., (1975) (Nature 256:495-497), as well as the trioma technique (Hering, et al., (1988) Biomed. Biochim. Acta. 47:211-216 and Hagiwara, et al., (1993) Hum. Antibod. Hybridomas 4:15), the human B-cell hybridoma technique (Kozbor, et al., (1983) Immunology Today 4:72 and Cote, et al., (1983) Proc. Natl. Acad. Sci. U.S.A 80:2026-2030), and the EBV-hybridoma technique (Cole, et al., in Monoclonal Antibodies and Cancer Therapy, Alan R. Liss, Inc., pp. 77-96, 1985). ELISA may be used to determine if hybridoma cells are expressing anti-NPC1L1 antibodies.

The anti-NPC1L1 antibody molecules of the present invention may also be produced recombinantly (*e.g.*, in an *E.coli*/T7 expression system as discussed above). In this embodiment, nucleic acids encoding the antibody molecules of the invention (*e.g.,* V_{H} or V_{L}) may be inserted into a pet-based plasmid and expressed in the *E.coli*/T7 system. There are several methods by which to produce recombinant antibodies which are known in the art. An example of a method for recombinant production of antibodies is disclosed in U.S. Patent No. 4,816,567. See also Skerra, A., et al., (1988) Science 240:1038-1041; Better, M., et al., (1988) Science 240:1041-1043 and Bird, RE., et al., (1988) Science 242:423-426.

The term "monoclonal antibody," includes an antibody obtained from a population of substantially homogeneous antibodies, *i.e.,* the individual antibodies comprising the population are identical except for possible, naturally occurring mutations that may be present in minor amounts. Monoclonal antibodies are highly specific, being directed against a single antigenic site. Monoclonal antibodies are advantageous in that they may be synthesized by a hybridoma culture, essentially uncontaminated by other immunoglobulins. The modifier "monoclonal" indicates the character of the antibody as being obtained from a substantially homogeneous population of antibodies, and is not to be construed as requiring production of the antibody by any particular method. The monoclonal antibodies to be used in accordance with the present invention may be made by the hybridoma method as described by Kohler, et al., (1975) Nature 256:495.

The term "polyclonal antibody" includes an antibody which was produced among or in the presence of one or more other, non-identical antibodies. In general, polyclonal antibodies are produced from a B-lymphocyte in the presence of several other B-lymphocytes which produced non-identical antibodies. Typically, polyclonal antibodies are obtained directly from an immunized animal (*e.g.*, a rabbit).

A "bispecific antibody" comprises two different antigen binding regions which bind to distinct antigens. Bispecific antibodies, as well as methods of making and using the antibodies, are conventional and very well known in the art.

Anti-idiotypic antibodies or anti-idiotypes are antibodies directed against the antigen-combining region or variable region (called the idiotype) of another antibody molecule. As disclosed by Jerne (Jerne, N. K., (1974) Ann. Immunol. (Paris) 125c:373 and Jeme, N. K., et al., (1982) EMBO 1:234), immunization with an antibody molecule expressing a paratope (antigen-combining site) for a given antigen (*e.g.*, NPC1L1) will produce a group of anti-antibodies, some of which share, with the antigen, a complementary structure to the paratope. Immunization with a subpopulation of the anti-idiotypic antibodies will, in turn, produce a subpopulation of antibodies or immune cell subsets that are reactive to the initial antigen.

The term "fully human antibody" refers to an antibody which comprises human immunoglobulin sequences only. Similarly, "mouse antibody" refers to an antibody which comprises mouse immunoglobulin sequences only.

"Human/mouse chimeric antibody" refers to an antibody which comprises a mouse variable region (V_{H} and V_{L}) fused to a human constant region.

"Humanized" anti-NPC1L1 antibodies are also within the scope of the present invention. Humanized forms of non-human (*e.g.*, murine) antibodies are chimeric immunoglobulins, which contain minimal sequence derived from non-human immunoglobulin. For the most part, humanized antibodies are human immunoglobulins (recipient antibody) in which residues from a complementary determining region of the recipient are replaced by residues from a complementary determining region of a nonhuman species (donor antibody), such as mouse, rat or rabbit, having a desired specificity, affinity and capacity. In some instances, Fv framework residues of the human immunoglobulin are also replaced by corresponding non-human residues.

"Single-chain Fv" or "sFv" antibody fragments include the V_{H} and/or V_{L} domains of an antibody, wherein these domains are present in a single polypeptide chain. Generally, the sFv polypeptide further comprises a polypeptide linker between the V_{H} and V_{L} domains which enables the sFv to form the desired structure for antigen binding. Techniques described for the production of single chain antibodies (U.S. Patent Nos. 5,476,786; 5,132,405 and 4,946,778) can be adapted to produce anti-NPC1L1 specific, single chain antibodies. For a review of sFv see Pluckthun in The Pharmacology of Monoclonal Antibodies, vol. 113, Rosenburg and Moore eds. Springer-Verlag, N.Y., pp. 269-31 (1994).

"Disulfide stabilized Fv fragments" and "dsFv" include molecules having a variable heavy chain (V_{H}) and/or a variable light chain (V_{L}) which are linked by a disulfide bridge.

Antibody fragments within the scope of the present invention also include F(ab)₂ fragments which may be produced by enzymatic cleavage of an IgG by, for example, pepsin. Fab fragments may be produced by, for example, reduction of F(ab)₂ with dithiothreitol or mercaptoethylamine.

An F_{V} fragment is a V_{L} or V_{H} region.

Depending on the amino acid sequences of the constant domain of their heavy chains, immunoglobulins can be assigned to different classes. There are at least five major classes of immunoglobulins: IgA, IgD, IgE, IgG and IgM, and several of these may be further divided into subclasses (isotypes), *e.g*., IgG-1, IgG-2, IgG-3 and IgG-4; IgA-1 and IgA-2.

The anti-NPC1L1 antibody molecules of the invention may also be conjugated to a chemical moiety. The chemical moiety may be, *inter alia,* a polymer, a radionuclide or a cytotoxic factor. Preferably, the chemical moiety is a polymer which increases the half-life of the antibody molecule in the body of a subject. Suitable polymers include, but are by no means limited to, polyethylene glycol (PEG) (*e.g.*, PEG with a molecular weight of 2kDa, 5 kDa, 10 kDa, 12kDa, 20 kDa, 30kDa or 40kDa), dextran and monomethoxypolyethylene glycol (mPEG). Methods for producing PEGylated anti-IL8 antibodies which are described in U.S. Patent No. 6,133,426 can be applied to the production of PEGylated anti-NPC1L1 antibodies of the invention. Lee, et al., (1999) (Bioconj. Chem. 10:973-981) discloses PEG conjugated single-chain antibodies. Wen, et al., (2001) (Bioconj. Chem. 12:545-553) discloses conjugating antibodies with PEG which is attached to a radiometal chelator (diethylenetriaminpentaacetic acid (DTPA)).

The antibody molecules of the invention may also be conjugated with labels such as ⁹⁹TC,⁹⁰Y, ¹¹¹In, ³²P, ¹⁴C, ¹²⁵I, ³H, ¹³¹I, ¹¹C, ¹⁵O, ¹³N, ¹⁸F, ³⁵S, ⁵¹Cr, ⁵⁷To, ²²⁶Ra, ⁶Co, ⁵⁹Fe, ⁵⁷Se, ¹⁵²Eu, ⁶⁷CU, ²¹⁷Ci, ²¹¹At, ²¹²Pb, ⁴⁷Sc, ¹⁰⁹Pd, ²³⁴Th, ⁴⁰K, ¹⁵⁷Gd, ⁵⁵Mn, ⁵²Tr or ⁵⁶Fe.

The antibody molecules of the invention may also be conjugated with fluorescent or chemilluminescent labels, including fluorophores such as rare earth chelates, fluorescein and its derivatives, rhodamine and its derivatives, isothiocyanate, phycoerythrin, phycocyanin, allophycocyanin, o-phthaladehyde, fluorescamine, ¹⁵²Eu, dansyl, umbelliferone, luciferin, luminal label, isoluminal label, an aromatic acridinium ester label, an imidazole label, an acridimium salt label, an oxalate ester label, an aequorin label, 2,3-dihydrophthalazinediones, biotin/avidin, spin labels and stable free radicals.

The antibody molecules may also be conjugated to a cytotoxic factor such as diptheria toxin, *Pseudomonas aeruginosa* exotoxin A chain , ricin A chain, abrin A chain, modeccin A chain, alpha-sarcin, *Aleurites fordii* proteins and compounds (*e.g*., fatty acids), dianthin proteins, *Phytoiacca americana* proteins PAPI, PAPII, and PAP-S, *momordica charantia* inhibitor, curcin, crotin, *saponaria officinalis* inhibitors, mitogellin, restrictocin, phenomycin, and enomycin.

Any method known in the art for conjugating the antibody molecules of the invention to the various moieties may be employed, including those methods described by Hunter, et al., (1962) Nature 144:945; David, et al., (1974) Biochemistry 13:1014; Pain, et al., (1981) J. Immunol. Meth. 40:219; and Nygren, J., (1982) Histochem. and Cytochem. 30:407.

Methods for conjugating antibodies are conventional and very well known in the art.

### Screening Assays

The invention allows the discovery of selective agonists and antagonists of NPC1L1 (*e.g.*, SEQ ID NO: 2, 4 or 12) that may be useful in treatment and management of a variety of medical conditions including elevated serum sterol (*e.g.*, cholesterol) or 5α-stanol. Thus, NPC1L1 of this invention can be employed in screening systems to identify agonists or antagonists. Essentially, these systems provide methods for bringing together NPC1L1, an appropriate, known ligand or agonist or antagonist, including a sterol (*e.g.*, cholesterol, phytosterols (including, but not limited to, sitosterol, campesterol, stigmasterol and avenosterol)), a 5α-stanol (including but not limited to cholestanol, 5α-campestanol and 5α-sitostanol), a cholesterol oxidation product, a substituted azetidinone (*e.g.*, ezetimibe), BODIPY-ezetimibe (Altmann, et al., (2002) Biochim. Biophys. Acta 1580(1):77-93) or 4", 6"-bis[(2-fluorophenyl)carbamoyl]-beta-D-cellobiosyl derivative of 11-ketotigogenin as described in DeNinno, et al., (1997) (J. Med. Chem. 40(16):2547-54) (Merck; L-166,143) or any substituted azetidinone, and a sample to be tested for the presence of an NPC1L1 agonist or antagonist.

The term "specific" when used to describe binding of, for example, a ligand or antagonist of NPC1L1 in a screening assay is a term of art which refers to the extent by which the ligand or antagonist (*e.g.*, detectably labeled substituted azetidinone, detectably labeled ezetimibe, detectably labeled sterol (*e.g.*, cholesterol) or detectably labeled 5α-stanol) binds preferentially to NPC1L1 over that of other proteins in the assay system. For example, an antagonist or ligand of NPC1L1 binds specifically to NPC1L1 when the signal generated in the assay to indicate such binding exceeds, to any extent, a background signal in a negative control experiment wherein, for example, NPC1L1 or the antagonist or ligand is absent. Furthermore, "specific binding" includes binding of an antagonist or ligand either directly to NPC1L1 or indirectly, for example via another moiety, in a complex of which NPC1L1 is a part. The moiety to which an NPC1L1 ligand or antagonist binds can be another protein or a post-translational modification of NPC1L1 (*e.g.*, a lipid chain or a carbohydrate chain).

Non-limiting examples of suitable azetidinones include those disclosed in U.S. Patent Nos. RE37,721; 5,631,365; 5,767,115; 5,846,966; 5,688,990; 5,656,624; 5,624,920; 5,698,548 and 5,756,470 and U.S. Patent Application Publication No 2003/0105028-each of which is herein incorporated by reference in its entirety.

A convenient method by which to evaluate whether a sample contains an NPC1L1 agonist or antagonist is to determine whether the sample contains a substance which competes for binding between the known agonist or antagonist (*e.g.*, ezetimibe) and NPC1L1.

Ezetimibe can be prepared by a variety of methods well know to those skilled in the art, for example such as are disclosed in U.S. Patents Nos. 5,631,365, 5,767,115, 5,846,966, 6,207,822, U.S. Patent Application Publication No. 2002/0193607 and PCT Patent Application WO 93/02048, each of which is incorporated herein by reference in its entirety.

"Sample", "candidate compound" or "candidate substance" refers to a composition which is evaluated in a test or assay, for example, for the ability to bind to, agonize or antagonize NPC1L1 (*e.g.*, SEQ ID NO: 2, 4 or 12) or a functional fragment thereof. The composition may small molecules, peptides, nucleotides, polynucleotides, subatomic particles (*e.g.*, α particles, β particles) or antibodies.

Two basic types of screening systems that can be used include, a labeled-ligand binding assay (*e.g.*, direct binding assay or scintillation proximity assay (SPA)) and a "sterol (*e.g*., cholesterol) or 5α-stanol uptake" assay. A labeled ligand, for use in the binding assay, can be obtained by labeling a sterol (*e.g.*, cholesterol) or a 5α-stanol or a known NPC1L1 agonist or antagonist with a measurable group (*e.g.*, ¹²⁵I or ³H). Various labeled forms of sterols (*e.g*., cholesterol) or 5α-stanols are available commercially or can be generated using standard techniques (*e.g.*, Cholesterol- [1,2-³H(N)], Cholesterol-[1,2,6,7-³H(N)] or Cholesterol-[7-³H(N)]; American Radiolabeled Chemicals, Inc; St. Louis, MO). In a preferred embodiment, ezetimibe is fluorescently labeled with a BODIPY group (Altmann, et al., (2002) Biochim. Biophys. Acta 1580(1):77-93) or labeled with a detectable group such as ¹²⁵I or ³H.

***Direct Binding Assay.*** Typically, a given amount of NPC1L1 of the invention (*e.g.*, SEQ ID NO: 2, 4 or 12) or a complex including NPC1L1 is contacted with increasing amounts of labeled ligand or known antagonist or agonist (discussed above) and the amount of the bound, labeled ligand or known antagonist or agonist is measured after removing unbound, labeled ligand or known antagonist or agonist by washing. As the amount of the labeled ligand or known agonist or antagonist is increased, a point is eventually reached at which all receptor binding sites are occupied or saturated. Specific receptor binding of the labeled ligand or known agonist or antagonist is abolished by a large excess of unlabeled ligand or known agonist or antagonist.

Preferably, an assay system is used in which non-specific binding of the labeled ligand, or known antagonist or agonist to the receptor is minimal. Non-specific binding is typically less than 50%, preferably less than 15%, and more preferably less than 10% of the total binding of the labeled ligand or known antagonist or agonist.

A nucleic acid encoding an NPC1L1 polypeptide of the invention (*e.g.*, SEQ ID NO: 2, 4 or 12) can be transfected into an appropriate host cell, whereby the receptor will become incorporated into the membrane of the cell. A membrane fraction can then be isolated from the cell and used as a source of the receptor for assay. Alternatively, the whole cell expressing the receptor in the cell surface can be used in an assay. Preferably, specific binding of the labeled ligand or known antagonist or agonist to an untransfected/untransformed host cell or to a membrane fraction from an untransfected/untransformed host cell will be negligible.

In principle, a binding assay of the invention could be carried out using a soluble NPC1L1 polypeptide of the invention, *e.g.,* following production and refolding by standard methods from an *E. coli* expression system, and the resulting receptor-labeled ligand complex could be precipitated, *e.g.*, using an antibody against the receptor. The precipitate could then be washed and the amount of the bound, labeled ligand or antagonist or agonist could be measured.

In the basic binding assay, the method for identifying an NPC1L1 agonist or antagonist includes:
(a) contacting NPC1L1 (*e.g.*, SEQ ID NO: 2 or 4 or 12), a subsequence thereof or a complex including NPC1L1, in the presence of a known amount of labeled sterol (*e.g.*, cholesterol) or 5α-stanol or known antagonist or agonist (*e.g*., labeled ezetimibe or labeled L-166,143) with a sample to be tested for the presence of an NPC1L1 agonist or antagonist; and
(b) measuring the amount of labeled sterol (*e.g.*, cholesterol) or 5α-stanol or known antagonist or agonist directly or indirectly bound to NPC1L1.

An NPC1L1 antagonist or agonist in the sample is identified by measuring substantially reduced direct or indirect binding of the labeled sterol (*e.g.*, cholesterol) or 5α-stanol or known antagonist or agonist to NPC1L1, compared to what would be measured in the absence of such an antagonist or agonist. For example, reduced direct or indirect binding between [³H]-cholesterol and NPC1L1 in the presence of a sample might suggest that the sample contains a substance which is competing against [³H]-cholesterol for NPC1L1 binding.

This assay can include a control experiment lacking any NPC1L1-dependent ligand (*e.g.*, sterol such as cholesterol or 5α-stanol) binding. In this assay, for example, a whole cell or cell membrane lacking any functional NPC1L1, for example, a cell or membrane isolated or derived from a transgenic mutant *npc1l1⁻* mouse of the invention, is assayed for ligand binding. When screening a sample for the presence of an NPC1L1 antagonist, it is useful to compare the level of binding observed in the presence of a sample being tested with that of a control experiment, as described herein, which completely lacks NPC1L1-dependent binding. Ideally, though by no means necessarily, the level of binding seen in the presence of a sample containing an antagonist will be similar to that of the control experiment.

Alternatively, a sample can be tested directly for binding to NPC1L1 (*e.g.*, SEQ ID NO: 2, 4 or 12). A basic assay of this type may include the following steps:
(a) contacting NPC1L1 (*e.g.*, SEQ ID NO: 2 or 4 or 12), a subsequence thereof or a complex including NPC1L1 with a labeled candidate compound (*e.g.*, [³H]-ezetimibe); and
(b) detecting direct or indirect binding between the labeled candidate compound and NPC1L1.

Again, these experiment can be performed along with a control experiment wherein NPC1L1-dependent binding is completely lacking. For example, the assay can be performed using a whole cell or cell membrane lacking any functional NPC1L1 (*e.g*., cell or cell membrane derived from a transgenic, mutant *npc1l1⁻* mouse as described herein).

A candidate compound which is found to bind to NPC1L1 may function as an agonist or antagonist of NPC1L1 (*e.g.*, by inhibition of sterol (*e.g.*, cholesterol) or 5α-stanol uptake).

***SPA Assay.*** NPC1L1 antagonists or agonists may also be measured using scintillation proximity assays (SPA). SPA assays are conventional and very well known in the art; see, for example, U.S. Patent No. 4,568,649. In SPA, the target of interest is immobilised to a small microsphere approximately 5 microns in diameter. The microsphere, typically, includes a solid scintillant core which has been coated with a polyhydroxy film, which in turn contains coupling molecules, which allow generic links for assay design. When a radioisotopically labeled molecule binds to the microsphere, the radioisotope is brought into close proximity to the scintillant and effective energy transfer from electrons emitted by the isotope will take place resulting in the emission of light. While the radioisotope remains in free solution, it is too distant from the scintillant and the electron will dissipate the energy into the aqueous medium and therefore remain undetected. Scintillation may be detected with a scintillation counter. In general, ³H and ¹²⁵I labels are well suited to SPA.

For the assay of receptor-mediated binding events, the lectin wheat germ agglutinin (WGA) may be used as the SPA bead coupling molecule (Amersham Biosciences; Piscataway, NJ). The WGA coupled bead captures glycosylated, cellular membranes and glycoproteins and has been used for a wide variety of receptor sources and cultured cell membranes. The receptor is immobilized onto the WGA-SPA bead and a signal is generated on binding of an isotopically labeled ligand. Other coupling molecules which may be useful for receptor binding SPA assays include poly-L-lysine and WGAlpolyethyleneimine (Amersham Biosciences; Piscataway, NJ). See, for example, Berry, J.A., et al., (1991) Cardiovascular Pharmacol. 17 (Suppl.7): S143-S145; Hoffman, R., et al., (1992) Anal. Biochem. 203: 70-75; Kienhus, et al., (1992) J. Receptor Research 12: 389-399; Jing, S., et al., (1992) Neuron 9: 1067-1079.

The scintillant contained in SPA beads may include, for example, yttrium silicate (YSi), yttrium oxide (YOx), diphenyloxazole or polyvinyltoluene (PVT) which acts as a solid solvent for diphenylanthracine (DPA).

SPA assays may be used to analyze whether a sample contains an NPC1L1 antagonist or agonist. In these assays, a host cell which expresses NPC1L1 (*e.g.*, SEQ ID NO: 2 or 4 or 12) on the cell surface or a membrane fraction thereof is incubated with and captured by SPA beads (*e.g.*, WGA coated YOx beads or WGA coated YSi beads). The beads bearing the NPC1L1 are incubated with labeled, known ligand or agonist or antagonist (*e.g.*, ³H-cholesterol, ³H-ezefimibe or ¹²⁵I-ezetimibe). The assay mixture further includes either the sample to be tested or a blank (*e.g.*, water). After an optional incubation, scintillation is measured using a scintillation counter. An NPC1L1 agonist or antagonist may be identified in the sample by measuring substantially reduced fluorescence, compared to what would be measured in the absence of such agonist or antagonist (blank). Measuring substantially reduced fluorescence may suggest that the sample contains a substance which competes for direct or indirect NPC1L1 binding with the known ligand, agonist or antagonist.

Alternatively, a sample may be identified as an antagonist or agonist of NPC1L1 by directly detecting binding in a SPA assay. In this assay, a labeled version of a candidate compound to be tested may be put in contact with the host cell expressing NPC1L1 or a membrane fraction thereof which is bound to the SPA bead. Fluorescence may then be assayed to detect the presence of a complex between the labeled candidate compound and the host cell or membrane fraction expressing NPC1L1 or a complex including IVPC1L1. A candidate compound which binds directly or indirectly to NPC1L1 may possess NPC1L1 agonistic or antagonistic activity.

SPA Assays can also be performed along with a control experiment lacking any NPC1L1-dependent binding. The control experiment can be performed, for example, with a cell or cell membrane lacking any functional NPC1L1 (*e.g.*, cell or cell membrane derived from a transgenic, mutant *npc1l1⁻* mouse as described herein). When the control experiment is performed, the level of binding observed in the presence of sample being tested for the presence of an antagonist can be compared with that observed in the control experiment.

Host cells expressing NPC1L1 may be prepared by transforming or transfecting a nucleic acid encoding an NPC1L1 of the invention into an appropriate host cell, whereby the receptor becomes incorporated into the membrane of the cell. A membrane fraction can then be isolated from the cell and used as a source of the receptor for assay. Alternatively, the whole cell expressing the receptor on the cell surface can be used in an assay. Preferably, specific binding of the labeled ligand or known antagonist or agonist to an untransfected/untransformed host cell or membrane fraction from an untransfected/untransformed host cell will be negligible. Preferred host cells include Chinese Hamster Ovary (CHO) cells, murine macrophage J774 cells or any other macrophage cell line and human intestinal epithelial Caco2 cells.

***steroV5a-stanol Uptake Assay.*** Assays may also be performed to determine if a sample can agonize or antagonize NPC1L1 mediated sterol (*e.g.*, cholesterol) or 5α-stanol uptake. In these assays, a host cell expressing NPC1L1 (*e.g.*, SEQ ID NO: 2 or 4 or 12) on the cell surface (discussed above) can be contacted with detectably labeled sterol (*e.g*.*,* ³H-cholesterol or ¹²⁵I-cholesteml)) or 5α-stanol along with either a sample or a blank. After an optional incubation, the cells can be washed to remove unabsorbed sterol or 5α-stanol. Sterol or 5a-stanol uptake can be determined by detecting the presence of labeled sterol or 5α-stanol in the host cells. For example, assayed cells or lysates or fraction thereof (*e.g.*, fractions resolved by thin-layer chromatography) can be contacted with a liquid scintillant and scintillation can be measured using a scintillation counter.

In these assays, an NPC1L1 antagonist in the sample may be identified by measuring substantially reduced uptake of labeled sterol (*e.g.*, ³H-cholesterol) or 5α-stanol, compared to what would be measured in the absence of such an antagonist and an agonist may be identified by measuring substantially increased uptake of labeled sterol (*e.g.*, ³H-cholesterol) or 5α-stanol, compared to what would be measured in the absence of such an agonist.

Uptake assays can also be performed along with a control experiment lacking any NPC1L1-dependent uptake. The control experiment can be performed, for example, with a cell lacking any functional NPC1L1 (*e.g.*, cell derived from a transgenic, mutant *npc1l1⁻* mouse as described herein). When the control experiment is performed, the level of uptake observed in the presence of sample being tested for the presence of an antagonist can be compared with that observed in the control experiment.

***Mouse Assay.*** The present invention comprises a mutant, transgenic, mouse which lacks any functional NPC1L1. This mouse may serve as a convenient control experiment in screening assays for identifying inhibitors of intestinal sterol (*e.g.*, cholesterol) or 5α-stanol absorption, preferably inhibitors of NPC1L1. Preferably, a mouse assay of the present invention would comprise the following steps:
(a) feeding a sterol (*e.g.*, cholesterol) or 5α-stanol-containing substance (*e.g.,* comprising radiolabeled cholesterol, such as ¹⁴C-cholesterol or ³H-cholesterol) to a first and second mouse comprising a functional *NPC1L1* gene and to a third, mutant mouse lacking a functional *NPC1L1*;
   The sterol (*e.g.*, cholesterol) or 5α-stanol containing substance preferably contains labeled cholesterol, such as a radiolabeled cholesterol, for example, ³H or ¹⁴C labeled cholesterol. The sterol (*e.g.*, cholesterol) or 5α-stanol containing substance may also include cold, unlabeled sterol (*e.g.*, cholesterol) or 5α-stanol such as in corn oil.
   In these assays, the third *npc1l1* mutant mouse serves as a (+)-control experiment which exhibits low levels of intestinal sterol (*e.g.*, cholesterol) or 5α-stanol absorption and the second mouse serves as a (-)-control experiment which exhibits normal, uninhibited levels of intestinal sterol (*e.g.*, cholesterol) or 5α-stanol absorption. The second mouse is not administered the sample to be tested for an NPC1L1 antagonist. The first mouse is the experiment.
(b) administering the sample to the first mouse comprising a functional *NPC1L1* but not to the second mouse;
(c) measuring the amount of sterol (*e.g.*, cholesterol) or 5α-stanol absorption in the intestine of said first, second and third mouse;
   Intestinal sterol (*e.g.*, cholesterol) or 5α-stanol absorption maybe measured by any method known in the art. For example, the level intestinal absorption can be assayed by measuring the level of serum sterol (*e.g.*, cholesterol) or 5α-stanol.
(d) comparing the levels of intestinal sterol (*e.g.*, cholesterol) or 5α-stanol absorption in each mouse;
   wherein the sample is determined to contain the intestinal sterol (*e.g.*, cholesterol) or 5α-stanol absorption antagonist when the level of intestinal sterol (*e.g.*, cholesterol) or 5α-stanol absorption in the first mouse and in the third mouse are less than the amount of intestinal sterol (*e.g.*, cholesterol) or 5α-stanol absorption in the second mouse.

Preferably, if the sample contains an intestinal sterol (*e.g.*, cholesterol) or 5α-stanol absorption inhibitor (*e.g.*, an NPC1L1 inhibitor), the level of sterol (*e.g.,* cholesterol) or 5α-stanol absorption in the first mouse will be similar to that of the third, *npc1l1* mutant mouse.

An alternative, (+)-control experiment which may be used in these screening assays is a mouse comprising functional NPC1L1 which is administered a known antagonist of NPC1L1, such as ezetimibe.

### Pharmaceutical Compositions

NPC1L1 agonists and antagonists discovered, for example, by the screening methods described above may be used therapeutically (*e.g.*, in a pharmaceutical composition) to stimulate or block the activity of NPC1L1 and, thereby, to treat any medical condition caused or mediated by NPC1L1. In addition, the antibody molecules of the invention may also be used therapeutically (*e.g.*, in a pharmaceutical composition) to bind NPC1L1 and, thereby, block the ability of NPC1L1 to bind a sterol (*e.g.*, cholesterol) or 5α-stanol. Blocking the binding of a sterol (*e.g.*, cholesterol) or 5α-stanol would prevent absorption of the molecule (*e.g.*, by intestinal cells such as enterocytes). Blocking absorption of sterol (*e.g.*, cholesterol) or 5α-stanol would be a useful way to lower serum sterol (*e.g.*, cholesterol) or 5α-stanol levels in a subject and, thereby, reduce the incidence of, for example, hyperlipidemia, atherosclerosis, coronary heart disease, stroke or arteriosclerosis.

The term "subject" or "patient" includes any organism, preferably animals, more preferably mammals (*e.g.*, mice, rats, rabbits, dogs, horses, primates, cats) and most preferably humans.

The term "pharmaceutical composition" refers to a composition including an active ingredient and a pharmaceutically acceptable carrier and/or adjuvant.

Although the compositions of this invention could be administered in simple solution, they are more typically used in combination with other materials such as carriers, preferably pharmaceutically acceptable carriers. Useful, pharmaceutically acceptable carriers can be any compatible, non-toxic substances suitable for delivering the compositions of the invention to a subject. Sterile water, alcohol, fats, waxes, and inert solids may be included in a pharmaceutically acceptable carrier. Pharmaceutically acceptable adjuvants (buffering agents, dispersing agents) may also be incorporated into the pharmaceutical composition.

Preferably, the pharmaceutical compositions of the invention are in the form of a pill or capsule. Methods for formulating pills and capsules are very well known in the art. For example, for oral administration in the form of tablets or capsules, the active drug component may be combined with any oral, non-toxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Suitable binders include starch, gelatin, natural sugars, corn sweeteners, natural and synthetic gums such as acacia, sodium alginate, carboxymethylcellulose, polyethylene glycol and waxes. Among the lubricants there may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate.

The pharmaceutical compositions of the invention may be administered in conjunction with a second pharmaceutical composition or substance. In preferred embodiments, the second composition includes a cholesterol-lowering drug. When a combination therapy is used, both compositions may be formulated into a single composition for simultaneous delivery or formulated separately into two or more compositions (*e.g.*, a kit).

The formulations may conveniently be presented in unit dosage form and may be prepared by any methods well known in the art of pharmacy. See, *e.g.,* Gilman et al. (eds.) (1990), The Pharmacological Bases of Therapeutics, 8th Ed., Pergamon Press; and Remington's Pharmaceutical Sciences, *supra,* Easton, Penn.; Avis et al. (eds.) (1993) Pharmaceutical Dosage Forms: Parenteral Medications Dekker, New York; Lieberman et al. (eds.) (1990) Pharmaceutical Dosage Forms: Tablets Dekker, New York; and Lieberman et al. (eds.) (1990), Pharmaceutical Dosage Forms: Disperse Systems Dekker, New York.

The dosage regimen involved in a therapeutic application may be determined by a physician, considering various factors which may modify the action of the therapeutic substance, *e.g.*, the condition, body weight, sex and diet of the patient, the severity of any infection, time of administration, and other clinical factors. Often, treatment dosages are titrated upward from a low level to optimize safety and efficacy. Dosages may be adjusted to account for the smaller molecular sizes and possibly decreased half-lives (clearance times) following administration.

An "effective amount" of an antagonist of the invention may be an amount that will detectably reduce the level of intestinal sterol (*e.g.*, cholesterol) or 5α-stanol absorption or detectably reduce the level of serum sterol (*e.g.*, cholesterol) or 5α-stanol in a subject administered the composition.

Typical protocols for the therapeutic administration of such substances are well known in the art. Pharmaceutical composition of the invention may be administered, for example, by any parenteral or non-parenteral route.

Pills and capsules of the invention can be administered orally. Injectable compositions can be administered with medical devices known in the art; for example, by injection with a hypodermic needle.

Injectable pharmaceutical compositions of the invention may also be administered with a needleless hypodermic injection device; such as the devices disclosed in U.S. Patent Nos. 5,399,163; 5,383,851; 5,312,335; 5,064,413; 4,941,880; 4,790,824 or 4,596,556.

### Anti-Sense

The present invention also encompasses anti-sense oligonucleotides capable of specifically hybridizing to mRNA encoding NPC1L1 (*e.g.*, any of SEQ ID NOs: 1,3,5-11 or 13) having an amino acid sequence defined by, for example, SEQ ID NO: 2 or 4 or 12 or a subsequence thereof so as to prevent translation of the mRNA. Additionally, this invention contemplates anti-sense oligonucleotides capable of specifically hybridizing to the genomic DNA molecule encoding NPC1L1, for example, having an amino acid sequence defined by SEQ ID NO: 2 or 4 or 12 or a subsequence thereof.

This invention further provides pharmaceutical compositions comprising (a) an amount of an oligonucleotide effective to reduce NPC1L1-mediated sterol (*e.g.*, cholesterol) or 5α-stanol absorption by passing through a cell membrane and binding specifically with mRNA encoding NPC1L1 in the cell so as to prevent its translation and (b) a pharmaceutically acceptable carrier capable of passing through a cell membrane. In an embodiment, the oligonucleotide is coupled to a substance that inactivates mRNA. In another embodiment, the substance that inactivates mRNA is a ribozyme.

Reducing the level of NPC1L1 expression by introducing anti-sense *NPC1L1* RNA into the cells of a patient is a useful method reducing intestinal sterol (*e.g.*, cholesterol) or 5α-stanol absorption and serum cholesterol in the patient.

### Kits

Kits of the present invention include ezetimibe, preferably combined with a pharmaceutically acceptable carrier, in a pharmaceutical formulation, more preferably in a pharmaceutical dosage form such as a pill, a powder, an injectable liquid, a tablet, dispersible granules, a capsule, a cachet or a suppository. See for example, Gilman et al. (eds.) (1990), The Pharmacological Bases of Therapeutics, 8th Ed., Pergamon Press; and Remington's Pharmaceutical Science*s*, *supra,* Easton, Penn.; Avis et al. (eds.) (1993) Pharmaceutical Dosage Forms: Parenteral Medications Dekker, New York; Lieberman et al. (eds.) (1990) Pharmaceutical Dosage Forms: Tablets Dekker, New York; and Lieberman et al. (eds.) (1990), Pharmaceutical Dosage Forms: Disperse Systems Dekker, New York. Preferably, the dosage form is a Zetia® tablet (Merck/Schering-Plough Corp.). Ezetimibe may be supplied in any convenient form. For example, tablets including ezetimibe may be supplied in bottles of 30, 90 or 500.

The kits of the present invention also include information, for example in the form of a package insert, indicating that the target of ezetimibe is NPC1L1 (NPC3). The term "target of ezetimibe" indicates that ezetimibe reduces intestinal sterol (*e.g.*, cholesterol) or 5α-stanol absorption, either directly or indirectly, by antagonizing NPC1L1. The form of the insert may take any form, such as paper or on electronic media such as a magnetically recorded medium (*e.g.*, floppy disk) or a CD-ROM.

The package insert may also include other information concerning the pharmaceutical compositions and dosage forms in the kit. Generally, such information aids patients and physicians in using the enclosed pharmaceutical compositions and dosage forms effectively and safely. For example, the following information regarding ezetimibe (*e.g.*, Zetia®) and/or simvastatin (*e.g.*, Zocor®) may be supplied in the insert: pharmacokinetics, pharmacodynamics, clinical studies, efficacy parameters, indications and usage, contraindications, warnings, precautions, adverse reactions, overdosage, proper dosage and administration, how supplied, proper storage conditions, references and patent information.

The kits of the invention may also include simvastatin ( 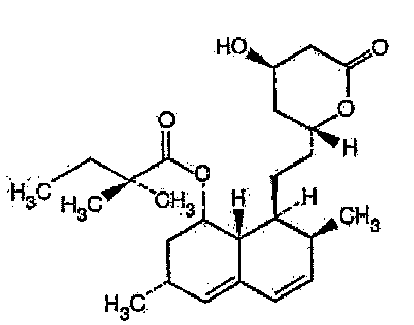 ) preferably combined with a pharmaceutically acceptable carrier, in a pharmaceutical formulation, more preferably in a pharmaceutical dosage form such as a pill, a powder, an injectable liquid, a tablet, dispersible granules, a capsule, a cachet or a suppository. Preferably, the dosage form of simvastatin is a Zocor® tablet (Merck & Co.; Whitehouse Station, NJ).

Tablets or pills comprising simvastatin may be supplied in any convenient form. For example, pills or tablets comprising 5mg simvastatin can be supplied as follows: bottles of 30, 60, 90, 100 or 1000. Pills or tablets comprising 10 mg simvastatin may be supplied as follows: bottles of 30, 60, 90, 100, 1000 or 10,000. Pills or tablets comprising 20 mg simvastatin maybe supplied as follows: bottles of 30, 60, 90, 100, 1000 or 10,000. Pills or tablets comprising 40 mg simvastatin may be supplied as follows: bottles of 30, 60, 90, 100 or 1000. Pills or tablets comprising 80 mg simvastatin may be supplied as follows: bottles of 30, 60, 90, 100, 1000 or 10,000.

Ezetimibe and simvastatin may be supplied, in the kit, as separate compositions or combined into a single composition. For example, ezetimibe and simvastatin may be supplied within a single, common pharmaceutical dosage form (*e.g.*, pill or tablet) as in separate pharmaceutical dosage forms (*e.g.*, two separate pills or tablets).

### npc1l1⁻ Cells

The present invention provides any isolated mammalian cell, (*e.g.*, an isolated mouse cell, an isolated rat cell or an isolated human cell) which lacks an *NPC1L1* gene which encodes or can produce a functional NPC1L1 protein. Included within this embodiment are mutant *npc1l1* genes comprising a point mutation, truncation or deletion of the genetic coding region or of any regulatory element (*e.g.*, a promoter).

For example, the cell can be isolated from a mutant mouse comprising a homozygous mutation of endogenous, chromosomal *NPC1L1* wherein the mouse does not produce any functional NPC1L1 protein (*e.g.*, the mouse described below in Example 22). Moreover, the present invention comprises any cell, tissue, organ, fluid, nucleic acid, peptide or other biological substance derived or isolated from such a mutant mouse, particularly a mutant, transgenic mouse which does not produce any functional NPC1L1, wherein the region of endogenous, chromosomal *NPC1L1* deleted, in the mouse, corresponds to nucleotides 790-998 of the nucleotide sequence set forth in SEQ ID NO: 45.

The isolated cell can be isolated or derived, for example, from the duodenum, gall bladder, liver, small intestine or stomach of the mutant mouse. Further, the cell can be an enterocyte.

The *npc1l1⁻* mutant cells are useful, for example, for use in control experiments in screening assays (see *e.g., supra*) since they lack any NPC1L1-dependent uptake or binding of sterol, 5α-stanol or ezetimibe. The level of inhibition caused by a particular sample, in a screening assay, can be compared to that of an assay performed with the mutant cell. Ideally, though by no means necessarily, in a screening assay, for example, as described herein, the same amount of binding will be observed by a non-mutant cell or cell membrane, in the presence of an antagonist, as is observed in connection with a mutant *npc1l1⁻* cell or cell membrane alone.

### EXAMPLES

The following examples are provided to more clearly describe the present invention and should not be construed to limit the scope of the invention in any way.

### Example 1: Cloning and Expression of Rat, Mouse and Human NPC1L1.

Rat *NPC,* mouse *NPC1L1* or human *NPC1L1* can all conveniently be amplified using polymerase chain reaction (PCR). In this approach, DNA from a rat, mouse or human cDNA library can be amplified using appropriate primers and standard PCR conditions. Design of primers and optimal amplification conditions constitute standard techniques which are commonly known in the art.

An amplified *NPC1L1* gene may conveniently be expressed, again, using methods which are commonly known in the art. For example, NPC1L1 may be inserted into a pET-based plasmid vector (Stratagene; La Joola, CA), downstream of the T7 RNA polymerase promoter. The plasmid may then be transformed into a T7 expression system (e.g., BL21DE3 E.coli cells), grown in a liquid culture and induced (e.g., by adding IPTG to the bacterial culture).

### Example 2: Direct Binding Assay.

**Membrane preparation:** Caco2 cells transfected with an expression vector containing a polynucleotide encoding NPC1L1 (*e.g.*, SEQ ID NO: 2, 4 or 12) are harvested by incubating in 5 mM EDTA/phosphate-buffered saline followed by repeated pipeting. The cells are centrifuged 5 min at 1000 x g. The EDTA/PBS is decanted and an equal volume of ice-cold 50mM Tris-HCl, pH 7.5 is added and cells are broken up with a Polytron (PT10 tip, setting 5, 30 sec). Nuclei and unbroken cells are sedimented at 1000 x g for 10 min and then the supernatant is centrifuged at 50,000 x g for 10 min. The supernatant is decanted, the pellet is resuspended by Polytron, a sample is taken for protein assay (bicinchoninic acid, Pierce), and the tissue is again centrifuged at 50,000 x g. Pellets are stored frozen at -20°C.

Binding assay: For saturation binding, four concentrations of [³H]-ezetimibe (15 Ci/mmol) are incubated without and with 10⁻⁵ M ezetimibe in triplicate with 50 µg of membrane protein in a total volume of 200 µl of 50 mM Tris-HCl, pH 7.5, for 30 min at 30°C. Samples are filtered on GFB filters and washed three times with 2 ml of cold Tris buffer. Filters are dried in a microwave oven, impregnated with Meltilex wax scintillant, and counted at 45% efficiency. For competition binding assays, five concentrations of a sample are incubated in triplicate with 18 nM [³H]-ezetimibe and 70 µg of membrane protein under the conditions described above. Curves are fit to the data with Prism (GraphPad Software) nonlinear least-squares curve-fitting program and Kᵢ values are derived from IC₅₀ values according to Cheng and Prusoff (Cheng, Y. C., et al., (1973) Biochem. Pharmacol. 22:3099-3108).

### Example 3: SPA Assay.

For each well of a 96 well plate, a reaction mixture of 10 µg human, mouse or rat NPC1L1-CHO overexpressing membranes (Biosignal) and 200 µg/well YSi-WGA-SPA beads (Amersham) in 100 µl is prepared in NPC1L1 assay buffer (25 mM HEPES, pH 7.8, 2 mM CaCl₂, 1mM MgCl₂, 125 mM NaCl, 0.1% BSA). A 0.4 nM stock ofligand-[¹²⁵I]-ezetimibe- is prepared in the NPC1L1 assay buffer. The above solutions are added to a 96-well assay plate as follows: 50 µl NPC1L1 assay buffer, 100 µl of reaction mixture, 50 µl of ligand stock (final ligand concentration is 0.1 nM). The assay plates are shaken for 5 minutes on a plate shaker, then incubated for 8 hours before cpm/well are determined in Microbeta Trilux counter (PerkinElmer).

These assays will indicate that [¹²⁵I]-ezetimibe binds to the cell membranes expressing human, mouse or rat NPC1L1. Similar results will be obtained if the same experiment is performed with radiolabeled cholesterol (*e.g.*, ¹²⁵I-cholesterol).

### Example 4: Cholesterol Uptake Assay.

CHO cells expressing either SR-B1 or three different clones of rat NPC1L1 or one clone of mouse NPC1L1 were starved overnight in cholesterol free media then dosed with [³H]-cholesterol in a mixed synthetic micelle emulsion for 4 min, 8 min ,12 min or 24 min in the absence or presence of 10 µM ezetimibe. The cells were harvested and the lipids were organically extracted. The extracted lipids were spotted on thin-layer chromatography (TLC) plates and resolved within an organic vapor phase. The free cholesterol bands for each assay were isolated and counted in a scintillation counter.

The SR-B1 expressing cells exhibited an increase in [³H]-cholesterol uptake as early as 4 min which was also inhibited by ezetimibe. The three rat clones and the one mouse clone appeared to give background levels of [³H]-cholesterol uptake which was similar to that of the untransformed CHO cell.

These experiments will yield data demonstrating that CHO cells can perform mouse, rat and human NPC1L1-dependent uptake of [³H]-cholesterol when more optimal experimental conditions are developed.

### Example 5: Expression of Rat NPC1L1 in Wistar Rat Tissue.

In these experiments, the expression of rat *NPC1L1* mRNA, in several rat tissues, was evaluated. The tissues evaluated were esophagus, stomach, duodenum, jejunum, ileum, proximal colon, distal colon, liver, pancreas, heart, aorta, spleen, lung, kidney, brain, muscle, testes, ovary, uterus, adrenal gland and thyroid gland. Total RNA samples were isolated from at least 3 male and 3 female animals and pooled. The samples were then subjected to real time quantitative PCR using Taqman analysis using standard dual-labeled fluorogenic oligonucleotide probes. Typical probe design incorporated a 5' reporter dye (*e.g.*, 6FAM (6-carboxyfluorescein) or VIC) and a 3' quenching dye (*e.g.,* TAMRA (6-carboxytetramethyl-rhodamine)).

### rat NPC1L1:

Forward: TCTTCACCCTTGCTCTTTGC (SEQ ID NO: 14)
Reverse: AATGATGGAGAGTAGGTTGAGGAT (SEQ ID NO: 15)
Probe: [6FAM]TGCCCACCTTTGTTGTCTGCTACC[TAMRA] (SEQ ID NO: 16)

### rat β-actin:

Forward: ATCGCTGACAGGATGCAGAAG (SEQ ID NO: 17)
Reverse: TCAGGAGGAGCAATGATCTTGA (SEQ ID NO: 18)
Probe: [VIC]AGATTACTGCCCTGGCTCCTAGCACCAT[TAMRA] (SEQ ID NO: 19)

PCR reactions were run in 96-well format with 25 µl reaction mixture in each well containing: Platinum SuperMix (12.5 µl), ROX Reference Dye (0.5 ul), 50 mM magnesium chloride (2 µl), cDNA from RT reaction (0.2 µl). Multiplex reactions contained gene specific primers at 200 nM each and FAM labeled probe at 100 nM and gene specific primers at 100 nM each and VIC labeled probe at 50 nM. Reactions were run with a standard 2-step cycling program, 95° C for 15 sec and 60° C for 1 min, for 40 cycles.

The highest levels of expression were observed in the duodenum, jejunum and ileum tissue. These data indicate that NPC1L1 plays a role in cholesterol absorption in the intestine.

### Example 6: Expression of Mouse NPC1L1 in Mouse Tissue.

In these experiments, the expression of mouse *NPC1L1* mRNA, in several tissues, was evaluated. The tissues evaluated were adrenal gland, BM, brain, heart, islets of langerhans, LI, small intestine, kidney, liver, lung, MLN, PLN, muscle, ovary, pituitary gland, placenta, Peyers Patch, skin, spleen, stomach, testes, thymus, thyroid gland, uterus and trachea. Total RNA samples were isolate from at least 3 male and 3 female animals and pooled. The samples were then subjected to real time quantitative PCR using Taqman analysis using the following primers and probes:

### mouse NPC1L1:

Forward: ATCCTCATCCTGGGCTTTGC (SEQ ID NO: 20)
Reverse: GCAAGGTGATCAGGAGGTTGA (SEQ ID NO: 21)
Probe: [6FAM]CCCAGCTfATCCAGATTTTCITCITCCGC[TAMRA] (SEQ ID NO: 22)

The highest levels of expression were observed in the Peyer's Patch, small intestine, gall bladder and stomach tissue. These data are consistent with a cholesterol absorption role for NPC1L1 which takes place in the digestive system.

### Example 7: Expression of Human NPC1L1 in Human Tissue.

In these experiments, the expression level of human *NPC1L1* mRNA was evaluated in 2045 samples representing 46 normal tissues. Microarray-based gene expression analysis was performed on the Affymetrix HG-U95 GeneChip using a cRNA probe corresponding to base pairs 4192-5117 (SEQ ID NO: 43) in strict accordance to Affymetrix's established protocols. Gene Chips were scanned under low photo multiplier tube (PMT), and data were normalized using either Affymetrix MAS 4.0 or MAS 5.0 algorithms. In addition "spike ins" for most samples were used to construct a standard curve and obtain RNA concentration values according Gene Logic algorithms and procedures. A summary of these results are indicated, below, in Table 2.

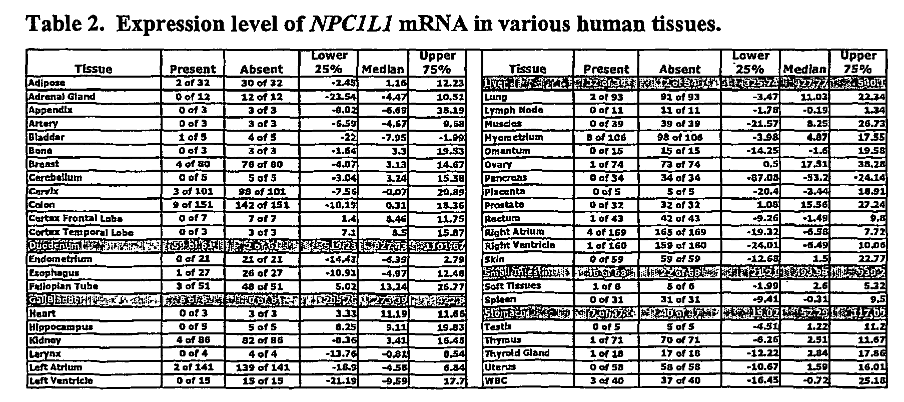

Shaded data corresponds to tissues wherein the highest levels of *NPC1L1* mRNA was detected. The "Present" column indicates the proportion of specified tissue samples evaluated wherein *NPC1L1* mRNA was detected. The "Absent" column indicates the proportion of specified tissue samples evaluated wherein *NPC1L1* RNA was not detected. The "lower 25%", "median" and "upper 75%" columns indicate statistical distribution of the relative *NPC1L1* signal intensities observed for each set of tissue evaluated.

### Example 8: Distribution of Rat NPC1L1, Rat IBAT or Rat SR-B1 mRNA in Rat Small Intestine.

In these experiments, the distribution of rat *NPC1L1* mRNA along the proximal-distal axis of rat small intestines was evaluated. Intestines were isolated from five independent animals and divided into 10 sections of approximately equal length. Total RNA was isolated and analyzed, by real time quantitative PCR using Taqman analysis, for localized expression levels of rat *NPC1L1,* rat *IBAT* (ileal bile acid transporter) or rat *SR-B1* mRNA. The primers and probes used in the analysis were:

### rat NPC1L1:

Forward: TCTTCACCCTTGCTCTTTGC (SEQ ID NO: 23)
Reverse: AATGATGGAGAGTAGGTTGAGGAT (SEQ ID NO: 24)
Probe: [6FAM]TGCCCACCTTTGTTGTCTGCTACC[TAMRA] (SEQ >D NO: 25)

### rat Villin:

Forward: AGCACCTGTCCACTGAAGATTTC (SEQ ID NO: 26)
Reverse: TGGACGCTGAGCTTCAGTTCT (SEQ ID NO: 27)
Probe: [VIC]CTTCTCTGCGCTGCCTCGATGGAA[TAMRA] (SEQ ID NO: 28)

### rat SR-B1:

Forward: AGTAAAAAGGGCTCGCAGGAT (SEQ ID NO: 29)
Reverse: GGCAGCTGGTGACATCAGAGA (SEQ ID NO: 30)
Probe: [6FAM]AGGAGGCCATGCAGGCCTACTCTGA[TAMRA] (SEQ ID NO: 31)

### rat IBAT:

Forward: GAGTCCACGGTCAGTCCATGT (SEQ ID NO: 32)
Reverse: TTATGAACAACAATGCCAAGCAA (SEQ ID NO: 33)
Probe: [6FAM]AGTCCTTAGGTAGTGGCTTAGTCCCTGGAAGCTC[TAMRAI (SEQ ID NO: 34)

The mRNA expression levels of each animal intestinal section were analyzed separately, then the observed expression level was normalized to the observed level of villin mRNA in that intestinal section. The observed, normalized mRNA expression levels for each section where then averaged.

The expression level of *NPC1L1* and *SR-B1* were highest in the jejunum (sections 2-5) as compared to that of the more distal ileum sections. Since the jejunum is believed to be the site of cholesterol absorption, these data suggest such a role for rat NPC1L1. *IBAT* distribution favoring the ileum is well document and served as a control for the experiment.

### Example 9: In situ Analysis of Rat NPC1L1 mRNA in Rat Jejunum Tissue.

The localization of rat *NPC1L1* mRNA was characterized by *in situ* hybridization analysis of rat jejunum serial sections. The probes used in this analysis were:

T7-sense probe: GTAATACGACTCACTATAGGGCCCTGACGGTCCTTCCTGA
GGGAATCTTCAC (SEQ ID NO: 35)

T7-antisense probe: GTAATACGACTCACTATAGGGCCTGGGAAGTTGGTCAT
GGCCACTCCAGC (SEQ ID NO: 36)

The RNA probes were synthesized using T7 RNA polymerase amplification of a PCR amplified DNA fragment corresponding rat *NPC1L1* nucleotides 3318 to 3672 (SEQ ID NO 1). Sense and anti-sense digoxigenin-UTP labeled cRNA probes were generated from the T7 promoter using the DIG RNA Labeling Kit following the manufacturer's instructions. Serial cryosections rat jejunum were hybridized with the sense and antiisense probes. Digoxigenin labeling was detected with the DIG Nucleic Acid Detection Kit based on previous methods. A positive signal is characterized by the deposition of a red reaction product at the site of hybridization.

The anti-sense probe showed strong staining of epithelium along the crypt-villus axis under low magnification (40X). The observed rat *NPC1L1* mRNA expression levels may have been somewhat greater in the crypts than in the villus tips. Under high magnification (200X), staining was observed in the enterocytes but not in the goblet cells. A lack of staining observed with the sense probe (control) confirmed the high specificity of the *NPC1L1* anti-sense signal. These data provided further evidence of the role of rat NPC1L1 in intestinal cholesterol absorption.

### Example 10: FACS Analysis of Fluorescently Labeled Ezetimibe Binding to Transiently Transfected CHO Cells.

In these experiments, the ability of BODIPY-labeled ezetimibe (Altmann, et al., (2002) Biochim. Biophys. Acta 1580(1):77-93) to bind to NPC1L1 and SR-B1 was evaluated. "BODIPY" is a fluorescent group which was used to detect the BODIPY-ezetimibe. Chinese hamster ovary (CHO) cells were transiently transfected with rat *NPC1L1* DNA (rNPC1L1/CHO), mouse *NPC1L1* DNA (mNPC1L1/CHO), mouse *SR-B1* DNA (mSRBI/CHO) or *EGFP* DNA (EGFP/CHO). EGFP is enhanced green fluorescent protein which was used as a positive control. The transfected CHO cells or untransfected CHO cells were then stained with 100 nM BODIPY-labeled ezetimibe and analyzed by FACS. Control experiments were also performed wherein the cells were not labeled with the BODIPY-ezetimibe and wherein untransfected CHO cells were labeled with the BODIPY-ezetimibe.

No staining was observed in the untransfected CHO, rNPC1L1/CHO or mNPC1L1/CHO cells. Fluorescence was detected in the positive-control EGFP/CHO cells. Staining was also detected in the mouse SR-B1/CHO cells. These data show that, under the conditions tested, BODIPY-ezetimibe is capable of binding to SR-B1 and that such binding is not ablated by the presence of the fluorescent BODIPY group. When more optimal conditions are determined, BODIPY-ezetimibe will be shown to label the rNPC1L1/CHO and mNPC1L1/CHO cells.

### Example 11: FACS Analysis of Transiently Transfected CHO Cells Labeled with Anti-FLAG Antibody M2.

In these experiments, the expression of FLAG-tagged NPC1L1 on CHO cells was evaluated. CHO cells were transiently transfected with mouse *NPC1L1* DNA, rat *NPC1L1* DNA, FLAG- rat *NPC1L1* DNA or FLAG- mouse *NPC1L1* DNA. The 8 amino acid FLAG tag used was DYKDDDDK (SEQ ID NO: 37) which was inserted on the amino-terminal extracellular loop just past the secretion signal sequence. The cells were incubated with commercially available anti-FLAG monoclonal mouse antibody M2 followed by a BODIPY-tagged anti-mouse secondary antibody. The treated cells were then analyzed by FACS.

The M2 antibody stained the CHO cells transfected with FLAG-rat *NPC1L1* DNA and with FLAG-mouse NPC1L1. No staining was observed in the CHO cells transfected with mouse *NPC1L1* DNA and with rat *NPC1L1* DNA. These data showed that rat NPC1L1 and mouse NPC1L1 possess no significant, inherent fluorescence and are not bound by the anti-FLAG antibody. The observed, FLAG-dependent labeling of the cells indicated that the FLAG-mouse NPC1L1 and FLAG-rat NPC1L1 proteins are localized at the cell membrane of the CHO cells.

### Example 12: FACS Analysis of FLAG-rat NPC1L1-EGFP Chimera in Transiently Transfected CHO Cells.

In these experiments, the surface and cytoplasmic localization of rat NPC1L1 in CHO cells was evaluated. CHO cells were transiently transfected with FLAG- rat *NPC1L1* DNA or with FLAG-rat *NPC1L1*-EGFP DNA. In these fusions, the FLAG tag is at amino-terminus of rat NPC1L1 and EGFP fusion is at the carboxy-terminus of rat NPC1L1. The cells were then stained with the M2 anti-FLAG mouse (primary) antibody followed by secondary staining with a BODIPY-labeled anti-mouse antibody. In control experiments, cells were stained with only the secondary antibody and not with the primary antibody (M2). The stained cells were then analyzed by FACS.

In a control experiment, FLAG-ratNPC1L1 transfected cells were stained with BODIPY anti-mouse secondary antibody but not with the primary antibody. The data demonstrated that the secondary, anti-mouse antibody possessed no significant specificity for FLAG-rat NPC1L1 and that the FLAG-rat NPC1L1, itself, possesses no significant fluorescence.

In another control experiment, unlabeled FLAG-rat NPC1L1-EGFP cells were FACS analyzed. In these experiments, autofluorescence of the enhanced green fluorescent protein (EGFP) was detected.

FLAG-rat NPC1L1 cells were stained with anti-FLAG mouse antibody M2 and with the BODIPY-labeled anti-mouse secondary antibody and FACS analyzed. The data from this analysis showed that the cells were labeled with the secondary, BODIPY-labeled antibody which indicated expression of the FLAG-rat NPC1L1 protein on the surface of the CHO cells.

FLAG-rat NPC1L1-EGFP cells were stained with anti-FLAG mouse antibody M2 and with the BODIPY-labeled anti-mouse secondary antibody and FACS analyzed. The data from this analysis showed that both markers (BODIPY and EGFP) were present indicating surface expression of the chimeric protein. The data also indicated that a portion of the protein was located within the cells and may be associated with transport vesicles. These data supported a role for rat NPC1L1 in vesicular transport of cholesterol or protein expressed in subcellular organelles such as the rough endoplasmic reticulum.

### Example 13: FACS Analysis and Fluorescent Microscopy of FLAG-rat NPC1L1-EGFP Chimera in a Cloned CHO Cell Line.

In these experiments, the cellular localization of rat NPC1L1 was evaluated by FACS analysis and by immunohistochemistry. CHO cells were transfected with FLAG-rat *NPC1L1*-EGFP DNA-and stained with anti-FLAG mouse antibody M2 and then with a BODIPY-labeled anti-mouse secondary antibody. In the fusion, the FLAG tag is at the amino-terminus of rat NPC1L1 and the enhanced green fluorescent protein (EGFP) tag is located at the carboxy-terminus of the rat NPC1L1. The stained cells were then analyzed by FACS and by fluorescence microscopy.

Cells transfected with FLAG-rat *NPC1L1*-EGFP DNA were stained with the anti-FLAG mouse antibody M2 and then with the BODIPY-labeled anti-mouse secondary antibody. FACS analysis of the cells detected both markers indicating surface expression of the chimeric protein.

FLAG-rat NPC1L1-EGFP transfected cells were analyzed by fluorescent microscopy at 63X magnification. Fluorescent microscopic analysis of the cells indicated non-nuclear staining with significant perinuclear organelle staining. Resolution of the image could not confirm the presence of vesicular associated protein. These data indicated that the fusion protein was expressed on the cell membrane of CHO cells.

### Example 14: Generation of Polyclonal Anti-rat NPC1L1 Rabbit Antibodies.

Synthetic peptides (SEQ ID NO: 39-42) containing an amino- or carboxy-terminal cysteine residue were coupled to keyhole limpet hemocyanin (KLH) carrier protein through a disulfide linkage and used as antigen to raise polyclonal antiserum in New Zealand white rabbits (range 3-9 months in age). The KLH-peptide was emulsified by mixing with an equal volume of Freund's Adjuvant, and injected into three subcutaneous dorsal sites. Prior to the 16 week immunization schedule a pre-immune sera sample was collected which was followed by a primary injection of 0.25 mg KLH-peptide and 3 scheduled booster injections of 0.1 mg KLH-peptide. Animals were bled from the auricular artery and the blood was allowed to clot and the serum was then collected by centrifugation

The anti-peptide antibody titer was determined with an enzyme linked immunosorbent assay (ELISA) with free peptide bound in solid phase (1µg/well). Results are expressed as the reciprocal of the serum dilution that resulted in an OD₄₅₀ of 0.2. Detection was obtained using the biotinylated anti-rabbit IgG, horse radish peroxidase-streptavidin (HRP-SA) conjugate, and ABTS.

### Example 15: FACS Analysis of Rat NPC1L1 Expression in CHO Cells Transiently Transfected with Rat NPC1L1 DNA Using Rabbit Anti-rat NPC1L1 Antisera.

In these experiments, the expression of rat NPC1L1 on the surface of CHO cells was evaluated. CHO cells were transfected with rat *NPC1L1* DNA, then incubated with either rabbit preimmune serum or with 10 week anti-rat NPC1L1 serum described, above, in Example 14 (*i.e*., A0715, A0716, A0867 or A0868). Cells labeled with primary antisera were then stained with a BODIPY-modified anti-rabbit secondary antibody followed by FACS analysis.

No antibody surface labeling was observed for any of the pre-immune sera samples. Specific cell surface labeling of rat NPC1L1 transfected cells was observed for both A0715 and A0868. Antisera A0716 and A0867 did not recognize rat NPC1L1 surface expression in this assay format. This indicates that the native, unfused rat NPC1L1 protein is expressed in the CHO cells and localized to the CHO cell membranes. Cell surface expression of NPC1L1 is consistent with a role in intestinal cholesterol absorption.

### Example 16: FACS Analysis of CHO Cells Transiently Transfected with FLAG-Mouse NPC1L1 DNA or FLAG-rat NPC1L1 DNA or Untransfected CHO Cells Using Rabbit Anti-rat NPC1L1 Antisera.

In these experiments, the expression of FLAG-mouse NPC1L1 and FLAG-rat NPC1L1 in CHO cells was evaluated. CHO cells were transiently transfected with FLAG-mouse *NPC1L1* DNA or with FLAG-rat *NPC1L1* DNA. The FLAG-mouse *NPC1L1* and FLAG-rat *NPC1L1* transfected cells were labeled with either A0801, A0802, A0715 or A0868 sera (see Example 14) or with anti-FLAG antibody, M2. The labeled cells were then stained with BODIPY-labeled anti-rabbit secondary antibody and FACS analyzed. The untransfected CHO cells were analyzed in the same manner as the transfected cell lines.

Positive staining of the untransfected CHO cells was not observed for any of the antisera tested. Serum A0801-dependent labeling of FLAG-rat *NPC1L1* transfected cells was observed but such labeling of FLAG-mouse *NPC1L1* transfected cells was not observed. Serum A0802-dependent labeling of FLAG-mouse *NPC1L1* or FLAG-rat *NPC1L1* transfected cells was not observed. Strong serum A0715-dependent labeling of FLAG-rat *NPC1L1* transfected cells was observed and weak serum A0715-dependent labeling of FLAG-mouse *NPC1L1* transfected cells was observed. Weak serum A0868-dependent labeling of rat *NPC1L1* and mouse *NPC1L1* transfected cells was observed. Strong Anti-FLAG M2 antibody-dependent labeling of FLAG-rat *NPC1L1* and FLAG-mouse *NPC1L1* transfected cells was observed. The strong M2 staining is likely to be due to the fact that M2 is an affinity-purified, monoclonal antibody of known concentration. In contrast, the respective antisera are polyclonal, unpurified and contain an uncertain concentration of anti-rat NPC1L1 antibody. These date provide further evidence that the FLAG-mouse NPC1L1 and FLAG-rat NPC1L1 proteins are expressed in CHO cells and localized to the CHO cell membranes. Cell surface expression of NPC1L1 is consistent with a role in intestinal cholesterol absorption.

### Example 17: Immunohistochemical Analysis of Rat Jejunum Tissue with Rabbit Anti-rat NPC1L1 Antisera A0715.

In these experiments, the localization of rat NPC1L1 in rat jejunum was analyzed by immunohistochemistry. Rat jejunum was removed, immediately embedded in O.C.T. compound and frozen in liquid nitrogen. Sections (6µm) were cut with a cryostat microtome and mounted on glass slides. Sections were air dried at room temperature and then fixed in Bouin's fixative. Streptavidin-biotin-peroxidase immunostaining was carried out using Histostain-SP kit. Endogenous tissue peroxidase activity was blocked with a 10 minute incubation in 3% H₂O₂ in methanol, and nonspecific antibody binding was minimized by a 45 minute incubation in 10% nonimmune rabbit serum. Sections were incubated with a rabbit anti-rat NPC1L1 antisera A0715 or A0868 at a 1:500 dilution at 4°C, followed by incubation with biotinylated goat anti-rabbit IgG and with streptavidin-peroxidase. Subsequently, the sections were developed in an aminoethyl carbazole (AEC)-H₂O₂ staining system and counterstained with hematoxylin and examined by microscopy. A positive reaction using this protocol is characterized by the deposition of a red reaction product at the site of the antigen-antibody reaction. Nuclei appeared blue from the hematoxylin counterstain. Controls were performed simultaneously on the neighboring sections from the same tissue block. Control procedures consisted of the following: (1) substitute the primary antibody with the pre-immune serum, (2) substitute the primary antibody with the non-immune rabbit serum, (3) substitute the primary antibody with PBS, (4) substitute the second antibody with PBS.

The example shows tissue stained with anti-rat NPC1L1 sera A0715 or with the preimmune sera analyzed at low magnification (40X) and at high magnification (200X). The A0715-stained tissue, at low magnification, showed positive, strong staining of the villi epithelial layer (enterocytes). The A0715-stained tissue at high magnification showed positive, strong staining of the enterocyte apical membranes. No staining was observed in tissue treated only with preimmune sera. Similar results were obtained with sera A0868. These data indicate that rat NPC1L1 is expressed in rat jejunum which is consistent with a role in intestinal cholesterol absorption.

### Example 18: Labeled Cholesterol Uptake Assay.

In this example, the ability of CHO cells stably transfected with rat *NPC1L1* to take up labeled cholesterol was evaluated. In these assays, cholesterol uptake, at a single concentration, was evaluated in a pulse-chase experiment. The data generated in these experiments are set forth, below, in Table 3.

### Cells:

A. CHO cells stably transfected with rat *NPC1L1* cDNA
B. CHO background (no transfection)
   Cells were seeded at 500,000 cells/ well (mL) in 12-well plates.

### Procedure:

All reagents and culture plates were maintained at 37°C unless otherwise noted.

***Starve.*** The maintenance media (F12 HAMS, 1%Pen/Strep, 10%FCS) was removed and the cells were rinsed with serum-free HAMS media. The serum-free media was then replaced with 1 mL "starve" media (F12 HAMS, Pen/Strep, 5% lipoprotein deficient serum (LPDS).

One plate of each cell line was starved overnight. The remaining 2 plates were designated "No Starve" (see below).

***Pre-Incubation.*** Media was removed from all plates, rinsed with serum-free HAMS and replaced with starve media for 30 minutes.

***3H-Cliolesterol Pulse.*** The following was added directly to each well.
0.5µCi ³H-cholesterol (∼1.1 X 10⁶ dpm/well) in 50µl of a mixed bile salt micelle.
4.8mM sodium taurocholate (2.581mg/mL)
0.6 mM sodium oleate (0.183mg/mL)
0.25 mM cholesterol (0.1 mg/mL)
Dispersed in "starve" media by ultrasonic vibration
Final media cholesterol concentration = 5µg/mL

Labeled cholesterol pulse time points were 0, 4, 12 and 24 minutes. Triplicate wells for each treatment were prepared.

***Wash.*** At the designated times, media was aspirated and the cells were washed once with Hobbs Buffer A (50mM Tris, 0.9% NaCl, 0.2% BSA, pH 7.4) and once with Hobbs Buffer B (50mM Tris, 0.9% NaCl, pH 7.4 (no BSA)) at 37°C.

***Processing*/*Analysis.*** Cells were digested overnight with 0.2N NaOH, 2mL/well at room temperature. One 1.5 mL aliquot was removed from each well, neutralized & counted for radioactivity by scintillation counting. Two additional 50µl aliquots from all wells are assayed for total protein by the Pierce micro BCA method. The quantity of labeled cholesterol observed in the cells was normalized by the quantity of protein in the cells.

**Table 3. Uptake of ³H-cholesterol by CHO cells transfected with rat NPC1L1 or mouse SR-B1 or untransfected CHO cells.**

| **Time, min** | **Total Cholesterol, dpm protein ± sem** | | | | | **Total Cholesterol, dpm/mg protein ± sem** | | | |
|---|---|---|---|---|---|---|---|---|---|
| | **NPC1L1** | | **CHO** | | | **NPC1L1** | | **CHO** | |
| **After³H-Cholesterol** | **No Starve** | | | | | | | | |
| 0 | 2067 | ±46 | 4568 | ±1937 | | 10754 | ±166 | 22881 | ±9230 |
| | | | | | | | | | |
| 4 | 2619 | ±130 | 2868 | ±193 | | 15366 | ±938 | 15636 | ±1471 |
| | | | | | | | | | |
| 12 | 2868 | ±193 | 4459 | ±170 | | 15636 | ±1471 | 24622 | ±966 |
| | | | | | | | | | |
| 24 | 7010 | ±89 | 7204 | ±173 | | 41129 | ±685 | 39361 | ±1207 |
| | | | | | | | | | |
| | | | | **Starve** | | | | | |
| 0 | 1937 | ±273 | 2440 | ±299 | | 10909 | ±1847 | 12429 | ±1673 |
| | | | | | | | | | |
| 4 | 3023 | ±308 | 2759 | ±105 | | 17278 | ±1650 | 14307 | ±781 |
| | | | | | | | | | |
| 12 | 2759 | ±105 | 4857 | ±186 | | 14307 | ±781 | 26270 | ±1473 |
| | | | | | | | | | |
| 24 | 6966 | ±72 | 7344 | ±65 | | 39196 | ±174 | 38381 | ±161 |

| | | | | | | | | | |
|---|---|---|---|---|---|---|---|---|---|
| dpm=disintegrations per minute sem=standard error of the mean | | | | | | | | | |

### Example 19: Effect of Ezetimibe on Cholesterol Uptake.

The effect of ezetimibe on the ability of CHO cells stably transfected with mouse or rat *NPC1L1* or mouse *SR-B1* to take up ³H-labeled cholesterol was evaluated in pulse-chase experiments. One cDNA clone of mouse *NPC1L1* (C7) and three clones of rat *NPC1L1* (C7, C17 and C21) were evaluated. The ability of CHO cells stably transfected with mouse *SR-B1,* mouse *NPC1L1* and rat *NPC1L1* to take up labeled cholesterol, in the absence of ezetimibe, was also evaluated in the pulse-chase experiments. Data generated in these experiments are set forth, below, in Tables 4 and 5. Additionally, the quantity of total cholesterol taken up by transfected and untransfected CHO cells in the presence of four different unlabeled cholesterol concentrations was also evaluated. The data from these experiments is set forth, below, in Table 6.

### Cells:

A. CHO cells stably transfected with rat or mouse *NPC1L1* cDNA
B. CHO background (no transfection)
C. *SR-B1* transfected CHO cells
   Cells seeded at 500,000 cells / well (mL) in 12-well plates.

### Procedure:

All reagents and culture plates were maintained at 37°C unless otherwise noted.

***Starve.*** The maintenance media (F12 HAMS, 1%Pen/Strep, 10%FCS) was removed and the cells were rinsed with serum-free HAMS media. The serum-free media was then replaced with 1 mL "starve" media (F12 HAMS, Pen/Strep, 5% lipoprotein deficient serum (LPDS). The cells were then starved overnight.

***Pre-Incubation*/*pre-dose.*** Media was removed from all plates and replaced with fresh starve media and preincubated for 30 minutes. Half of the wells received media containing ezetimibe (stock soln in EtOH; final conc. = 10µM).

***³H-Cliolesterol Pulse.*** The following was added directly to each well:
0.5µCi ³H-cholesterol (∼1.1 X 10⁶ dpm/well) in 50µl of a mixed bile salt micelle
4.8mM sodium taurocholate (2.581mg/mL)
0.6 mM sodium oleate (0.183mg/mL)
0.25 mM cholesterol (0.1 mg/mL)
Dispersed in "starve" media by ultrasonic vibration
Final media cholesterol concentration = 5µg/mL

Labeled cholesterol pulse time points were 4, 12, 24 minutes and 4 hours. Triplicate wells were prepared for each treatment.

***Wash.*** At designated times, media was aspirated and cells were washed once with Hobbs Buffer A (50mM Tris, 0.9% NaCl, 0.2% bovine serum albumin (BSA), pH 7.4) and once with Hobbs Buffer B (50mM Tris, 0.9% NaCl, pH 7.4 (no BSA)) at 37°C.

### Processing/Analysis.

A. 4, 12, 24 minute time points: Cells were digested overnight with 0.2N NaOH, 2mL/well, room temperature. One 1.5 mL aliquot was removed from each well, neutralized & counted for radioactivity by scintillation counting.
B. 4 hour time point: The digested cells were analyzed by thin-layer chromatography to determine the content of cholesterol ester in the cells.
Extracts were spotted onto TLC plates and run for 30 minutes in 2 ml hexane:isopropanol (3:2) mobile phase for 30 minutes, followed by a second run in 1ml hexane:isopropanol (3:2) mobile phase for 15 minutes.
C. Protein determination of cell extracts. Plates containing a sample of the cell extracts were placed on orbital shaker at 120 rpm for indicated times and then extracts are pooled into 12 X 75 tubes. Plates were dried and NaOH (2ml/well) added. The protein content of the samples were then determined. Two additional 50µl aliquots from all wells were assayed for total protein by the Pierce micro BCA method. The quantity of labeled cholesterol observed in the cells was normalized to the quantity of protein in the cells.

**Table 4. Total Cholesterol in Transfected CHO Cells in the Presence and Absence of Ezetimibe.**

| | Total Cholesterol, dpm ± sem | | | | | Total Cholesterol, dpm/mg protein ± sem | | | |
|---|---|---|---|---|---|---|---|---|---|
| | Vehicle | | EZ (10 µM) | | | Vehicle | | EZ (10µM) | |
| **Clones:** | **4 Min Pulse** | | | | | | | | |
| **CHO Control** | 3413 | ±417 | 3222 | ±26 | | 33443 | ±4070 | 31881 | ±483 |
| | | | | | | | | | |
| **SR-BI** | 14207 | ±51 | 10968 | ±821 | | 118242 | ±1261 | 92474 | ±2902 |
| | | | | | | | | | |
| **mNPC1L1(C7)** | 4043 | ±419 | 4569 | ±222 | | 30169 | ±3242 | 30916 | ±1137 |
| | | | | | | | | | |
| **rNPC1L1(C21)** | 3283 | ±288 | 3769 | ±147 | | 23728 | ±2111 | 27098 | ±689 |
| | | | | | | | | | |
| **rNPC1L1(C17)** | 3188 | ±232 | 3676 | ±134 | | 24000 | ±832 | 28675 | ±527 |
| | | | | | | | | | |
| **rNPC1L1(C7)** | 1825 | ±806 | 3268 | ±121 | | 15069 | ±6794 | 27285 | ±968 |
| | | | | | | | | | |
| | **12 Min Pulse** | | | | | | | | |
| **CHO Control** | 4710 | ±246 | 4532 | ±165 | | 44208 | ±2702 | 43391 | ±1197 |
| | | | | | | | | | |
| **SR-BI** | 16970 | ±763 | 12349 | ±298 | | 140105 | ±6523 | 98956 | ±447 |
| | | | | | | | | | |
| **mNPC1L1(C7)** | 6316 | ±85 | 6120 | ±755 | | 45133 | ±342 | 41712 | ±4054 |
| | | | | | | | | | |
| **rNPC1L1(C21)** | 5340 | ±12 | 4703 | ±231 | | 40018 | ±1181 | 33985 | ±1928 |
| | | | | | | | | | |
| **rNPC1L1(C17)** | 4831 | ±431 | 4579 | ±257 | | 37378 | ±3461 | 34063 | ±1619 |
| | | | | | | | | | |
| **rNPC1L1(C7)** | 4726 | ±272 | 4664 | ±63 | | 39100 | ±2350 | 38581 | ±784 |
| | | | | | | | | | |
| | **24 Min Pulse** | | | | | | | | |
| **CHO Control** | 7367 | ±232 | 6678 | ±215 | | 65843 | ±1281 | 61764 | ±2131 |
| | | | | | | | | | |
| **SR-BI** | 39166 | ±2152 | 23558 | ±1310 | | 324126 | ±11848 | 198725 | ±11713 |
| | | | | | | | | | |
| **mNPC1L1(C7)** | 10616 | ±121 | 9749 | ±482 | | 77222 | ±1040 | 74041 | ±3670 |
| | | | | | | | | | |
| **rNPC1L1(C21)** | 9940 | ±587 | 8760 | ±293 | | 76356 | ±9618 | 66165 | ±2181 |
| | | | | | | | | | |
| **rNPC1L1(C17)** | 8728 | ±721 | 8192 | ±737 | | 70509 | ±5189 | 62279 | ±4352 |
| | | | | | | | | | |
| **rNPC1L1(C7)** | 8537 | ±148 | 7829 | ±204 | | 72134 | ±1305 | 63482 | ±369 |

| | | | | | | | | | |
|---|---|---|---|---|---|---|---|---|---|
| EZ = ezetimibe | | | | | | | | | |

**Table 5. Cholesterol Ester in CHO cells in the Presence or Absence of Ezetimibe.**

| | **Cholesteryl Ester, dpm ± sem** | | | | | **Cholesteryl Ester, dpm/mg protein ± sem** | | | |
|---|---|---|---|---|---|---|---|---|---|
| | **Vehicle** | | **EZ (10 µM)** | | | **Vehicle** | | **EZ(10µM)** | |
| **Clones:** | | | | **4 Hour Pulse** | | | | | |
| **CHO Control** | 652 | ±13 | 208 | ±9 | | 5647 | ±55 | 1902 | ±87 |
| | | | | | | | | | |
| **SR-BI** | 47608 | ±1292 | 9305 | ±401 | | 391067 | ±14391 | 72782 | ±3181 |
| | | | | | | | | | |
| **mNPC1L1(C7)** | 732 | ±127 | 453 | ±118 | | 4994 | ±827 | 3057 | ±776 |
| | | | | | | | | | |
| **rNPC1L1(C21)** | 2667 | ±90 | 454 | ±33 | | 18655 | ±1032 | 3193 | ±265 |
| | | | | | | | | | |
| **rNPC1L1(C17)** | 751 | ±74 | 202 | ±10 | | 5379 | ±481 | 1510 | ±62 |
| | | | | | | | | | |
| **rNPC1L1(C7)** | 462 | ±25 | 191 | ±54 | | 3597 | ±193 | 1496 | ±403 |
| | | | | | | | | | |

| | **Free Cholesterol, dpm±sem** | | | | | **Free Cholesterol, dmg/mg protein±sem** | | | |
|---|---|---|---|---|---|---|---|---|---|
| | **Vehicle** | | **EZ (10 µM)** | | | **Vehicle** | | **EZ (10µM)** | |
| | | | | **4 Hour Pulse** | | | | | |
| **CHO Control** | 61612 | ±1227 | 56792 | ±568 | | 533876 | ±17770 | 519607 | ±16203 |
| | | | | | | | | | |
| **SR-BI** | 214678 | ±4241 | 194519 | ±474 | | 1762873 | ±146607 | 1521341 | ±4185 |
| | | | | | | | | | |
| **mNPC1L1(C7)** | 79628 | ±793 | 77516 | ±1910 | | 544661 | ±1269 | 523803 | ±10386 |
| | | | | | | | | | |
| **rNPC1L1(C21)** | 71352 | ±1343 | 69106 | ±711 | | 498016 | ±8171 | 485460 | ±4410 |
| | | | | | | | | | |
| **rNPC1L1(C17)** | 78956 | ±3782 | 71646 | ±446 | | 566456 | ±29204 | 536651 | ±7146 |
| | | | | | | | | | |
| **rNPC1L1(C7)** | 75348 | ±2093 | 70628 | ±212 | | 586127 | ±13932 | 556855 | ±7481 |

| | | | | | | | | | |
|---|---|---|---|---|---|---|---|---|---|
| EZ =ezetimibe | | | | | | | | | |

**Table 6. Uptake of labeled cholesterol in the presence of increasing amounts of unlabeled cholesterol.**

| | Total Cholesterol, dpm ± sem | | | | | Total Cholesterol, dp m/mg, protein ± sem | | | |
|---|---|---|---|---|---|---|---|---|---|
| | CHO Control | SR-BI | mNPC1L1(C7) | rNPC1L1 (C21) | | CHO Control | SR-BI | mNPC1L1 (C7) | rNPC1L1 (C21 |
| **Cold Cholesterol** | | | | **24 Min Pulse** | | | | | |
| 3 µg/mL | 12271 ±430 | 49603 ±2423 | 14250 ±1623 | 10656 ±1233 | | 103936 ±5413 | 541562 ±13783 | 140764 ±14433 | 94945 ±12916 |
| 10 µg/mL | 16282 ±2433 | 79967 ±3151 | 25465 ±3037 | 13223 ±4556 | | 151283 ±23345 | 830224 ±32254 | 250985 ±27431 | 123433 ±34092 |
| 30 µg/mL | 14758 ±1607 | 71925 ±3863 | 19001 ±1530 | 13218 ±1149 | | 135109 ±12106 | 796236 ±18953 | 180436 ±12112 | 111522 ±6941 |
| 100 µg/mL | 16458 ±1614 | 58183 ±4542 | 15973 ±1665 | 11560 ±1132 | | 149559 ±17977 | 630143 ±3718 | 147717 ±8261 | 101328 ±7191 |
| | | | | | | | | | |

| | Cholesteryl Ester, dpm ± sem | | | | | Cholesteryl Ester, dp m/mg, protein ± sem | | | |
|---|---|---|---|---|---|---|---|---|---|
| | CHO Control | SR-BI | mNPC1L1(C7) | rNPC1L1 (C21) | | CHO Control | SR-BI | mNPC1L1 (C7) | rNPC1L1 (C21) |
| | | | | **4 Hour Pulse** | | | | | |
| 3 µg/mL | 2737 ±114 | 39596 ±1241 | 1561 ±1 | 4015 ±47 | | 22050 ±978 | 382641 ±5955 | 13634 ±217 | 32020 ±641 |
| 10 µg/mL | 1646 ±76 | 17292 ±362 | 998 ±36 | 1866 ±33 | | 13323 ±606 | 157914 ±3400 | 8917 ±467 | 14849 ±127 |
| 30 µg/mL | 970 ±46 | 6642 ±153 | 537 ±82 | 970 ±9 | | 7627 ±323 | 63547 ±1760 | 485 ±748 | 7741 ±100 |
| 100 µg/mL | 895 ±156 | 4777 ±27 | 405 ±7 | 777 ±16 | | 7135 ±1230 | 45038 ±1526 | 3663 ±63 | 6005 ±793 |
| | | | | | | | | | |

| | Free Cholesterol, dpm ± sem | | | | | Free Cholesterol, dp m/mg, protein ± sem | | | |
|---|---|---|---|---|---|---|---|---|---|
| | CHO Control | SR-BI | mNPC1L1(C7) | rNPC1L1 (C21) | | CHO Control | SR-BI | mNPC1L1 (C7) | rNPC1L1 (C21) |
| | | | | **4 Hour Pulse** | | | | | |
| 3 µg/mL | 89013 ±3724 | 211783 ±3268 | 104343 ±2112 | 92244 ±987 | | 717308 ±34130 | 2047695 ±16213 | 914107 ±5869 | 735498 ±11209 |
| 10 µg/mL | 136396 ±8566 | 278216 ±10901 | 196173 ±4721 | 125144 ±377 | | 1105118 ±76074 | 2540130 ±92471 | 1753072 ±86378 | 996324 ±27850 |
| 30 µg/mL | 131745 ±2922 | 224429 ±2556 | 149172 ±19689 | 117143 ±4976 | | 1036195 ±21142 | 2149315 ±78068 | 1357136 ±150264 | 934772 ±43202 |
| 100 µg/mL | 79336 ±4011 | 231470 ±4221 | 114599 ±2803 | 93538 ±1538 | | 632965 ±29756 | 2182022 ±36793 | 1035979 ±30329 | 723225 ±21694 |
| | | | | | | | | | |

| | Cholesteryl Ester, dpm ± sem | | | | | Cholesteryl Ester, dp m/mg, protein ± sem | | | |
|---|---|---|---|---|---|---|---|---|---|
| | CHO Control | SR-BI | mNPCIL1(C7) | rNPCIL1 (C21) | | CHO Control | SR-BI | mNPC1L1 (C7) | rNPC1L (C21) |
| | | | | **24 Hour Pulse** | | | | | |
| 3 µg/mL | 57373 ±2704 | 162296 ±1644 | 22986 ±940 | 59377 ±953 | | 357629 ±14639 | 1248900 ±18565 | 160328 ±6565 | 401315 ±5557 |
| 10 µg/mL | 33730 ±1296 | 112815 ±373 | 14836 ±552 | 31979 ±523 | | 215004 ±5942 | 830231 ±12764 | 93594 ±4205 | 200451 ±5239 |
| 30 µg/mL | 19193 ±100 | 58668 ±1413 | 8878 ±335 | 18963 ±380 | | 122071 ±1271 | 446581 ±3472 | 59091 ±2697 | 119728 ±2131 |
| 100 µg/mL | 16761 ±398 | 31280 ±1270 | 8784 ±946 | 14933 ±311 | | 103235 ±1739 | 272796 ±13392 | 60670 ±4567 | 96215 ±1023 |
| | | | | | | | | | |

| | Free Cholesterol, dpm ± sem | | | | | Free Cholesterol, dp m/mg, protein ± sem | | | |
|---|---|---|---|---|---|---|---|---|---|
| | CHO Control | SR-BI | mNPC1L1(C7) | rNPC1L1 (C21) | | CHO Control | I SR-BI | mNPC1L1 (C7) | rNPC1L1 (C21) |
| | | | | **24 Hour Pulse** | | | | | |
| 3 µg/mL | 248985 ±4207 | 337819 ±4519 | 285610 ±5187 | 227244 ±1016 | | 1552637 ±18954 | 2752957 ±24984 | 1993256 ±56968 | 153023 ±10304 |
| 10 µg/mL | 231208 ±1927 | 269822 ±5372 | 311777 ±3227 | 231666 ±6198 | | 1477414 ±85954 | 1984473 ±13420 | 2069980 ±25517 | 1464457 ±58517 |
| 30 µg/mL | 203566 ±6003 | 225273 ±5932 | 279604 ±6612 | 209372 ±3336 | | 1294878 ±41819 | 1716066 ±52531 | 1859476 ±29507 | 1321730 ±5452 |
| 100 µg/mL | 178424 ±2379 | 167082 ±3311 | 229832 ±4199 | 182678 ±7709 | | 1099648 ±25160 | 1455799 ±9385 | 1599244 ±76932 | 1177546 ±51191 |

### Example 20: Labeled Cholesterol Uptake Assay.

In this example, the ability of CHO cells transiently transfected with rat *NPC1L1* or mouse *SR-B1* to take up labeled cholesterol was evaluated. Also evaluated was the ability of rat NPC1L1 to potentiate the ability of CHO cells transfected with mouse *SR-BI* to take up labeled cholesterol. In these assays, cholesterol uptake, at a single concentration, was evaluated in pulse-chase experiments. The data generated in these experiments are set forth, below, in Table 7.

### Cells:

A. CHO background cells (mock transfection).
B. CHO cells transiently transfected with mouse *SR-B1.*
C. CHO transiently transfected with rat *NPC1L1* cDNAs (n=8 clones).

Transiently transfected cells were seeded at 300,000 cells / well (mL) in 12-well plates.

### Procedure:

All reagents and culture plates were maintained at 37°C unless otherwise noted.

***Starve.*** The maintenance media (F12 HAMS, 1%Pen/Strep, 10%FCS) was removed from the cells and replaced with 1 mL "starve" media (F12 HAMS, Pen/Strep, 5% lipoprotein deficient serum (LPDS). Cells were starved for 1 hour.

***³H-Cholesterol Pulse.*** The following was added directly to each well.
0.5µCi ³H-cholesterol (∼1.1 X 10⁶ dpm/well) in 50µl of a mixed bile salt micelle.
4.8mM sodium taurocholate (2.581mg/mL)
0.6 mM sodium oleate (0.183mg/mL)
0.25 mM cholesterol (0.1 mg/mL)
Dispersed in "starve" media by ultrasonic vibration
Final media cholesterol concentration = 5µg/mL

Labeled cholesterol pulse time points were 24 Min and 4 hours. Triplicate wells for each treatment.

***Wash.*** At the designated times, media was aspirated and cells were washed once with Hobbs Buffer A (50mM Tris, 0.9% NaCl, 0.2% BSA, pH 7.4) and once with Hobbs Buffer B (50mM Tris, 0.9% NaCl, pH 7.4 (no BSA)) at 37°C.

### Processing/Analysis.

A. 24 minute time point: Cells were digested overnight with 0.2N NaOH, 2mL/well at room temp. One, 1.5 mL aliquot was removed from each well, neutralized & counted for radioactivity by scintillation counting.
B. 4 hour time point: The digested cells were analyzed by thin-layer chromatography to determine the content of cholesterol ester in the cells.
The extracts were spotted onto thin layer chromatography plates and run in 2 ml hexane:isopropanol (3:2) containing mobile phase for 30 minutes, followed by a second run in 1ml hexane:isopropanol (3:2) containing mobile phase for 15min.
C. Protein determination of cell extracts: Plates containing a sample of the cell extracts were placed on orbital shaker at 120 rpm for indicated times and then extracts are pooled into 12X75 tubes. Plates were dried and NaOH (2ml/well) added. The protein content of the samples were then determined. Two additional 50µl aliquots from all wells were assayed for total protein by the Pierce micro BCA method. The quantity of labeled cholesterol observed in the cells was normalized to the quantity of protein in the cells.

**Table 7. Labeled cholesterol uptake in transiently transfected CHO cells.**

| | **Total Cholesterol, ± sem** | | |
|---|---|---|---|
| | **dpm** | | **dpm/mg protein** |
| **Transfection** | **24 Min Pulse** | | |
| **CHO Control (mock)** | 4721 ±436 | | 49024 ±4328 |
| **SR-BI(Tramient)** | 5842 ±82 | | 59445 ±1099 |
| **NPC1L1 (Transient)** | 4092 ±377 | | 47026 ±2658 |
| **SR-BI/NPC1L1 (trans)** | 3833 ±158 | | 52132 ±3071 |

| | **Cholesteryl Ester, ± sem** | | |
|---|---|---|---|
| | **dpm** | | **dpm/mg protein** |
| | **4 Hour Pulse** | | |
| **CHO Control (mock)** | 2132 ±40 | | 20497 ±640 |
| **SR-BI(Transient)** | 5918 ±237 | | 51812 ±1417 |
| **NPC1L1 (Transient)** | 1944 ±93 | | 19788 ±642 |
| **SR-BI/NPC1L1 (trans)** | 4747 ±39 | | 58603 ±1156 |

| | **Free Cholesterol, ± sem** | | |
|---|---|---|---|
| | **dpm** | | **dpm/mg protein** |
| | **4 Hour Pulse** | | |
| **CHO Control (mock)** | 45729 ±328 | | 439346 ±5389 |
| **SR-BI(Transient)** | 50820 +2369 | | 444551 ±9785 |
| **NPC1L1 (Transient)** | 39913 ±1211 | | 406615 ±6820 |
| **SR-BI/NPC1L1 (trans)** | 37269 ±1225 | | 459509 ±6195 |

### Example 21: Expression of rat, mouse and human NPC1L1.

In this example, *NPC1L1* was introduced into cells and expressed. Species specific NPC1L1 expression constructs were cloned into the plasmid pCDNA3 using clone specific PCR primers to generate the ORF flanked by appropriate restriction sites compatible with the polylinker of the vector. For all three species of NPC1L1, small intestine total tissue RNA was used as a template for reverse transcriptase-polymerase chain reaction (RT-PCR) using oligo dT as the template primer. The rat *NPC1L1* was cloned as an EcoRI fragment, human *NPC1L1* was cloned as a XbaI/NotI fragment and mouse *NPC1L1* was cloned as an EcoRI fragment. Forward and reverse strand sequencing of each clone was performed to confirm sequence integrity. Standard transient transfection procedures were used with CHO cells. In a 6-well plate CHO cells were plated 1 day before transfection at a plating density of 2 X 10⁵ cells/well. The following day, cells were incubated with 2 µg plasmid DNA and 6 µL Lipofectamine for 5 hours followed a fresh media change. Forty-eight hours later, cells were analyzed for NPC1L1 expression using anti-NPC1L1 antisera by either FACS or western blot. To establish stable long term cell lines expressing NPC1L1, transfected CHO cells were selected in the presence of geneticin (G418, 0.8 mg/ml) as recommended by the manufacturer (Life Technologies). Following one month of selection in culture, the cell population was stained with anti-NPC1L1 antisera and sorted by FACS. Individual positive staining cells were cloned after isolation by limiting dilution and then maintained in selective media containing geneticin (0.5 mg/ ml).

Other cell types less susceptible to transfection procedures have been generated using adenoviral vector systems. This system used to express NPC1L1 is dervied from Ad 5, a type C adenovirus. This recombinant replication-defective adenoviral vector is made defective through modifications of the E1, E2 and E4 regions. The vector also has additional modifications to the E3 region generally affecting the E3b region genes RIDa and RIDb. NPC1L1 expression was driven using the CMV promoter as an expression cassette substituted in the E3 region of the adenovirus. Rat and mouse NPC1L1 were amplified using clone specific primers flanked by restriction sites compatible with the adenovirus vector Adenovirus infective particles were produced from 293-D22 cells in titers of 5 X 10¹⁰ P/mL. Viral lysates were used to infect cells resistant to standard transfection methodologies. In Caco2 cells, which are highly resistant to heterologous protein expression, adenovirus mediated expression of NPC1L1 has been shown by western blot analysis to persist at least 21 days post-infection.

### Example 22: NPC1L1 Knock-Out Transgenic Mouse.

*NPC1L1* knockout mice were constructed via targeted mutagenesis. This methodology utilized a targeting construct designed to delete a specific region of the mouse *NPC1L1* gene. During the targeting process the *E*. *coli lacZ* reporter gene was inserted under the control of the endogenous *NPC1L1* promoter. The region in *NPC1L1* (SEQ ID NO: 45) being deleted is from nucleotide 790 to nucleotide 998. The targeting vector contains the *LacZ-Neo* cassette flanked by 1.9 kb 5' arm ending with nucleotide 789 and a 3.2 kb 3' arm starting with nucleotide 999. Genomic DNA from the recombinant embryonic stem cell line was assayed for homologous recombination using PCR Amplified DNA fragments were visualized by agarose gel electrophoresis. The test PCRs employed a gene specific primer, which lies outside of and adjacent to the targeting vector arm, paired with one of three primers specific to the *LacZ-Neo* cassette sequence. For 5' PCR reconfirmation, the *NPC1L1* specific oligonucleotide ATGTTAGGTGAGTCTGAACCTACCC (SEQ ID NO: 46) and for 3'PCR reconfirmation the *NPC1L1* specific oligonucleotide GGATTGCATTTCCTTCAA GAAAGCC (SEQ ID NO: 47) were used. Genotyping of the F2 mice was performed by multiplex PCR using the *NPC1L1* specific forward primer TATGGCTCTGCCC TCTGCAATGCTC (SEQ ID NO: 48) the *LacZ-Neo* cassette specific forward primer TCAGCAGCCTCTGTTCCACATACACTTC (SEQ ID NO: 49) in combination with the *NPC1L1* gene specific reverse primer GTTCCACAGGGTCTGTGGTGAGTTC (SEQ ID NO: 50) allowed for determination of both the targeted and endogenous alleles. Analysis of the PCR products by agarose gel electrophoresis distinguished the wild-type, heterozygote and homozygote null mouse from each other.

### Example 23: Acute Cholesterol Absorption in NPC1L1-Deficient Mice.

To determine whether *NPC1L1* plays a role in cholesterol absorption, *NPC1L1* deficient mice were studied.

Mice deficient in *NPC1L1* (-/-) were generated by breeding heterozygote mice (+/) to obtain wild-type (+/+) and *NPC1L1* deficient mice (-/-). Non-fasted mice (6.5-9 weeks old, mixed 129 and C57BL/6 background) were weighed and grouped (n=2 -/- and n=4 +/+). All animals were gavaged (Feeding needles, 24G x 1 inch, Popper and Sons, NY) with 0.1 ml corn oil (Sigma; St. Louis, MO) containing 1µCi ¹⁴C-cholesterol (New England Nuclear, [⁴⁻¹⁴C] Cholesterol, NEC-018) and 0.1mg carrier cholesterol mass (Sigma; St. Louis, MO). Two hours later, blood was collected by heart puncture. The liver was removed, weighed, and three samples were placed into 20 ml counting vials. Tissues were digested in 1 ml of 1N NaOH at 60°C overnight. The tissue digests were acidified by addition of 250µl of 4N HCl prior to liquid scintillation counting (LSC). Plasma was isolated by centrifugation at 10,000 rpm for 5 minutes in a microfuge and duplicate 100µl aliquots of plasma were taken for LSC.

Cholesterol absorption, evaluated by this acute technique and expressed as the total amount of radioactive cholesterol in the plasma and liver, demonstrated that the wild type mice (+/+) absorbed an average of 11,773 dpm and NPC1L1 deficient mice absorbed 992 dpm of the ¹⁴C-cholesterol. These results indicate that the NPC1L1 deficient mice have a 92% reduction in cholesterol absorption. These data confirm the role of NPC1L1 in intestinal cholesterol absorption. Inhibition of NPC1L1-mediated cholesterol absorption, in a subject, by administering NPC1L1 antagonists, such as ezetimibe, to the subject, are a useful way to reduce serum cholesterol levels and the occurrence of atherosclerosis in the subject

### Examples 24: Cholesterol Absorption in NPC1L1 (NPC3) Knockout Mice (Fecal Ratio Method: Cholesterol/Sitostanol).

In this example, cholesterol absorption and the activity of ezetimibe was determined in the *NPC1L1* knockout mice (-/-), heterozygous mice (+/-), and age matched wild-type mice (+/+).

Cholesterol absorption in the mice was determined by the dual fecal isotope ratio method as described by Altmann et al. (Biochim. Biophys. Acta. 1580(1):77-93 (2002)). Mice (n= 4-6/group) were fed a standard rodent chow diet and in some groups treated daily with a maximally effective dose of ezetimibe (10 mg/kg). Mice were gavaged with ¹⁴C-cholesterol (1µCi, 0.1mg unlabeled cholesterol) and ³H-sitostanol (2µCi) in 0.1ml corn oil. Feces were collected for 2 days and fecal ¹⁴C-cholesterol and ³H-sitostanol levels were determined by combustion in a Packard Oxidizer. The fraction of cholesterol absorbed, as evaluated by the fecal dual isotope technique, was similar in wild type (+/+) and heterozygous mice (+/-) fed a chow diet (heterozygous mice absorbed 46 ±5% and age matched wild type mice absorbed 51 ±3% of the dose of ¹⁴C-cholesterol). The *NPC1L1* knockout mice (-/-) absorbed 15.6 ±0.4% of the ¹⁴C-cholesterol, which was similar to the wild type mice treated with a maximally effective dose of ezetimibe (16.1 ±0.3%), and reduced by 69% compared to wild type mice (p<0.001). In *NPC1L1* knockout treated with ezetimibe at 10 mg/kg/day, cholesterol absorption was similar to that seen in the untreated knockout mice (16.2 ±0.6% compared to 15.6% ±0.4%, respectively). Thus, the majority of cholesterol absorption is dependent on the presence of *NPC1L1* and the residual cholesterol absorption in mice lacking *NPC1L1* is insensitive to ezetimibe treatment. These results indicate that *NPC1L1* is involved in the small intestinal enterocyte uptake and absorption of cholesterol and is in the ezetimibe sensitive pathway.

### Example 25: Mouse Screening Assay (Acute Cholesterol Absorption).

The following screening assay is used to identify the presence of an NPC1L1 antagonist in a sample.

Mice deficient in *NPC1L1* (-/-) are generated by breeding heterozygote mice (+/) to obtain wild-type (+/+) and *NPC1L1* deficient mice (-/-).

In a first set of experiments, non-fasted mice (6.5-9 weeks old, mixed 129 and C57BL/6 background) are weighed and grouped (n=1 to 4 -/- and n=1 to 4 +/+). All animals are gavaged (Feeding needles, 24G x 1 inch, Popper and Sons, NY) with 0.1 ml corn oil (Sigma; St. Louis, MO) containing 1µCi ¹⁴C-cholesterol (New England Nuclear, [⁴⁻¹⁴C] Cholesterol, NEC-018) and 0.1mg carrier cholesterol mass (Sigma; St. Louis, MO).

In another set of experiments, 1 to 4 wild-type *NPC1L1* mice (+/+) are treated identically to the mice in the first set of experiments, above, except that the mice are additionally fed a sample to be tested for the presence of an NPC1L1 antagonist.

Two hours later, blood is collected from each mouse by heart puncture. The liver is removed, weighed, and three samples are placed into 20 ml counting vials. Tissues are digested in 1 ml of 1N NaOH at 60°C overnight. The tissue digests are acidified by addition of 250µl of 4N HCl prior to liquid scintillation counting (LSC). Plasma is isolated by centrifugation at 10,000 rpm for 5 minutes in a microfuge and duplicate 100µ1 aliquots of plasma are taken for LSC.

Cholesterol absorption, evaluated by this acute technique is expressed as the total amount of radioactive cholesterol in the plasma and liver. The sample tested is determined to contain an NPC1L1 antagonist when the level of cholesterol absorption (as measured by the above described methods) in the wild-type NPC1L1 mouse (+/+) which was fed the sample and in the NPC1L1 deficient mouse (-/-) are less than the amount of cholesterol absorption in the wild-type NPC1L1 mouse (+/+) which was not fed the sample.

### Example 26: Mouse Screening Assay (Fecal Ratio Method: Cholesterol/Sitostanol).

The following screening assay is used to identify the presence of an NPC1L1 antagonist in a sample.

Cholesterol absorption in the mice is determined by the dual fecal isotope ratio method as described by Altmann et al. (Biochim. Biophys. Acta. 1580(1):77-93 (2002)).

Three groups of mice (n=1-6/group) are assembled. Two separate groups comprise wild-type NPC1L1 mice (+/+) and one group comprises NPC1L1 deficient mice (-/-).

Each group is fed a standard rodent chow diet and in some groups treated daily. Mice are gavaged with ¹⁴C-cholesterol (1µCi, 0.1mg unlabeled cholesterol) and ³H-sitostanol (2µCi) in 0.1ml corn oil. One group of mice, which comprise wild-type NPC1L1 mice (+/+) are further fed a sample to be tested for the presence of an NPC1L1 antagonist. Feces are collected for 2 days and fecal ¹⁴C-cholesterol and ³H-sitostanol levels are determined by combustion in a Packard Oxidizer.

The sample tested is determined to contain an NPC1L1 antagonist when the level of cholesterol and/or sitostanol absorption (as measured by the above described methods) in the wild-type NPC1L1 mouse (+/+) which was fed the sample and in the NPC1L1 deficient mouse (-/-) are less than the amount of cholesterol and/or sitostanol absorption in the wild-type NPC1L1 mouse (+/+) which was not fed the sample.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

Patents, patent applications, publications, product descriptions, Genbank Accession Numbers and protocols are cited throughout this application, the disclosures of which are incorporated herein by reference in their entireties for all purposes.

The following represent further embodiments of the present invention:
1. A mutant transgenic mouse comprising a homozygous mutation of endogenous, chromosomal *NPC1L1* wherein the mouse does not produce any functional NPC1L1 protein.
2. The mouse of embodiment 1 wherein the mouse exhibits a reduced serum sterol or 5α-stanol level, a reduced liver sterol or 5α-stanol level or a reduced level of intestinal absorption of sterol or 5α-stanol.
3. The mouse of embodiment 1 wherein the region of endogenous, chromosomal *NPC1L1* deleted corresponds to nucleotides 790-998 of the nucleotide sequence set forth in SEQ ID NO: 45.
4. An offspring or progeny of the mouse of embodiment 1 wherein the offspring or progeny has inherited a mutated *NPC1L1* allele of said mouse.
5. A method for screening a sample for an intestinal sterol or 5α-stanol absorption antagonist comprising:
   (a) feeding a sterol or 5α-stanol-containing substance to a first and second mouse comprising a functional *NPC1L1* gene and to a third, mutant mouse of embodiment 1;
   (b) administering the sample to the first mouse but not the second mouse;
   (c) measuring the amount of sterol or 5α-stanol absorption in the intestine of said first, second and third mouse; and
   (d) comparing the levels of intestinal sterol or 5α-stanol absorption in said first, second and third mouse;
   wherein the sample is determined to contain the intestinal sterol or 5α-stanol absorption antagonist when the level of intestinal sterol or 5α-stanol absorption in the first mouse and third mouse are less than the amount of intestinal sterol or 5α-stanol absorption in the second mouse.
6. The method of embodiment 5 wherein the sterol is cholesterol.
7. The method of embodiment 6 wherein the cholesterol is radiolabeled.
8. The method of embodiment 5 wherein the level of sterol or 5α-stanol cholesterol absorption is determined by measuring the level of serum sterol or 5α-stanol in the mice.
9. A method for inhibiting NPC1L1 mediated sterol or 5α-stanol uptake, in a subject, by administering, to the subject, a substance identified by a method for identifying an antagonist of NPC1L1 comprising the steps of:
   (a) contacting a host cell expressing a polypeptide comprising an amino acid sequence selected from SEQ ID NOs: 2, 4 and 12 or a functional fragment thereof on a cell surface, in the presence of a known amount of detectably labeled ezetimibe, with a sample to be tested for the presence of the antagonist; and
   (b) measuring the amount of detectably labeled ezetimibe specifically bound, directly or indirectly, to the polypeptide;
      wherein an NPC1L1 antagonist in the sample is identified by measuring substantially reduced direct or indirect binding of the detectably labeled ezetimibe to the polypeptide, compared to what would be measured in the absence of such an antagonist.
10. A method for inhibiting NPC1L1 mediated sterol or 5α-stanol uptake, in a subject, by administering, to the subject, a substance identified by a method for identifying an antagonist of NPCIL1 comprising the steps of:
   (a) placing, in an aqueous suspension, a plurality of support particles, impregnated with a fluorescer, to which a host cell expressing a polypeptide comprising an amino acid sequence selected from SEQ ID NOs: 2, 4 and 12 or a functional fragment thereof on a cell surface are attached;
   (b) adding, to the suspension, radiolabeled ezetimibe and a sample to be tested for the presence of the antagonist, wherein the radiolabel emits radiation energy capable of activating the fluorescer upon direct or indirect binding of the ezetimibe to the polypeptide to produce light energy, whereas radiolabeled ezetimibe that does not directly or indirectly bind to the polypeptide is, generally, too far removed from the support particles to enable the radioactive energy to activate the fluorescer; and
   (c) measuring the light energy emitted by the fluorescer in the suspension;
   wherein an NPC1L1 antagonist in the sample is identified by measuring substantially reduced light energy emission, compared to what would be measured in the absence of such an antagonist.
11. A method for inhibiting NPC1L1 mediated sterol or 5α-stanol uptake, in a subject, by administering, to the subject, a substance identified by a method for identifying an antagonist of NPC1L comprising the steps of:
   (a) contacting a host cell expressing a polypeptide comprising an amino acid sequence selected from SEQ ID NOs: 2, 4 and 12 or a functional fragment thereof on a cell surface with a detectably labeled sterol or 5α-stanol and with a sample to be tested for the presence of the antagonist; and
   (b) measuring the amount of detectably labeled sterol or 5α-stanol in the cell;
   wherein an NPC1L1 antagonist in the sample is identified by measuring substantially reduced detectably labeled sterol or 5α-stanol within the host cell, compared to what would be measured in the absence of such an antagonist..
12. A method for inhibiting NPC1L1 mediated sterol or 5α-stanol uptake, in a subject, by administering, to the subject, a substance identified by the method of embodiment 5.
13. A kit comprising:
   (a) ezetimibe in a pharmaceutical dosage form; and
   (b) information indicating that NPC1L1 is a target of ezetimibe.
14. The kit of embodiment 13 wherein the dosage form is a tablet comprising 10 mg ezetimibe.
15. The kit of embodiment 13 further comprising simvastatin in a pharmaceutical dosage form.
16. The kit of embodiment 15 wherein the simvastatin in pharmaceutical dosage form comprises 5 mg, 10 mg, 20 mg, 40 mg or 80mg simvastatin.
17. The kit of embodiment 15 wherein the simvastatin in pharmaceutical dosage form and the ezetimibe in pharmaceutical dosage form are associated in a single pill or tablet.
18. A method for decreasing the level of intestinal sterol or 5α-stanol absorption in a subject comprising reducing the level of expression of NPC1L1 in the subject.
19. The method of embodiment 18 wherein the subject is a mouse, rat or human.
20. The method of embodiment 18 wherein the level of expression of NPC1L1 in the subject is reduced by mutating *NPC1L1* in the subject.
21. The method of embodiment 18 wherein the sterol is cholesterol.
22. A method for identifying an antagonist of NPC1L1 comprising:
   (a) contacting a host cell expressing a polypeptide comprising an amino acid sequence selected from SEQ ID NOs: 2, 4 and 12 or a functional fragment thereof on a cell surface, in the presence of a known amount of a detectably labeled substituted azetidinone, with a sample to be tested for the presence of the antagonist; and
   (b) measuring the amount of detectably labeled substituted azetidinone specifically bound, directly or indirectly, to the polypeptide;
      wherein an NPC1L1 antagonist in the sample is identified by measuring substantially reduced direct or indirect binding of the detectably labeled substituted azetidinone to the polypeptide, compared to what would be measured in the absence of such an antagonist.
23. A kit comprising:
   (a) a substituted azetidinone in a pharmaceutical dosage form; and
   (b) information indicating that NPC1L1 is a target of the substituted azetidinone.
24. An isolated mammalian cell which lacks a gene which encodes a functional NPC1L1 protein.
25. The cell of embodiment 24 isolated from a mutant mouse comprising a homozygous mutation of endogenous, chromosomal *NPC1L1* wherein the mouse does not produce any functional NPC1L1 protein.
26. The cell of embodiment 25 wherein the mutation is of a gene which, when unmutated, encodes an amino acid sequence of SEQ ID NO: 12.
27. The cell of embodiment 24 isolated or derived from the duodenum, gall bladder, liver, small intestine or stomach tissue.
28. The cell of embodiment 27 which is an enterocyte.
29. An isolated polypeptide comprising 42 or more contiguous amino acids from an amino acid sequence selected from SEQ ID NOs: 2 and 12.
30. An isolated polypeptide comprising an amino acid sequence selected from SEQ ID NOs: 2, 4 and 12.
31. An isolated polynucleotide encoding a polypeptide of embodiment 29.
32. An isolated polynucleotide comprising a nucleotide sequence selected from SEQ ID NOs: 1, 3 and 11.
33. A recombinant vector comprising the polynucleotide of embodiment 31.
34. A host cell comprising the vector of embodiment 33.
35. An isolated antibody which specifically binds to a polypeptide comprising 42 or more contiguous amino acids from an amino acid sequence selected from SEQ ID NOs: 2 and 12 or to an isolated polypeptide comprising an amino acid of SEQ ID NO: 4.
36. An isolated antibody which specifically binds to a polypeptide comprising an amino acid sequence selected from SEQ ID NOs: 39-42.
37. A method for making a polypeptide comprising culturing a host cell of embodiment 34 under conditions in which the polynucleotide is expressed.
38. The method of embodiment 37 wherein the polypeptide is isolated from the culture.
39. A method for identifying an antagonist of NPC1L1 comprising:
   (a) contacting a host cell expressing a polypeptide comprising an amino acid sequence selected from SEQ ID NOs: 2, 4 and 12 or a functional fragment thereof on a cell surface, in the presence of a known amount of detectably labeled ezetimibe, with a sample to be tested for the presence of the antagonist; and
   (b) measuring the amount of detectably labeled ezetimibe specifically bound, directly or indirectly, to the polypeptide;
      wherein an NPC1L1 antagonist in the sample is identified by measuring substantially reduced direct or indirect binding of the detectably labeled ezetimibe to the polypeptide, compared to what would be measured in the absence of such an antagonist.
40. A method for identifying an antagonist of NPC1L1 comprising:
   (a) placing, in an aqueous suspension, a plurality of support particles, impregnated with a fluorescer, to which a host cell expressing a polypeptide comprising an amino acid sequence selected from SEQ ID NOs: 2, 4 and 12 or a functional fragment thereof on a cell surface are attached;
   (b) adding, to the suspension, radiolabeled ezetimibe and a sample to be tested for the presence of the antagonist, wherein the radiolabel emits radiation energy capable of activating the fluorescer upon direct or indirect binding of the ezetimibe to the polypeptide to produce light energy, whereas radiolabeled ezetimibe that does not directly or indirectly bind to the polypeptide is, generally, too far removed from the support particles to enable the radioactive energy to activate the fluorescer; and
   (c) measuring the light energy emitted by the fluorescer in the suspension;
      wherein an NPC1L1 antagonist in the sample is identified by measuring substantially reduced light energy emission, compared to what would be measured in the absence of such an antagonist.
41. The method of embodiment 40 wherein the fluorescer is selected from yttrium silicate, yttrium oxide, diphenyloxazole and polyvinyltoluene.
42. A method of embodiment 39 wherein the ezetimibe is labeled with a radiolabel selected from ³H and ¹²⁵I.
43. A method of embodiment 40 wherein the ezetimibe is labeled with a radiolabel selected from ³H and ¹²⁵I.
44. A method for identifying an antagonist of NPC1L1 comprising:
   (a) contacting a host cell expressing a polypeptide comprising an amino acid sequence selected from SEQ ID NOs: 2, 4 and 12 or a functional fragment thereof on a cell surface with a detectably labeled sterol or 5α-stanol and with a sample to be tested for the presence of the antagonist; and
   (b) measuring the amount of detectably labeled sterol or 5α-stanol in the cell;
   wherein an NPC1L1 antagonist in the sample is identified by measuring substantially reduced detectably labeled sterol or 5α-stanol within the host cell, compared to what would be measured in the absence of such an antagonist.
45. The method of embodiment 44 wherein the sterol or 5α-stanol is detectably labeled with a radiolabel selected from ³H, ¹⁴C and ¹²⁵I.
46. The method of embodiment 44 wherein the sterol is cholesterol.
47. A method according to embodiment 39 wherein the host cell is selected from a chinese hamster ovary (CHO) cell, a J774 cell, a macrophage cell and a Caco2 cell.
48. A method according to embodiment 40 wherein the host cell is selected from a chinese hamster ovary (CHO) cell, a J774 cell, a macrophage cell and a Caco2 cell.
49. A method according to embodiment 44 wherein the host cell is selected from a chinese hamster ovary (CHO) cell, a J774 cell, a macrophage cell and a Caco2 cell.

SEQUENCE LISTING

<110> Schering Corporation

<120> NPC1L1 (NPC3) AND METHODS OF USE THEREOF

<130> JB01603-K3-WI

<140>

<141> 2003-12-16

<150> 60/397,442

<151> 2002-07-19

<150> 10/621,758

<151> 2003-07-17

<150> 10/646,301

<151> 2003-08-22

<150> 10/663,208

<151> 2003-09-16

<160> 51

<170> PatentIn version 3.1

<210> 1

<211> 3996

<212> DNA

<213> Rattus sp.

<220>

<221> CDS

<222> (1)..(3996)

<223>

<400> 1
atg gca gct gcc tgg ctg gga tgg ctg ctc tgg gcc ctg ctc ctg agc 48
Met Ala Ala Ala Trp Leu Gly Trp Leu Leu Trp Ala Leu Leu Leu Ser
1               5                   10                  15

gcg gcc cag ggt gag cta tac aca ccc aaa cac gaa gct ggg gtc tgc 96
Ala Ala Gln Gly Glu Leu Tyr Thr Pro Lys His Glu Ala Gly Val Cys
            20                  25                  30

acc ttt tac gaa gag tgc ggg aaa aac cca gag ctc tct gga ggc ctc 144
Thr Phe Tyr Glu Glu Cys Gly Lys Asn Pro Glu Leu Ser Gly Gly Leu
        35                  40                  45

acg tca cta tcc aat gta tcc tgc ctg tct aac acc ccg gcc cgc cac 192
Thr Ser Leu Ser Asn Val Ser Cys Leu Ser Asn Thr Pro Ala Arg His
    50                  55                  60

gtc acg ggt gaa cac ctg gct ctt ctc cag cgc atc tgt ccc cgc ctg 240
Val Thr Gly Glu His Leu Ala Leu Leu Gln Arg Ile Cys Pro Arg Leu
65                  70                  75                  80

tac aac ggc ccc aat acc act ttt gcc tgt tgc tct acc aag cag ctg 288
Tyr Asn Gly Pro Asn Thr Thr Phe Ala Cys Cys Ser Thr Lys Gln Leu
                85                  90                  95

ctg tcc tta gaa agc agc atg tcc atc acc aag gcc ctt ctc acg cgc 336
Leu Ser Leu Glu Ser Ser Met Ser Ile Thr Lys Ala Leu Leu Thr Arg
            100                 105                 110

tgc ccg gcc tgc tct gac aat ttt gtg agc tta cac tgc cac aac act 384
Cys Pro Ala Cys Ser Asp Asn Phe Val Ser Leu His Cys His Asn Thr
        115                 120                 125

tgc agc cct gac cag agc ctc ttc atc aac gtc acc cgg gtg gtt gag 432
Cys Ser Pro Asp Gln Ser Leu Phe Ile Asn Val Thr Arg Val Val Glu
    130                 135                 140

cgg ggc gct gga gag cct cct gcc gtg gtg gcc tat gag gcc ttt tat 480
Arg Gly Ala Gly Glu Pro Pro Ala Val Val Ala Tyr Glu Ala Phe Tyr
145                 150                 155                 160

cag cgc agc ttt gct gag aag gcc tat gag tcc tgc agc cag gtg cgc 528
Gln Arg Ser Phe Ala Glu Lys Ala Tyr Glu Ser Cys Ser Gln Val Arg
                165                 170                 175

atc cct gcg gcc gct tcc ttg gcc gtg ggc agc atg tgt gga gtg tat 576
Ile Pro Ala Ala Ala Ser Leu Ala Val Gly Ser Met Cys Gly Val Tyr
            180                 185                 190

ggc tcc gcc ctc tgc aat gct cag cgc tgg ctc aac ttc caa gga gac 624
Gly Ser Ala Leu Cys Asn Ala Gln Arg Trp Leu Asn Phe Gln Gly Asp
        195                 200                 205

aca ggg aat ggc ctg gct ccg ctg gat atc acc ttc cac ctc ttg gag 672
Thr Gly Asn Gly Leu Ala Pro Leu Asp Ile Thr Phe His Leu Leu Glu
    210                 215                 220

cct ggc cag gcc cta ccg gat ggg atc cag cca ctg aat ggg aag atc 720
Pro Gly Gln Ala Leu Pro Asp Gly Ile Gln Pro Leu Asn Gly Lys Ile
225                 230                 235                 240

gca ccc tgc aac gag tct cag ggt gat gac tca gca gtc tgc tcc tgc 768
Ala Pro Cys Asn Glu Ser Gln Gly Asp Asp Ser Ala Val Cys Ser Cys
                245                 250                 255

cag gac tgt gcg gcg tcc tgc cct gtc atc cct ccg ccc gag gcc ttg 816
Gln Asp Cys Ala Ala Ser Cys Pro Val Ile Pro Pro Pro Glu Ala Leu
            260                 265                 270

cgc cct tcc ttc tac atg ggt cgc atg cca ggc tgg ctg gcc ctc atc 864
Arg Pro Ser Phe Tyr Met Gly Arg Met Pro Gly Trp Leu Ala Leu Ile
        275                 280                 285

atc atc ttc act gct gtc ttt gtg ttg ctc tct gca gtc ctt gtg cgt 912
Ile Ile Phe Thr Ala Val Phe Val Leu Leu Ser Ala Val Leu Val Arg
    290                 295                 300

ctc cga gtg gtt tcc aac agg aac aag aac aag gca gaa ggc ccc cag 960
Leu Arg Val Val Ser Asn Arg Asn Lys Asn Lys Ala Glu Gly Pro Gln
305                 310                 315                 320

gaa gcc ccc aaa ctc cct cat aag cac aaa ctc tca ccc cat acc atc 1008
Glu Ala Pro Lys Leu Pro His Lys His Lys Leu Ser Pro His Thr Ile
                325                 330                 335

ctg ggc cgg ttc ttc cag aac tgg ggc aca agg gtg gcc tcg tgg cca 1056
Leu Gly Arg Phe Phe Gln Asn Trp Gly Thr Arg Val Ala Ser Trp Pro
            340                 345                 350

ctc acc gtc tta gca ctg tcc ttc atc gtt gtg ata gcc tta gca gca 1104
Leu Thr Val Leu Ala Leu Ser Phe Ile Val Val Ile Ala Leu Ala Ala
        355                 360                 365

ggc ctg acc ttt att gaa ctc acc aca gac cct gtg gaa ctg tgg tcg 1152
Gly Leu Thr Phe Ile Glu Leu Thr Thr Asp Pro Val Glu Leu Trp Ser
    370                 375                 380

gcc ccc aag agc cag gcc cgg aaa gag aag tct ttc cat gat gag cat 1200
Ala Pro Lys Ser Gln Ala Arg Lys Glu Lys Ser Phe His Asp Glu His
385                 390                 395                 400

ttc ggc ccc ttc ttt cga acc aac cag att ttc gtg aca gct cgg aac 1248
Phe Gly Pro Phe Phe Arg Thr Asn Gln Ile Phe Val Thr Ala Arg Asn
                405                 410                 415

agg tcc agc tac aag tac gac tcc cta ctg cta ggg tcc aag aac ttc 1296
Arg Ser Ser Tyr Lys Tyr Asp Ser Leu Leu Leu Gly Ser Lys Asn Phe
            420                 425                 430

agt ggg atc ctg tcc ctg gac ttc ctg ctg gag ctg ctg gag ctt cag 1344
Ser Gly Ile Leu Ser Leu Asp Phe Leu Leu Glu Leu Leu Glu Leu Gln
        435                 440                 445

gag agg ctt cga cac ctg caa gtg tgg tcc cct gag gca gag cgc aac 1392
Glu Arg Leu Arg His Leu Gln Val Trp Ser Pro Glu Ala Glu Arg Asn
    450                 455                 460

atc tcc ctc cag gac atc tgc tat gcc ccc ctc aac cca tat aac acc 1440

Ile Ser Leu Gln Asp Ile Cys Tyr Ala Pro Leu Asn Pro Tyr Asn Thr
465                 470                 475                 480

agc ctc tcc gac tgc tgt gtc aac agc ctc ctt cag tac ttc cag aac     1488
Ser Leu Ser Asp Cys Cys Val Asn Ser Leu Leu Gln Tyr Phe Gln Asn
                485                 490                 495

aac cgc acc ctc ctg atg ctc acg gcc aac cag act ctg aat ggc cag     1536
Asn Arg Thr Leu Leu Met Leu Thr Ala Asn Gln Thr Leu Asn Gly Gln
            500                 505                 510

acc tcc ctg gtg gac tgg aag gac cat ttc ctc tac tgt gca aat gcc     1584
Thr Ser Leu Val Asp Trp Lys Asp His Phe Leu Tyr Cys Ala Asn Ala
        515                 520                 525

cct ctc acg ttc aaa gat ggc acg tct ctg gcc ctg agc tgc atg gct     1632
Pro Leu Thr Phe Lys Asp Gly Thr Ser Leu Ala Leu Ser Cys Met Ala
    530                 535                 540

gac tac ggg gct cct gtc ttc ccc ttc ctt gct gtt ggg gga tac caa     1680
Asp Tyr Gly Ala Pro Val Phe Pro Phe Leu Ala Val Gly Gly Tyr Gln
545                 550                 555                 560

ggc acg gac tat tcc gag gca gaa gcg ctg atc ata acc ttc tct ctc     1728
Gly Thr Asp Tyr Ser Glu Ala Glu Ala Leu Ile Ile Thr Phe Ser Leu
                565                 570                 575

aat aac tac ccc gct gat gat ccc cgc atg gcc cag gcc aag ctc tgg     1776
Asn Asn Tyr Pro Ala Asp Asp Pro Arg Met Ala Gln Ala Lys Leu Trp
            580                 585                 590

gag gag gct ttc ttg aag gaa atg gaa tcc ttc cag agg aac aca agt     1824
Glu Glu Ala Phe Leu Lys Glu Met Glu Ser Phe Gln Arg Asn Thr Ser
        595                 600                 605

gac aag ttc cag gtt gcg ttc tca gct gag cgc tct ctg gag gat gag     1872
Asp Lys Phe Gln Val Ala Phe Ser Ala Glu Arg Ser Leu Glu Asp Glu
    610                 615                 620

atc aac cgc acc acc atc cag gac ctg cct gtc ttt gcc gtc agc tac     1920
Ile Asn Arg Thr Thr Ile Gln Asp Leu Pro Val Phe Ala Val Ser Tyr
625                 630                 635                 640

att atc gtc ttc ctg tac atc tcc ctg gcc ctg ggc agc tac tcc aga     1968
Ile Ile Val Phe Leu Tyr Ile Ser Leu Ala Leu Gly Ser Tyr Ser Arg
                645                 650                 655

tgc agc cga gta gcg gtg gag tcc aag gct act ctg ggc cta ggt ggg     2016
Cys Ser Arg Val Ala Val Glu Ser Lys Ala Thr Leu Gly Leu Gly Gly
            660                 665                 670

gtg att gtt gtg ctg gga gca gtt ctg gct gcc atg ggc ttc tac tcc     2064
Val Ile Val Val Leu Gly Ala Val Leu Ala Ala Met Gly Phe Tyr Ser
        675                 680                 685

tac ctg ggt gtc ccc tct tct ctg gtt atc atc caa gtg gta cct ttc     2112
Tyr Leu Gly Val Pro Ser Ser Leu Val Ile Ile Gln Val Val Pro Phe
    690                 695                 700

ctg gtg cta gct gtg gga gct gac aac atc ttc atc ttt gtt ctt gag     2160
Leu Val Leu Ala Val Gly Ala Asp Asn Ile Phe Ile Phe Val Leu Glu

705                 710                 715                 720

tac cag agg cta cct agg atg cct ggg gaa cag cga gag gct cac att     2208
Tyr Gln Arg Leu Pro Arg Met Pro Gly Glu Gln Arg Glu Ala His Ile
                725                 730                 735

ggc cgc acc ctg ggc agt gtg gcc ccc agc atg ctg ctg tgc agc ctc     2256
Gly Arg Thr Leu Gly Ser Val Ala Pro Ser Met Leu Leu Cys Ser Leu
            740                 745                 750

tct gag gcc atc tgc ttc ttt cta ggg gcc ctg acc ccc atg cca gct     2304
Ser Glu Ala Ile Cys Phe Phe Leu Gly Ala Leu Thr Pro Met Pro Ala
        755                 760                 765

gtg agg acc ttc gcc ttg acc tct ggc tta gca att atc ctc gac ttc     2352
Val Arg Thr Phe Ala Leu Thr Ser Gly Leu Ala Ile Ile Leu Asp Phe
    770                 775                 780

ctg ctc cag atg act gcc ttt gtg gcc ctg ctc tcc ctg gat agc aag     2400
Leu Leu Gln Met Thr Ala Phe Val Ala Leu Leu Ser Leu Asp Ser Lys
785                 790                 795                 800

agg cag gag gcc tct cgc ccg gat gtc tta tgc tgc ttt tca acc cgg     2448
Arg Gln Glu Ala Ser Arg Pro Asp Val Leu Cys Cys Phe Ser Thr Arg
                805                 810                 815

aag ctg ccc cca cct aaa gaa aaa gaa ggc ctc tta ctc cgc ttc ttc     2496
Lys Leu Pro Pro Pro Lys Glu Lys Glu Gly Leu Leu Leu Arg Phe Phe
            820                 825                 830

cgc aag ata tac gct cct ttc ctg ctg cac aga ttc atc cgc cct gtt     2544
Arg Lys Ile Tyr Ala Pro Phe Leu Leu His Arg Phe Ile Arg Pro Val
        835                 840                 845

gtg atg ctg ctg ttt ctg acc ctg ttt gga gca aat ctc tac tta atg     2592
Val Met Leu Leu Phe Leu Thr Leu Phe Gly Ala Asn Leu Tyr Leu Met
    850                 855                 860

tgc aac atc aac gtg ggg cta gac cag gag ctg gct ctg ccc aag gac     2640
Cys Asn Ile Asn Val Gly Leu Asp Gln Glu Leu Ala Leu Pro Lys Asp
865                 870                 875                 880

tcg tac ttg ata gac tac ttc ctc ttt ctg aac cga tac ctt gaa gtg     2688
Ser Tyr Leu Ile Asp Tyr Phe Leu Phe Leu Asn Arg Tyr Leu Glu Val
                885                 890                 895

ggg cct cca gtg tac ttt gtc acc acc tcg ggc ttc aac ttc tcc agc     2736
Gly Pro Pro Val Tyr Phe Val Thr Thr Ser Gly Phe Asn Phe Ser Ser
            900                 905                 910

gag gca ggc atg aac gcc act tgc tct agc gca ggc tgt aag agc ttc     2784
Glu Ala Gly Met Asn Ala Thr Cys Ser Ser Ala Gly Cys Lys Ser Phe
        915                 920                 925

tcc cta acc cag aaa atc cag tat gcc agt gaa ttc cct gac cag tct     2832
Ser Leu Thr Gln Lys Ile Gln Tyr Ala Ser Glu Phe Pro Asp Gln Ser
    930                 935                 940

tac gtg gct att gct gca tcc tcc tgg gta gat gac ttc atc gac tgg     2880
Tyr Val Ala Ile Ala Ala Ser Ser Trp Val Asp Asp Phe Ile Asp Trp
945                 950                 955                 960

ctg acc ccg tcc tcc tcc tgc tgt cgc ctt tat ata cgt ggc ccc cat 2928
Leu Thr Pro Ser Ser Ser Cys Cys Arg Leu Tyr Ile Arg Gly Pro His
                965                 970                 975

aag gat gag ttc tgt ccc tca acg gat act tcc ttc aac tgc tta aaa 2976
Lys Asp Glu Phe Cys Pro Ser Thr Asp Thr Ser Phe Asn Cys Leu Lys
            980                 985                 990

aac tgc atg aac cgc act ctg ggt cct gtg agg ccc aca gcg gaa cag 3024
Asn Cys Met Asn Arg Thr Leu Gly Pro Val Arg Pro Thr Ala Glu Gln
        995                 1000                1005

ttt cat aag tac ctg ccc tgg ttc ctg aat gat ccg ccc aat atc 3069
Phe His Lys Tyr Leu Pro Trp Phe Leu Asn Asp Pro Pro Asn Ile
    1010                1015                1020

aga tgt ccc aaa ggg ggt cta gca gcg tat aga acg tct gtg aat 3114
Arg Cys Pro Lys Gly Gly Leu Ala Ala Tyr Arg Thr Ser Val Asn
    1025                1030                1035

ttg agc tca gat ggc cag gtt ata gcc tcc cag ttc atg gcc tac 3159
Leu Ser Ser Asp Gly Gln Val Ile Ala Ser Gln Phe Met Ala Tyr
    1040                1045                1050

cac aag ccc tta agg aac tca cag gac ttc aca gaa gct ctc cgg 3204
His Lys Pro Leu Arg Asn Ser Gln Asp Phe Thr Glu Ala Leu Arg
    1055                1060                1065

gcg tcc cgg ttg cta gca gcc aac atc aca gct gac cta cgg aag 3249
Ala Ser Arg Leu Leu Ala Ala Asn Ile Thr Ala Asp Leu Arg Lys
    1070                1075                1080

gtg cct ggg aca gat cca aac ttt gag gtc ttc cct tac acg atc 3294
Val Pro Gly Thr Asp Pro Asn Phe Glu Val Phe Pro Tyr Thr Ile
    1085                1090                1095

tcc aac gtg ttc tac cag caa tac ctg acg gtc ctt cct gag gga 3339
Ser Asn Val Phe Tyr Gln Gln Tyr Leu Thr Val Leu Pro Glu Gly
    1100                1105                1110

atc ttc acc ctt gct ctt tgc ttt gtg ccc acc ttt gtt gtc tgc 3384
Ile Phe Thr Leu Ala Leu Cys Phe Val Pro Thr Phe Val Val Cys
    1115                1120                1125

tac ctc cta ctg ggc ctg gac atg tgc tca ggg atc ctc aac cta 3429
Tyr Leu Leu Leu Gly Leu Asp Met Cys Ser Gly Ile Leu Asn Leu
    1130                1135                1140

ctc tcc atc att atg att ctc gtg gac acc att ggc ctc atg gct 3474
Leu Ser Ile Ile Met Ile Leu Val Asp Thr Ile Gly Leu Met Ala
    1145                1150                1155

gtg tgg ggt atc agc tat aat gcg gta tcc ctc atc aac ctt gtc 3519
Val Trp Gly Ile Ser Tyr Asn Ala Val Ser Leu Ile Asn Leu Val
    1160                1165                1170

acg gca gtg ggc atg tct gtg gag ttt gtg tcc cac atc act cgg 3564
Thr Ala Val Gly Met Ser Val Glu Phe Val Ser His Ile Thr Arg
    1175                1180                1185

tcc ttt gct gta agc acc aag cct acc cgg ctg gag agg gct aaa 3609
Ser Phe Ala Val Ser Thr Lys Pro Thr Arg Leu Glu Arg Ala Lys
    1190                1195                1200

gat gct act gtc ttc atg ggc agt gcg gtg ttt gct gga gtg gcc 3654
Asp Ala Thr Val Phe Met Gly Ser Ala Val Phe Ala Gly Val Ala
    1205                1210                1215

atg acc aac ttc cca ggc atc ctc atc ttg ggc ttt gcc caa gcc 3699
Met Thr Asn Phe Pro Gly Ile Leu Ile Leu Gly Phe Ala Gln Ala
    1220                1225                1230

cag ctt att cag atc ttc ttc ttc cgc ctc aac ctt ctg atc acc 3744
Gln Leu Ile Gln Ile Phe Phe Phe Arg Leu Asn Leu Leu Ile Thr
    1235                1240                1245

ttg ctg ggt ctg ctg cat ggc ctg gtc ttc ctg ccg gtt gtc ctc 3789
Leu Leu Gly Leu Leu His Gly Leu Val Phe Leu Pro Val Val Leu
    1250                1255                1260

agc tat ctg gga cca gat gtt aac caa gct ctg gta cag gag gag 3834
Ser Tyr Leu Gly Pro Asp Val Asn Gln Ala Leu Val Gln Glu Glu
    1265                1270                1275

aaa cta gcc agc gag gca gca gtg gcc cca gag cct tct tgc cca 3879
Lys Leu Ala Ser Glu Ala Ala Val Ala Pro Glu Pro Ser Cys Pro
    1280                1285                1290

cag tac ccc tcc cct gct gat gcg gat gcc aat gtt aac tac ggc 3924
Gln Tyr Pro Ser Pro Ala Asp Ala Asp Ala Asn Val Asn Tyr Gly
    1295                1300                1305

ttt gcc cca gaa ctt gcc cac gga gct aat gct gct aga agc tct 3969
Phe Ala Pro Glu Leu Ala His Gly Ala Asn Ala Ala Arg Ser Ser
    1310                1315                1320

ttg ccc aaa agt gac caa aag ttc taa 3996
Leu Pro Lys Ser Asp Gln Lys Phe
    1325                1330

<210> 2

<211> 1331

<212> PRT

<213> Rattus sp.

<400> 2

Met Ala Ala Ala Trp Leu Gly Trp Leu Leu Trp Ala Leu Leu Leu Ser
1               5                   10                  15

Ala Ala Gln Gly Glu Leu Tyr Thr Pro Lys His Glu Ala Gly Val Cys
            20                  25                  30

Thr Phe Tyr Glu Glu Cys Gly Lys Asn Pro Glu Leu Ser Gly Gly Leu
        35                  40                  45

Thr Ser Leu Ser Asn Val Ser Cys Leu Ser Asn Thr Pro Ala Arg His
    50                  55                  60

Val Thr Gly Glu His Leu Ala Leu Leu Gln Arg Ile Cys Pro Arg Leu
65                  70                  75                  80

Tyr Asn Gly Pro Asn Thr Thr Phe Ala Cys Cys Ser Thr Lys Gln Leu
                85                  90                  95

Leu Ser Leu Glu Ser Ser Met Ser Ile Thr Lys Ala Leu Leu Thr Arg
            100                 105                 110

Cys Pro Ala Cys Ser Asp Asn Phe Val Ser Leu His Cys His Asn Thr
        115                 120                 125

Cys Ser Pro Asp Gln Ser Leu Phe Ile Asn Val Thr Arg Val Val Glu
    130                 135                 140

Arg Gly Ala Gly Glu Pro Pro Ala Val Val Ala Tyr Glu Ala Phe Tyr
145                 150                 155                 160

Gln Arg Ser Phe Ala Glu Lys Ala Tyr Glu Ser Cys Ser Gln Val Arg
                165                 170                 175

Ile Pro Ala Ala Ala Ser Leu Ala Val Gly Ser Met Cys Gly Val Tyr
            180                 185                 190

Gly Ser Ala Leu Cys Asn Ala Gln Arg Trp Leu Asn Phe Gln Gly Asp
        195                 200                 205

Thr Gly Asn Gly Leu Ala Pro Leu Asp Ile Thr Phe His Leu Leu Glu
    210                 215                 220

Pro Gly Gln Ala Leu Pro Asp Gly Ile Gln Pro Leu Asn Gly Lys Ile
225                 230                 235                 240

Ala Pro Cys Asn Glu Ser Gln Gly Asp Asp Ser Ala Val Cys Ser Cys
                245                 250                 255

Gln Asp Cys Ala Ala Ser Cys Pro Val Ile Pro Pro Pro Glu Ala Leu
            260                 265                 270

Arg Pro Ser Phe Tyr Met Gly Arg Met Pro Gly Trp Leu Ala Leu Ile

        275                 280                 285

Ile Ile Phe Thr Ala Val Phe Val Leu Leu Ser Ala Val Leu Val Arg
    290                 295                 300

Leu Arg Val Val Ser Asn Arg Asn Lys Asn Lys Ala Glu Gly Pro Gln
305                 310                 315                 320

Glu Ala Pro Lys Leu Pro His Lys His Lys Leu Ser Pro His Thr Ile
                325                 330                 335

Leu Gly Arg Phe Phe Gln Asn Trp Gly Thr Arg Val Ala Ser Trp Pro
            340                 345                 350

Leu Thr Val Leu Ala Leu Ser Phe Ile Val Val Ile Ala Leu Ala Ala
        355                 360                 365

Gly Leu Thr Phe Ile Glu Leu Thr Thr Asp Pro Val Glu Leu Trp Ser
    370                 375                 380

Ala Pro Lys Ser Gln Ala Arg Lys Glu Lys Ser Phe His Asp Glu His
385                 390                 395                 400

Phe Gly Pro Phe Phe Arg Thr Asn Gln Ile Phe Val Thr Ala Arg Asn
                405                 410                 415

Arg Ser Ser Tyr Lys Tyr Asp Ser Leu Leu Leu Gly Ser Lys Asn Phe
            420                 425                 430

Ser Gly Ile Leu Ser Leu Asp Phe Leu Leu Glu Leu Leu Glu Leu Gln
        435                 440                 445

Glu Arg Leu Arg His Leu Gln Val Trp Ser Pro Glu Ala Glu Arg Asn
    450                 455                 460

Ile Ser Leu Gln Asp Ile Cys Tyr Ala Pro Leu Asn Pro Tyr Asn Thr
465                 470                 475                 480

Ser Leu Ser Asp Cys Cys Val Asn Ser Leu Leu Gln Tyr Phe Gln Asn
                485                 490                 495

Asn Arg Thr Leu Leu Met Leu Thr Ala Asn Gln Thr Leu Asn Gly Gln
            500                 505                 510

Thr Ser Leu Val Asp Trp Lys Asp His Phe Leu Tyr Cys Ala Asn Ala
        515                 520                 525

Pro Leu Thr Phe Lys Asp Gly Thr Ser Leu Ala Leu Ser Cys Met Ala
    530                 535                 540

Asp Tyr Gly Ala Pro Val Phe Pro Phe Leu Ala Val Gly Gly Tyr Gln
545                 550                 555                 560

Gly Thr Asp Tyr Ser Glu Ala Glu Ala Leu Ile Ile Thr Phe Ser Leu
                565                 570                 575

Asn Asn Tyr Pro Ala Asp Asp Pro Arg Met Ala Gln Ala Lys Leu Trp
            580                 585                 590

Glu Glu Ala Phe Leu Lys Glu Met Glu Ser Phe Gln Arg Asn Thr Ser
        595                 600                 605

Asp Lys Phe Gln Val Ala Phe Ser Ala Glu Arg Ser Leu Glu Asp Glu
    610                 615                 620

Ile Asn Arg Thr Thr Ile Gln Asp Leu Pro Val Phe Ala Val Ser Tyr
625                 630                 635                 640

Ile Ile Val Phe Leu Tyr Ile Ser Leu Ala Leu Gly Ser Tyr Ser Arg
                645                 650                 655

Cys Ser Arg Val Ala Val Glu Ser Lys Ala Thr Leu Gly Leu Gly Gly
            660                 665                 670

Val Ile Val Val Leu Gly Ala Val Leu Ala Ala Met Gly Phe Tyr Ser
        675                 680                 685

Tyr Leu Gly Val Pro Ser Ser Leu Val Ile Ile Gln Val Val Pro Phe
    690                 695                 700

Leu Val Leu Ala Val Gly Ala Asp Asn Ile Phe Ile Phe Val Leu Glu
705                 710                 715                 720

Tyr Gln Arg Leu Pro Arg Met Pro Gly Glu Gln Arg Glu Ala His Ile
                725                 730                 735

Gly Arg Thr Leu Gly Ser Val Ala Pro Ser Met Leu Leu Cys Ser Leu
            740                 745                 750

Ser Glu Ala Ile Cys Phe Phe Leu Gly Ala Leu Thr Pro Met Pro Ala
        755                 760                 765

Val Arg Thr Phe Ala Leu Thr Ser Gly Leu Ala Ile Ile Leu Asp Phe
    770                 775                 780

Leu Leu Gln Met Thr Ala Phe Val Ala Leu Leu Ser Leu Asp Ser Lys
785                 790                 795                 800

Arg Gln Glu Ala Ser Arg Pro Asp Val Leu Cys Cys Phe Ser Thr Arg
                805                 810                 815

Lys Leu Pro Pro Pro Lys Glu Lys Glu Gly Leu Leu Leu Arg Phe Phe
            820                 825                 830

Arg Lys Ile Tyr Ala Pro Phe Leu Leu His Arg Phe Ile Arg Pro Val
        835                 840                 845

Val Met Leu Leu Phe Leu Thr Leu Phe Gly Ala Asn Leu Tyr Leu Met
    850                 855                 860

Cys Asn Ile Asn Val Gly Leu Asp Gln Glu Leu Ala Leu Pro Lys Asp
865                 870                 875                 880

Ser Tyr Leu Ile Asp Tyr Phe Leu Phe Leu Asn Arg Tyr Leu Glu Val
                885                 890                 895

Gly Pro Pro Val Tyr Phe Val Thr Thr Ser Gly Phe Asn Phe Ser Ser
            900                 905                 910

Glu Ala Gly Met Asn Ala Thr Cys Ser Ser Ala Gly Cys Lys Ser Phe
        915                 920                 925

Ser Leu Thr Gln Lys Ile Gln Tyr Ala Ser Glu Phe Pro Asp Gln Ser
    930                 935                 940

Tyr Val Ala Ile Ala Ala Ser Ser Trp Val Asp Asp Phe Ile Asp Trp
945                 950                 955                 960

Leu Thr Pro Ser Ser Ser Cys Cys Arg Leu Tyr Ile Arg Gly Pro His
                965                 970                 975

Lys Asp Glu Phe Cys Pro Ser Thr Asp Thr Ser Phe Asn Cys Leu Lys
            980                 985                 990

Asn Cys Met Asn Arg Thr Leu Gly Pro Val Arg Pro Thr Ala Glu Gln
        995                 1000                1005

Phe His Lys Tyr Leu Pro Trp Phe Leu Asn Asp Pro Pro Asn Ile
    1010                1015                1020

Arg Cys Pro Lys Gly Gly Leu Ala Ala Tyr Arg Thr Ser Val Asn
    1025                1030                1035

Leu Ser Ser Asp Gly Gln Val Ile Ala Ser Gln Phe Met Ala Tyr
    1040                1045                1050

His Lys Pro Leu Arg Asn Ser Gln Asp Phe Thr Glu Ala Leu Arg
    1055                1060                1065

Ala Ser Arg Leu Leu Ala Ala Asn Ile Thr Ala Asp Leu Arg Lys
    1070                1075                1080

Val Pro Gly Thr Asp Pro Asn Phe Glu Val Phe Pro Tyr Thr Ile
    1085                1090                1095

Ser Asn Val Phe Tyr Gln Gln Tyr Leu Thr Val Leu Pro Glu Gly
    1100                1105                1110

Ile Phe Thr Leu Ala Leu Cys Phe Val Pro Thr Phe Val Val Cys
    1115                1120                1125

Tyr Leu Leu Leu Gly Leu Asp Met Cys Ser Gly Ile Leu Asn Leu
    1130                1135                1140

Leu Ser Ile Ile Met Ile Leu Val Asp Thr Ile Gly Leu Met Ala
    1145                1150                1155

Val Trp Gly Ile Ser Tyr Asn Ala Val Ser Leu Ile Asn Leu Val
    1160                1165                1170

Thr Ala Val Gly Met Ser Val Glu Phe Val Ser His Ile Thr Arg
    1175                1180                1185

Ser Phe Ala Val Ser Thr Lys Pro Thr Arg Leu Glu Arg Ala Lys
    1190                1195                1200

Asp Ala Thr Val Phe Met Gly Ser Ala Val Phe Ala Gly Val Ala
    1205                1210                1215

Met Thr Asn Phe Pro Gly Ile Leu Ile Leu Gly Phe Ala Gln Ala
    1220                1225                1230

Gln Leu Ile Gln Ile Phe Phe Phe Arg Leu Asn Leu Leu Ile Thr

1235                    1240                    1245

Leu Leu Gly Leu Leu His Gly Leu Val Phe Leu Pro Val Val Leu
    1250                    1255                    1260

Ser Tyr Leu Gly Pro Asp Val Asn Gln Ala Leu Val Gln Glu Glu
    1265                    1270                    1275

Lys Leu Ala Ser Glu Ala Ala Val Ala Pro Glu Pro Ser Cys Pro
    1280                    1285                    1290

Gln Tyr Pro Ser Pro Ala Asp Ala Asp Ala Asn Val Asn Tyr Gly
    1295                    1300                    1305

Phe Ala Pro Glu Leu Ala His Gly Ala Asn Ala Ala Arg Ser Ser
    1310                    1315                    1320

Leu Pro Lys Ser Asp Gln Lys Phe
    1325                    1330

<210>  3

<211>  3999

<212>  DNA

<213>  Homo sapiens

<220>

<221>  CDS

<222>  (1)..(3999)

<223>

<400>  3
atg gcg gag gcc ggc ctg agg ggc tgg ctg ctg tgg gcc ctg ctc ctg       48
Met Ala Glu Ala Gly Leu Arg Gly Trp Leu Leu Trp Ala Leu Leu Leu
1               5                   10                  15

cgc ttg gcc cag agt gag cct tac aca acc atc cac cag cct ggc tac       96
Arg Leu Ala Gln Ser Glu Pro Tyr Thr Thr Ile His Gln Pro Gly Tyr
            20                  25                  30

tgc gcc ttc tat gac gaa tgt ggg aag aac cca gag ctg tct gga agc      144
Cys Ala Phe Tyr Asp Glu Cys Gly Lys Asn Pro Glu Leu Ser Gly Ser
        35                  40                  45

ctc atg aca ctc tcc aac gtg tcc tgc ctg tcc aac acg ccg gcc cgc      192

Leu Met Thr Leu Ser Asn Val Ser Cys Leu Ser Asn Thr Pro Ala Arg
    50                  55                  60

aag atc aca ggt gat cac ctg atc cta tta cag aag atc tgc ccc cgc      240
Lys Ile Thr Gly Asp His Leu Ile Leu Leu Gln Lys Ile Cys Pro Arg
65                  70                  75                  80

ctc tac acc ggc ccc aac acc caa gcc tgc tgc tcc gcc aag cag ctg      288
Leu Tyr Thr Gly Pro Asn Thr Gln Ala Cys Cys Ser Ala Lys Gln Leu
                85                  90                  95

gta tca ctg gaa gcg agt ctg tcg atc acc aag gcc ctc ctc acc cgc      336
Val Ser Leu Glu Ala Ser Leu Ser Ile Thr Lys Ala Leu Leu Thr Arg
            100                 105                 110

tgc cca gcc tgc tct gac aat ttt gtg aac ctg cac tgc cac aac acg      384
Cys Pro Ala Cys Ser Asp Asn Phe Val Asn Leu His Cys His Asn Thr
        115                 120                 125

tgc agc ccc aat cag agc ctc ttc atc aat gtg acc cgc gtg gcc cag      432
Cys Ser Pro Asn Gln Ser Leu Phe Ile Asn Val Thr Arg Val Ala Gln
    130                 135                 140

cta ggg gct gga caa ctc cca gct gtg gtg gcc tat gag gcc ttc tac      480
Leu Gly Ala Gly Gln Leu Pro Ala Val Val Ala Tyr Glu Ala Phe Tyr
145                 150                 155                 160

cag cat agc ttt gcc gag cag agc tat gac tcc tgc agc cgt gtg cgc      528
Gln His Ser Phe Ala Glu Gln Ser Tyr Asp Ser Cys Ser Arg Val Arg
                165                 170                 175

gtc cct gca gct gcc acg ctg gct gtg ggc acc atg tgt ggc gtg tat      576
Val Pro Ala Ala Ala Thr Leu Ala Val Gly Thr Met Cys Gly Val Tyr
            180                 185                 190

ggc tct gcc ctt tgc aat gcc cag cgc tgg ctc aac ttc cag gga gac      624
Gly Ser Ala Leu Cys Asn Ala Gln Arg Trp Leu Asn Phe Gln Gly Asp
        195                 200                 205

aca ggc aat ggt ctg gcc cca ctg gac atc acc ttc cac ctc ttg gag      672
Thr Gly Asn Gly Leu Ala Pro Leu Asp Ile Thr Phe His Leu Leu Glu
    210                 215                 220

cct ggc cag gcc gtg ggg agt ggg att cag cct ctg aat gag ggg gtt      720
Pro Gly Gln Ala Val Gly Ser Gly Ile Gln Pro Leu Asn Glu Gly Val
225                 230                 235                 240

gca cgt tgc aat gag tcc caa ggt gac gac gtg gcg acc tgc tcc tgc      768
Ala Arg Cys Asn Glu Ser Gln Gly Asp Asp Val Ala Thr Cys Ser Cys
                245                 250                 255

caa gac tgt gct gca tcc tgt cct gcc ata gcc cgc ccc cag gcc ctc      816
Gln Asp Cys Ala Ala Ser Cys Pro Ala Ile Ala Arg Pro Gln Ala Leu
            260                 265                 270

gac tcc acc ttc tac ctg ggc cag atg ccg ggc agt ctg gtc ctc atc      864
Asp Ser Thr Phe Tyr Leu Gly Gln Met Pro Gly Ser Leu Val Leu Ile
        275                 280                 285

atc atc ctc tgc tct gtc ttc gct gtg gtc acc atc ctg ctt gtg gga      912
Ile Ile Leu Cys Ser Val Phe Ala Val Val Thr Ile Leu Leu Val Gly

290                 295                 300

ttc cgt gtg gcc ccc gcc agg gac aaa agc aag atg gtg gac ccc aag     960
Phe Arg Val Ala Pro Ala Arg Asp Lys Ser Lys Met Val Asp Pro Lys
305                 310                 315                 320

aag ggc acc agc ctc tct gac aag ctc agc ttc tcc acc cac acc ctc     1008
Lys Gly Thr Ser Leu Ser Asp Lys Leu Ser Phe Ser Thr His Thr Leu
                325                 330                 335

ctt ggc cag ttc ttc cag ggc tgg ggc acg tgg gtg gct tcg tgg cct     1056
Leu Gly Gln Phe Phe Gln Gly Trp Gly Thr Trp Val Ala Ser Trp Pro
            340                 345                 350

ctg acc atc ttg gtg cta tct gtc atc ccg gtg gtg gcc ttg gca gcg     1104
Leu Thr Ile Leu Val Leu Ser Val Ile Pro Val Val Ala Leu Ala Ala
        355                 360                 365

ggc ctg gtc ttt aca gaa ctc act acg gac ccc gtg gag ctg tgg tcg     1152
Gly Leu Val Phe Thr Glu Leu Thr Thr Asp Pro Val Glu Leu Trp Ser
    370                 375                 380

gcc ccc aac agc caa gcc cgg agt gag aaa gct ttc cat gac cag cat     1200
Ala Pro Asn Ser Gln Ala Arg Ser Glu Lys Ala Phe His Asp Gln His
385                 390                 395                 400

ttc ggc ccc ttc ttc cga acc aac cag gtg atc ctg acg gct cct aac     1248
Phe Gly Pro Phe Phe Arg Thr Asn Gln Val Ile Leu Thr Ala Pro Asn
                405                 410                 415

cgg tcc agc tac agg tat gac tct ctg ctg ctg ggg ccc aag aac ttc     1296
Arg Ser Ser Tyr Arg Tyr Asp Ser Leu Leu Leu Gly Pro Lys Asn Phe
            420                 425                 430

agc gga atc ctg gac ctg gac ttg ctg ctg gag ctg cta gag ctg cag     1344
Ser Gly Ile Leu Asp Leu Asp Leu Leu Leu Glu Leu Leu Glu Leu Gln
        435                 440                 445

gag agg ctg cgg cac ctc cag gta tgg tcg ccc gaa gca cag cgc aac     1392
Glu Arg Leu Arg His Leu Gln Val Trp Ser Pro Glu Ala Gln Arg Asn
    450                 455                 460

atc tcc ctg cag gac atc tgc tac gcc ccc ctc aat ccg gac aat acc     1440
Ile Ser Leu Gln Asp Ile Cys Tyr Ala Pro Leu Asn Pro Asp Asn Thr
465                 470                 475                 480

agt ctc tac gac tgc tgc atc aac agc ctc ctg cag tat ttc cag aac     1488
Ser Leu Tyr Asp Cys Cys Ile Asn Ser Leu Leu Gln Tyr Phe Gln Asn
                485                 490                 495

aac cgc acg ctc ctg ctg ctc aca gcc aac cag aca ctg atg ggg cag     1536
Asn Arg Thr Leu Leu Leu Leu Thr Ala Asn Gln Thr Leu Met Gly Gln
            500                 505                 510

acc tcc caa gtc gac tgg aag gac cat ttt ctg tac tgt gcc aat gcc     1584
Thr Ser Gln Val Asp Trp Lys Asp His Phe Leu Tyr Cys Ala Asn Ala
        515                 520                 525

ccg ctc acc ttc aag gat ggc aca gcc ctg gcc ctg agc tgc atg gct     1632
Pro Leu Thr Phe Lys Asp Gly Thr Ala Leu Ala Leu Ser Cys Met Ala
    530                 535                 540

gac tac ggg gcc cct gtc ttc ccc ttc ctt gcc att ggg ggg tac aaa 1680
Asp Tyr Gly Ala Pro Val Phe Pro Phe Leu Ala Ile Gly Gly Tyr Lys
545                 550                 555                 560

gga aag gac tat tct gag gca gag gcc ctg atc atg acg ttc tcc ctc 1728
Gly Lys Asp Tyr Ser Glu Ala Glu Ala Leu Ile Met Thr Phe Ser Leu
                565                 570                 575

aac aat tac cct gcc ggg gac ccc cgt ctg gcc cag gcc aag ctg tgg 1776
Asn Asn Tyr Pro Ala Gly Asp Pro Arg Leu Ala Gln Ala Lys Leu Trp
            580                 585                 590

gag gag gcc ttc tta gag gaa atg cga gcc ttc cag cgt cgg atg gct 1824
Glu Glu Ala Phe Leu Glu Glu Met Arg Ala Phe Gln Arg Arg Met Ala
        595                 600                 605

ggc atg ttc cag gtc acg ttc acg gct gag cgc tct ctg gaa gac gag 1872
Gly Met Phe Gln Val Thr Phe Thr Ala Glu Arg Ser Leu Glu Asp Glu
    610                 615                 620

atc aat cgc acc aca gct gaa gac ctg ccc atc ttt gcc acc agc tac 1920
Ile Asn Arg Thr Thr Ala Glu Asp Leu Pro Ile Phe Ala Thr Ser Tyr
625                 630                 635                 640

att gtc ata ttc ctg tac atc tct ctg gcc ctg ggc agc tat tcc agc 1968
Ile Val Ile Phe Leu Tyr Ile Ser Leu Ala Leu Gly Ser Tyr Ser Ser
                645                 650                 655

tgg agc cga gtg atg gtg gac tcc aag gcc acg ctg ggc ctc ggc ggg 2016
Trp Ser Arg Val Met Val Asp Ser Lys Ala Thr Leu Gly Leu Gly Gly
            660                 665                 670

gtg gcc gtg gtc ctg gga gca gtc atg gct gcc atg ggc ttc ttc tcc 2064
Val Ala Val Val Leu Gly Ala Val Met Ala Ala Met Gly Phe Phe Ser
        675                 680                 685

tac ttg ggt atc cgc tcc tcc ctg gtc atc ctg caa gtg gtt cct ttc 2112
Tyr Leu Gly Ile Arg Ser Ser Leu Val Ile Leu Gln Val Val Pro Phe
    690                 695                 700

ctg gtg ctg tcc gtg ggg gct gat aac atc ttc atc ttt gtt ctc gag 2160
Leu Val Leu Ser Val Gly Ala Asp Asn Ile Phe Ile Phe Val Leu Glu
705                 710                 715                 720

tac cag agg ctg ccc cgg agg cct ggg gag cca cga gag gtc cac att 2208
Tyr Gln Arg Leu Pro Arg Arg Pro Gly Glu Pro Arg Glu Val His Ile
                725                 730                 735

ggg cga gcc cta ggc agg gtg gct ccc agc atg ctg ttg tgc agc ctc 2256
Gly Arg Ala Leu Gly Arg Val Ala Pro Ser Met Leu Leu Cys Ser Leu
            740                 745                 750

tct gag gcc atc tgc ttc ttc cta ggg gcc ctg acc ccc atg cca gct 2304
Ser Glu Ala Ile Cys Phe Phe Leu Gly Ala Leu Thr Pro Met Pro Ala
        755                 760                 765

gtg cgg acc ttt gcc ctg acc tct ggc ctt gca gtg atc ctt gac ttc 2352
Val Arg Thr Phe Ala Leu Thr Ser Gly Leu Ala Val Ile Leu Asp Phe
    770                 775                 780

ctc ctg cag atg tca gcc ttt gtg gcc ctg ctc tcc ctg gac agc aag 2400
Leu Leu Gln Met Ser Ala Phe Val Ala Leu Leu Ser Leu Asp Ser Lys
785                 790                 795                 800

agg cag gag gcc tcc cgg ttg gac gtc tgc tgc tgt gtc aag ccc cag 2448
Arg Gln Glu Ala Ser Arg Leu Asp Val Cys Cys Cys Val Lys Pro Gln
                805                 810                 815

gag ctg ccc ccg cct ggc cag gga gag ggg ctc ctg ctt ggc ttc ttc 2496
Glu Leu Pro Pro Pro Gly Gln Gly Glu Gly Leu Leu Leu Gly Phe Phe
            820                 825                 830

caa aag gct tat gcc ccc ttc ctg ctg cac tgg atc act cga ggt gtt 2544
Gln Lys Ala Tyr Ala Pro Phe Leu Leu His Trp Ile Thr Arg Gly Val
        835                 840                 845

gtg ctg ctg ctg ttt ctc gcc ctg ttc gga gtg agc ctc tac tcc atg 2592
Val Leu Leu Leu Phe Leu Ala Leu Phe Gly Val Ser Leu Tyr Ser Met
    850                 855                 860

tgc cac atc agc gtg gga ctg gac cag gag ctg gcc ctg ccc aag gac 2640
Cys His Ile Ser Val Gly Leu Asp Gln Glu Leu Ala Leu Pro Lys Asp
865                 870                 875                 880

tcg tac ctg ctt gac tat ttc ctc ttt ctg aac cgc tac ttc gag gtg 2688
Ser Tyr Leu Leu Asp Tyr Phe Leu Phe Leu Asn Arg Tyr Phe Glu Val
                885                 890                 895

ggg gcc ccg gtg tac ttt gtt acc acc ttg ggc tac aac ttc tcc agc 2736
Gly Ala Pro Val Tyr Phe Val Thr Thr Leu Gly Tyr Asn Phe Ser Ser
            900                 905                 910

gag gct ggg atg aat gcc atc tgc tcc agt gca ggc tgc aac aac ttc 2784
Glu Ala Gly Met Asn Ala Ile Cys Ser Ser Ala Gly Cys Asn Asn Phe
        915                 920                 925

tcc ttc acc cag aag atc cag tat gcc aca gag ttc cct gag cag tct 2832
Ser Phe Thr Gln Lys Ile Gln Tyr Ala Thr Glu Phe Pro Glu Gln Ser
    930                 935                 940

tac ctg gcc atc cct gcc tcc tcc tgg gtg gat gac ttc att gac tgg 2880
Tyr Leu Ala Ile Pro Ala Ser Ser Trp Val Asp Asp Phe Ile Asp Trp
945                 950                 955                 960

ctg acc ccg tcc tcc tgc tgc cgc ctt tat ata tct ggc ccc aat aag 2928
Leu Thr Pro Ser Ser Cys Cys Arg Leu Tyr Ile Ser Gly Pro Asn Lys
                965                 970                 975

gac aag ttc tgc ccc tcg acc gtc aac tct ctg aac tgc cta aag aac 2976
Asp Lys Phe Cys Pro Ser Thr Val Asn Ser Leu Asn Cys Leu Lys Asn
            980                 985                 990

tgc atg agc atc acg atg ggc tct gtg agg ccc tcg gtg gag cag ttc 3024
Cys Met Ser Ile Thr Met Gly Ser Val Arg Pro Ser Val Glu Gln Phe
        995                 1000                1005

cat aag tat ctt ccc tgg ttc ctg aac gac cgg ccc aac atc aaa 3069
His Lys Tyr Leu Pro Trp Phe Leu Asn Asp Arg Pro Asn Ile Lys
    1010                1015                1020

tgt ccc aaa ggc ggc ctg gca gca tac agc acc tct gtg aac ttg 3114

Cys Pro Lys Gly Gly Leu Ala Ala Tyr Ser Thr Ser Val Asn Leu
    1025                1030                1035

act tca gat ggc cag gtt tta gcc tcc agg ttc atg gcc tat cac 3159
Thr Ser Asp Gly Gln Val Leu Ala Ser Arg Phe Met Ala Tyr His
    1040                1045                1050

aag ccc ctg aaa aac tca cag gat tac aca gaa gct ctg cgg gca 3204
Lys Pro Leu Lys Asn Ser Gln Asp Tyr Thr Glu Ala Leu Arg Ala
    1055                1060                1065

gct cga gag ctg gca gcc aac atc act gct gac ctg cgg aaa gtg 3249
Ala Arg Glu Leu Ala Ala Asn Ile Thr Ala Asp Leu Arg Lys Val
    1070                1075                1080

cct gga aca gac ccg gct ttt gag gtc ttc ccc tac acg atc acc 3294
Pro Gly Thr Asp Pro Ala Phe Glu Val Phe Pro Tyr Thr Ile Thr
    1085                1090                1095

aat gtg ttt tat gag cag tac ctg acc atc ctc cct gag ggg ctc 3339
Asn Val Phe Tyr Glu Gln Tyr Leu Thr Ile Leu Pro Glu Gly Leu
    1100                1105                1110

ttc atg ctc agc ctc tgc ctt gtg ccc acc ttc gct gtc tcc tgc 3384
Phe Met Leu Ser Leu Cys Leu Val Pro Thr Phe Ala Val Ser Cys
    1115                1120                1125

ctc ctg ctg ggc ctg gac ctg cgc tcc ggc ctc ctc aac ctg ctc 3429
Leu Leu Leu Gly Leu Asp Leu Arg Ser Gly Leu Leu Asn Leu Leu
    1130                1135                1140

tcc att gtc atg atc ctc gtg gac act gtc ggc ttc atg gcc ctg 3474
Ser Ile Val Met Ile Leu Val Asp Thr Val Gly Phe Met Ala Leu
    1145                1150                1155

tgg gac atc agt tac aat gct gtg tcc ctc atc aac ctg gtc tcg 3519
Trp Asp Ile Ser Tyr Asn Ala Val Ser Leu Ile Asn Leu Val Ser
    1160                1165                1170

gcg gtg ggc atg tct gtg gag ttt gtg tcc cac att acc cgc tcc 3564
Ala Val Gly Met Ser Val Glu Phe Val Ser His Ile Thr Arg Ser
    1175                1180                1185

ttt gcc atc agc acc aag ccc acc tgg ctg gag agg gcc aaa gag 3609
Phe Ala Ile Ser Thr Lys Pro Thr Trp Leu Glu Arg Ala Lys Glu
    1190                1195                1200

gcc acc atc tct atg gga agt gcg gtg ttt gca ggt gtg gcc atg 3654
Ala Thr Ile Ser Met Gly Ser Ala Val Phe Ala Gly Val Ala Met
    1205                1210                1215

acc aac ctg cct ggc atc ctt gtc ctg ggc ctc gcc aag gcc cag 3699
Thr Asn Leu Pro Gly Ile Leu Val Leu Gly Leu Ala Lys Ala Gln
    1220                1225                1230

ctc att cag atc ttc ttc ttc cgc ctc aac ctc ctg atc act ctg 3744
Leu Ile Gln Ile Phe Phe Phe Arg Leu Asn Leu Leu Ile Thr Leu
    1235                1240                1245

ctg ggc ctg ctg cat ggc ttg gtc ttc ctg ccc gtc atc ctc agc 3789
Leu Gly Leu Leu His Gly Leu Val Phe Leu Pro Val Ile Leu Ser

1250                1255                1260

tac gtg ggg cct gac gtt aac ccg gct ctg gca ctg gag cag aag        3834
Tyr Val Gly Pro Asp Val Asn Pro Ala Leu Ala Leu Glu Gln Lys
    1265                1270                1275

cgg gct gag gag gcg gtg gca gca gtc atg gtg gcc tct tgc cca        3879
Arg Ala Glu Glu Ala Val Ala Ala Val Met Val Ala Ser Cys Pro
    1280                1285                1290

aat cac ccc tcc cga gtc tcc aca gct gac aac atc tat gtc aac        3924
Asn His Pro Ser Arg Val Ser Thr Ala Asp Asn Ile Tyr Val Asn
    1295                1300                1305

cac agc ttt gaa ggt tct atc aaa ggt gct ggt gcc atc agc aac        3969
His Ser Phe Glu Gly Ser Ile Lys Gly Ala Gly Ala Ile Ser Asn
    1310                1315                1320

ttc ttg ccc aac aat ggg cgg cag ttc tga                            3999
Phe Leu Pro Asn Asn Gly Arg Gln Phe
    1325                1330

<210>  4

<211>  1332

<212>  PRT

<213>  Homo sapiens

<400>  4

Met Ala Glu Ala Gly Leu Arg Gly Trp Leu Leu Trp Ala Leu Leu Leu
1               5                   10                  15

Arg Leu Ala Gln Ser Glu Pro Tyr Thr Thr Ile His Gln Pro Gly Tyr
            20                  25                  30

Cys Ala Phe Tyr Asp Glu Cys Gly Lys Asn Pro Glu Leu Ser Gly Ser
        35                  40                  45

Leu Met Thr Leu Ser Asn Val Ser Cys Leu Ser Asn Thr Pro Ala Arg
    50                  55                  60

Lys Ile Thr Gly Asp His Leu Ile Leu Leu Gln Lys Ile Cys Pro Arg
65                  70                  75                  80

Leu Tyr Thr Gly Pro Asn Thr Gln Ala Cys Cys Ser Ala Lys Gln Leu
                85                  90                  95

Val Ser Leu Glu Ala Ser Leu Ser Ile Thr Lys Ala Leu Leu Thr Arg
            100                 105                 110

Cys Pro Ala Cys Ser Asp Asn Phe Val Asn Leu His Cys His Asn Thr
        115                 120                 125

Cys Ser Pro Asn Gln Ser Leu Phe Ile Asn Val Thr Arg Val Ala Gln
    130                 135                 140

Leu Gly Ala Gly Gln Leu Pro Ala Val Val Ala Tyr Glu Ala Phe Tyr
145                 150                 155                 160

Gln His Ser Phe Ala Glu Gln Ser Tyr Asp Ser Cys Ser Arg Val Arg
                165                 170                 175

Val Pro Ala Ala Ala Thr Leu Ala Val Gly Thr Met Cys Gly Val Tyr
            180                 185                 190

Gly Ser Ala Leu Cys Asn Ala Gln Arg Trp Leu Asn Phe Gln Gly Asp
        195                 200                 205

Thr Gly Asn Gly Leu Ala Pro Leu Asp Ile Thr Phe His Leu Leu Glu
    210                 215                 220

Pro Gly Gln Ala Val Gly Ser Gly Ile Gln Pro Leu Asn Glu Gly Val
225                 230                 235                 240

Ala Arg Cys Asn Glu Ser Gln Gly Asp Asp Val Ala Thr Cys Ser Cys
                245                 250                 255

Gln Asp Cys Ala Ala Ser Cys Pro Ala Ile Ala Arg Pro Gln Ala Leu
            260                 265                 270

Asp Ser Thr Phe Tyr Leu Gly Gln Met Pro Gly Ser Leu Val Leu Ile
        275                 280                 285

Ile Ile Leu Cys Ser Val Phe Ala Val Val Thr Ile Leu Leu Val Gly
    290                 295                 300

Phe Arg Val Ala Pro Ala Arg Asp Lys Ser Lys Met Val Asp Pro Lys
305                 310                 315                 320

Lys Gly Thr Ser Leu Ser Asp Lys Leu Ser Phe Ser Thr His Thr Leu
                325                 330                 335

Leu Gly Gln Phe Phe Gln Gly Trp Gly Thr Trp Val Ala Ser Trp Pro
            340                 345                 350

Leu Thr Ile Leu Val Leu Ser Val Ile Pro Val Val Ala Leu Ala Ala
        355                 360                 365

Gly Leu Val Phe Thr Glu Leu Thr Thr Asp Pro Val Glu Leu Trp Ser
    370                 375                 380

Ala Pro Asn Ser Gln Ala Arg Ser Glu Lys Ala Phe His Asp Gln His
385                 390                 395                 400

Phe Gly Pro Phe Phe Arg Thr Asn Gln Val Ile Leu Thr Ala Pro Asn
                405                 410                 415

Arg Ser Ser Tyr Arg Tyr Asp Ser Leu Leu Leu Gly Pro Lys Asn Phe
            420                 425                 430

Ser Gly Ile Leu Asp Leu Asp Leu Leu Leu Glu Leu Leu Glu Leu Gln
        435                 440                 445

Glu Arg Leu Arg His Leu Gln Val Trp Ser Pro Glu Ala Gln Arg Asn
    450                 455                 460

Ile Ser Leu Gln Asp Ile Cys Tyr Ala Pro Leu Asn Pro Asp Asn Thr
465                 470                 475                 480

Ser Leu Tyr Asp Cys Cys Ile Asn Ser Leu Leu Gln Tyr Phe Gln Asn
                485                 490                 495

Asn Arg Thr Leu Leu Leu Leu Thr Ala Asn Gln Thr Leu Met Gly Gln
            500                 505                 510

Thr Ser Gln Val Asp Trp Lys Asp His Phe Leu Tyr Cys Ala Asn Ala
        515                 520                 525

Pro Leu Thr Phe Lys Asp Gly Thr Ala Leu Ala Leu Ser Cys Met Ala
    530                 535                 540

Asp Tyr Gly Ala Pro Val Phe Pro Phe Leu Ala Ile Gly Gly Tyr Lys
545                 550                 555                 560

Gly Lys Asp Tyr Ser Glu Ala Glu Ala Leu Ile Met Thr Phe Ser Leu
                565                 570                 575

Asn Asn Tyr Pro Ala Gly Asp Pro Arg Leu Ala Gln Ala Lys Leu Trp
            580                 585                 590

Glu Glu Ala Phe Leu Glu Glu Met Arg Ala Phe Gln Arg Arg Met Ala
        595                 600                 605

Gly Met Phe Gln Val Thr Phe Thr Ala Glu Arg Ser Leu Glu Asp Glu
    610                 615                 620

Ile Asn Arg Thr Thr Ala Glu Asp Leu Pro Ile Phe Ala Thr Ser Tyr
625                 630                 635                 640

Ile Val Ile Phe Leu Tyr Ile Ser Leu Ala Leu Gly Ser Tyr Ser Ser
                645                 650                 655

Trp Ser Arg Val Met Val Asp Ser Lys Ala Thr Leu Gly Leu Gly Gly
            660                 665                 670

Val Ala Val Val Leu Gly Ala Val Met Ala Ala Met Gly Phe Phe Ser
        675                 680                 685

Tyr Leu Gly Ile Arg Ser Ser Leu Val Ile Leu Gln Val Val Pro Phe
    690                 695                 700

Leu Val Leu Ser Val Gly Ala Asp Asn Ile Phe Ile Phe Val Leu Glu
705                 710                 715                 720

Tyr Gln Arg Leu Pro Arg Arg Pro Gly Glu Pro Arg Glu Val His Ile
                725                 730                 735

Gly Arg Ala Leu Gly Arg Val Ala Pro Ser Met Leu Leu Cys Ser Leu
            740                 745                 750

Ser Glu Ala Ile Cys Phe Phe Leu Gly Ala Leu Thr Pro Met Pro Ala
        755                 760                 765

Val Arg Thr Phe Ala Leu Thr Ser Gly Leu Ala Val Ile Leu Asp Phe
    770                 775                 780

Leu Leu Gln Met Ser Ala Phe Val Ala Leu Leu Ser Leu Asp Ser Lys
785                 790                 795                 800

Arg Gln Glu Ala Ser Arg Leu Asp Val Cys Cys Cys Val Lys Pro Gln
                805                 810                 815

Glu Leu Pro Pro Pro Gly Gln Gly Glu Gly Leu Leu Leu Gly Phe Phe
            820                 825                 830

Gln Lys Ala Tyr Ala Pro Phe Leu Leu His Trp Ile Thr Arg Gly Val

        835                 840                 845

Val Leu Leu Leu Phe Leu Ala Leu Phe Gly Val Ser Leu Tyr Ser Met
    850                 855                 860

Cys His Ile Ser Val Gly Leu Asp Gln Glu Leu Ala Leu Pro Lys Asp
865                 870                 875                 880

Ser Tyr Leu Leu Asp Tyr Phe Leu Phe Leu Asn Arg Tyr Phe Glu Val
                885                 890                 895

Gly Ala Pro Val Tyr Phe Val Thr Thr Leu Gly Tyr Asn Phe Ser Ser
            900                 905                 910

Glu Ala Gly Met Asn Ala Ile Cys Ser Ser Ala Gly Cys Asn Asn Phe
        915                 920                 925

Ser Phe Thr Gln Lys Ile Gln Tyr Ala Thr Glu Phe Pro Glu Gln Ser
    930                 935                 940

Tyr Leu Ala Ile Pro Ala Ser Ser Trp Val Asp Asp Phe Ile Asp Trp
945                 950                 955                 960

Leu Thr Pro Ser Ser Cys Cys Arg Leu Tyr Ile Ser Gly Pro Asn Lys
                965                 970                 975

Asp Lys Phe Cys Pro Ser Thr Val Asn Ser Leu Asn Cys Leu Lys Asn
            980                 985                 990

Cys Met Ser Ile Thr Met Gly Ser Val Arg Pro Ser Val Glu Gln Phe
        995                 1000                1005

His Lys Tyr Leu Pro Trp Phe Leu Asn Asp Arg Pro Asn Ile Lys
    1010                1015                1020

Cys Pro Lys Gly Gly Leu Ala Ala Tyr Ser Thr Ser Val Asn Leu
    1025                1030                1035

Thr Ser Asp Gly Gln Val Leu Ala Ser Arg Phe Met Ala Tyr His
    1040                1045                1050

Lys Pro Leu Lys Asn Ser Gln Asp Tyr Thr Glu Ala Leu Arg Ala
    1055                1060                1065

Ala Arg Glu Leu Ala Ala Asn Ile Thr Ala Asp Leu Arg Lys Val
    1070                1075                1080

Pro Gly Thr Asp Pro Ala Phe Glu Val Phe Pro Tyr Thr Ile Thr
    1085                1090                1095

Asn Val Phe Tyr Glu Gln Tyr Leu Thr Ile Leu Pro Glu Gly Leu
    1100                1105                1110

Phe Met Leu Ser Leu Cys Leu Val Pro Thr Phe Ala Val Ser Cys
    1115                1120                1125

Leu Leu Leu Gly Leu Asp Leu Arg Ser Gly Leu Leu Asn Leu Leu
    1130                1135                1140

Ser Ile Val Met Ile Leu Val Asp Thr Val Gly Phe Met Ala Leu
    1145                1150                1155

Trp Asp Ile Ser Tyr Asn Ala Val Ser Leu Ile Asn Leu Val Ser
    1160                1165                1170

Ala Val Gly Met Ser Val Glu Phe Val Ser His Ile Thr Arg Ser
    1175                1180                1185

Phe Ala Ile Ser Thr Lys Pro Thr Trp Leu Glu Arg Ala Lys Glu
    1190                1195                1200

Ala Thr Ile Ser Met Gly Ser Ala Val Phe Ala Gly Val Ala Met
    1205                1210                1215

Thr Asn Leu Pro Gly Ile Leu Val Leu Gly Leu Ala Lys Ala Gln
    1220                1225                1230

Leu Ile Gln Ile Phe Phe Phe Arg Leu Asn Leu Leu Ile Thr Leu
    1235                1240                1245

Leu Gly Leu Leu His Gly Leu Val Phe Leu Pro Val Ile Leu Ser
    1250                1255                1260

Tyr Val Gly Pro Asp Val Asn Pro Ala Leu Ala Leu Glu Gln Lys
    1265                1270                1275

Arg Ala Glu Glu Ala Val Ala Ala Val Met Val Ala Ser Cys Pro
    1280                1285                1290

Asn His Pro Ser Arg Val Ser Thr Ala Asp Asn Ile Tyr Val Asn
    1295                1300                1305

His Ser Phe Glu Gly Ser Ile Lys Gly Ala Gly Ala Ile Ser Asn
    1310                1315                1320

Phe Leu Pro Asn Asn Gly Arg Gln Phe
    1325                1330

<210> 5

<211> 885

<212> DNA

<213> Rattus sp.

<400> 5
ccacgcgtcc gcacctgcaa gtgtggtccc ctgaggcaga gcgcaacatc tccctccagg 60

acatctgcta tgcccccctc aacccatata acaccagcct ctccgactgc tgtgtcaaca 120

gcctccttca gtacttccag aacaaccgca ccctcctgat gctcacggcc aaccagactc 180

tgaatggcca gacctccctg gtggactgga aggaccattt cctctactgt gcaaatgccc 240

ctctcacgtt caaagatggc acgtctctgg ccctgagctg catggctgac tacggggctc 300

ctgtcttccc cttccttgct gttgggggat accaaggcac ggactattcc gaggcagaag 360

cgctgatcat aaccttctct ctcaataact accccgctga tgatccccgc atggcccagg 420

ccaagctctg ggaggaggct ttcttgaagg aaatggaatc cttccagagg aacacaagtg 480

acaagttcca ggttgcgttc tcagctgagc gctctctgga ggatgagatc aaccgcacca 540

ccatccagga cctgcctgtc tttgccgtca gctacattat cgtcttcctg tacatctccc 600

tggccctggg cagctactcc agatgcagcc gagtagcggt ggagtccaag gctactctgg 660

gcctaggtgg ggtgatagtg tgctgggagc agttctggct tgcatggggc ttctaactcc 720

tacctgggtg tcccctcttc tctggttatc atccaagtgg tacctttcct ggtgcttaag 780

ctgtgggagc tggacacatc tacatcctag acttgagtac cagaggtacc taggaagccg 840

cggaacagcg aaaaggacac attgggcgca ccctgggcat gtggc 885

<210> 6

<211> 458

<212> DNA

<213> Rattus sp.

<400> 6
gaccagatgt taaccaagct ctggtacagg aggagaaact agccagcgag gcagcagtgg 60

ccccagagcc ttcttgccca cagtacccct cccctgctga tgcggatgcc aatgttaact 120

acggctttgc cccagaactt gcccacggag ctaatgctgc tagaagctct ttgcccaaaa 180

gtgaccaaaa gttctaatgg agtaggagct tgtccatgct tctgctgatg agggatcatg 240

aaggtcttcc ctctggttgt cctcaaggcc tgggggaggg ttgttcagag aaaaatggct 300

ggcattcctg ccacgaggca accggcagct tggcactgac tccttggtct cataggtccc 360

taaggcttgg tcagattact cctcatggag agactatctt aagtatctaa gctatcgatt 420

gggatgcatc gctgttcatt aaaaaggcta tggctatg 458

<210> 7

<211> 896

<212> DNA

<213> Rattus sp.

<400> 7
ccacgcgtcc gcagtttcat aagtacctgc cctggttcct gaatgatccg cccaatatca 60

gatgtcccaa agggggtcta gcagcgtata gaacgtctgt gaatttgagc tcagatggcc 120

aggttatagc ctcccagttc atggcctacc acaagccctt aaggaactca caggacttca 180

cagaagctct ccgggcgtcc cggttgctag cagccaacat cacagctgac ctacggaagg 240

tgcctgggac agatccaaac tttgaggtct tcccttacac gatctccaac gtgttctacc 300

agcaatacct gacggtcctt cctgagggaa tcttcaccct tgctctttgc tttgtgccca 360

cctttgttgt ctgctacctc ctactgggcc tggacatgtg ctcagggatc ctcaacctac 420

tctccatcat tatgattctc gtggacacca ttggcctcat ggctgtgtgg ggtatcagct 480

ataatgcggt atccctcatc aaccttgtca cggcagtggg catgtctgtg gagtttgtgt 540

cccacatcac tcggtccttt gcttgtaagc accaagccta cccggctgga gagggctaaa 600

agatgctact gtcttcatgg gcagtgcggt gtttgctgga gtggccatga ccaacttccc 660

aggcatcctc atcttggggg ctttgcccca agcccaggct tattcagatc ttcttcttcc 720

gcctcaacct tctgatcacc tttgctgggg tctgctgcat ggctggtctt cctgcccggt 780

ttgtcctcag ctatctggga ccagatgtaa ccaaggctct gctacccgga ggagaaacta 840

gccagcgagg gcagcagtgg ccccagagac ttcttgccca caagtaccct tccctg 896

<210> 8

<211> 3124

<212> DNA

<213> Rattus sp.

<400> 8
tgcaagtgtg gtcccctgag gcagagcgca acatctccct ccaggacatc tgctatgccc 60

ccctcaaccc atataacacc agcctctccg actgctgtgt caacagcctc cttcagtact 120

tccagaacaa ccgcaccctc ctgatgctca cggccaacca gactctgaat ggccagacct 180

ccctggtgga ctggaaggac catttcctct actgtgcaaa tgcccctctc acgttcaaag 240

atggcacgtc tctggccctg agctgcatgg ctgactacgg ggctcctgtc ttccccttcc 300

ttgctgttgg gggataccaa ggcacggact attccgaggc agaagcgctg atcataacct 360

tctctctcaa taactacccc gctgatgatc cccgcatggc ccaggccaag ctctgggagg 420

aggctttctt gaaggaaatg gaatccttcc agaggaacac aagtgacaag ttccaggttg 480

cgttctcagc tgagcgctct ctggaggatg agatcaaccg caccaccatc caggacctgc 540

ctgtctttgc cgtcagctac attatcgtct tcctgtacat ctccctggcc ctgggcagct 600

actccagatg cagccgagta gcggtggagt ccaaggctac tctgggccta ggtggggtga 660

ttgttgtgct gggagcagtt ctggctgcca tgggcttcta ctcctacctg ggtgtcccct 720

cttctctggt tatcatccaa gtggtacctt tcctggtgct agctgtggga gctgacaaca 780

tcttcatctt tgttcttgag taccagaggc tacctaggat gcctggggaa cagcgagagg 840

ctcacattgg ccgcaccctg ggcagtgtgg cccccagcat gctgctgtgc agcctctctg 900

aggccatctg cttctttcta ggggccctga cccccatgcc agctgtgagg accttcgcct 960

tgacctctgg cttagcaatt atcctcgact tcctgctcca gatgactgcc tttgtggccc 1020

tgctctccct ggatagcaag aggcaggagg cctctcgccc ggatgtctta tgctgctttt 1080

caacccggaa gctgccccca cctaaagaaa aagaaggcct cttactccgc ttcttccgca 1140

agatatacgc tcctttcctg ctgcacagat tcatccgccc tgttgtgatg ctgctgtttc 1200

tgaccctgtt tggagcaaat ctctacttaa tgtgcaacat caacgtgggg ctagaccagg 1260

agctggctct gcccaaggac tcgtacttga tagactactt cctctttctg aaccgatacc 1320

ttgaagtggg gcctccagtg tactttgtca ccacctcggg cttcaacttc tccagcgagg 1380

caggcatgaa cgccacttgc tctagcgcag gctgtaagag cttctcccta acccagaaaa 1440

tccagtatgc cagtgaattc cctgaccagt cttacgtggc tattgctgca tcctcctggg 1500

tagatgactt catcgactgg ctgaccccgt cctcctcctg ctgtcgcctt tatatacgtg 1560

gcccccataa ggatgagttc tgtccctcaa cggatacttc cttcaactgc ttaaaaaact 1620

gcatgaaccg cactctgggt cctgtgaggc ccacagcgga acagtttcat aagtacctgc 1680

cctggttcct gaatgatccg cccaatatca gatgtcccaa agggggtcta gcagcgtata 1740

gaacgtctgt gaatttgagc tcagatggcc aggttatagc ctcccagttc atggcctacc 1800

acaagccctt aaggaactca caggacttca cagaagctct ccgggcgtcc cggttgctag 1860

cagccaacat cacagctgac ctacggaagg tgcctgggac agatccaaac tttgaggtct 1920

tcccttacac gatctccaac gtgttctacc agcaatacct gacggtcctt cctgagggaa 1980

tcttcaccct tgctctttgc tttgtgccca cctttgttgt ctgctacctc ctactgggcc 2040

tggacatgtg ctcagggatc ctcaacctac tctccatcat tatgattctc gtggacacca 2100

ttggcctcat ggctgtgtgg ggtatcagct ataatgcggt atccctcatc aaccttgtca 2160

cggcagtggg catgtctgtg gagtttgtgt cccacatcac tcggtccttt gctgtaagca 2220

ccaagcctac ccggctggag agggctaaag atgctactgt cttcatgggc agtgcggtgt 2280

ttgctggagt ggccatgacc aacttcccag gcatcctcat cttgggcttt gcccaagccc 2340

agcttattca gatcttcttc ttccgcctca accttctgat caccttgctg ggtctgctgc 2400

atggcctggt cttcctgccg gttgtcctca gctatctggg accagatgtt aaccaagctc 2460

tggtacagga ggagaaacta gccagcgagg cagcagtggc cccagagcct tcttgcccac 2520

agtacccctc ccctgctgat gcggatgcca atgttaacta cggctttgcc ccagaacttg 2580

cccacggagc taatgctgct agaagctctt tgcccaaaag tgaccaaaag ttctaatgga 2640

gtaggagctt gtccatgctt cttgctgatg agggatcatg aaggtcttcc ctctggttgt 2700

cctcaaggcc tgggggaggt tgtttcagag aaaaatggct ggcattcctg ccacgaggca 2760

aaccggcagc attggcactg acctccttgc tctcataggt ccctaaggcc ttggtcagat 2820

tacctcctcc atggagagac tatcttaagt atcttaagta tcgtatggga tgcatcgcct 2880

gtcaattaaa aaggctatgg cctatggctc aggcagggcc atccggaaga agagaggatt 2940

ctgggataaa gccaggtggg agattcgcct ggggaaaatg tgacaatggt tcctgagcat 3000

gggcaatcag ccatgtggca gaatgtaaat taatataaat gggttgtctt aagttatgat 3060

tctagctggg gaggagccta gctgtgtagc caagatattt gtaaatataa aaaaaaaaaa 3120

aaaa 3124

<210> 9

<211> 4484

<212>  DNA

<213>  Rattus sp.

<400>  9
   atggcagctg cctggctggg atggctgctc tgggccctgc tcctgagcgc ggcccagggt      60
   gagctataca cacccaaaca cgaagctggg gtctgcacct tttacgaaga gtgcgggaaa     120
   aacccagagc tctctggagg cctcacgtca ctatccaatg tatcctgcct gtctaacacc     180
   ccggcccgcc acgtcacggg tgaacacctg gctcttctcc agcgcatctg tccccgcctg     240
   tacaacggcc ccaataccac ttttgcctgt tgctctacca agcagctgct gtccttagaa     300
   agcagcatgt ccatcaccaa ggcccttctc acgcgctgcc cggcctgctc tgacaatttt     360
   gtgagcttac actgccacaa cacttgcagc cctgaccaga gcctcttcat caacgtcacc     420
   cgggtggttg agcggggcgc tggagagcct cctgccgtgg tggcctatga ggccttttat     480
   cagcgcagct ttgctgagaa ggcctatgag tcctgcagcc aggtgcgcat ccctgcggcc     540
   gcttccttgg ccgtgggcag catgtgtgga gtgtatggct ccgccctctg caatgctcag     600
   cgctggctca acttccaagg agacacaggg aatggcctgg ctccgctgga tatcaccttc     660
   cacctcttgg agcctggcca ggccctaccg gatgggatcc agccactgaa tgggaagatc     720
   gcaccctgca acgagtctca gggtgatgac tcagcagtct gctcctgcca ggactgtgcg     780
   gcgtcctgcc ctgtcatccc tccgcccgag gccttgcgcc cttccttcta catgggtcgc     840
   atgccaggct ggctggccct catcatcatc ttcactgctg tctttgtgtt gctctctgca     900
   gtccttgtgc gtctccgagt ggtttccaac aggaacaaga acaaggcaga aggcccccag     960
   gaagccccca aactccctca taagcacaaa ctctcacccc ataccatcct gggccggttc    1020
   ttccagaact ggggcacaag ggtggcctcg tggccactca ccgtcttagc actgtccttc    1080
   atcgttgtga tagccttagc agcaggcctg acctttattg aactcaccac agaccctgtg    1140
   gaactgtggt cggcccccaa gagccaggcc cggaaagaga agtctttcca tgatgagcat    1200
   ttcggcccct tctttcgaac caaccagatt ttcgtgacag ctcggaacag gtccagctac    1260
   aagtacgact ccctactgct agggtccaag aacttcagtg ggatcctgtc cctggacttc    1320
   ctgctggagc tgctggagct tcaggagagg cttcgacacc tgcaagtgtg gtcccctgag    1380
   gcagagcgca acatctccct ccaggacatc tgctatgccc ccctcaaccc atataacacc    1440
   agcctctccg actgctgtgt caacagcctc cttcagtact tccagaacaa ccgcaccctc    1500
   ctgatgctca cggccaacca gactctgaat ggccagacct ccctggtgga ctggaaggac    1560
   catttcctct actgtgcaaa tgcccctctc acgttcaaag atggcacgtc tctggccctg    1620
   agctgcatgg ctgactacgg ggctcctgtc ttccccttcc ttgctgttgg gggataccaa 1680
   ggcacggact attccgaggc agaagcgctg atcataacct tctctctcaa taactacccc 1740
   gctgatgatc cccgcatggc ccaggccaag ctctgggagg aggctttctt gaaggaaatg 1800
   gaatccttcc agaggaacac aagtgacaag ttccaggttg cgttctcagc tgagcgctct 1860
   ctggaggatg agatcaaccg caccaccatc caggacctgc ctgtctttgc cgtcagctac 1920
   attatcgtct tcctgtacat ctccctggcc ctgggcagct actccagatg cagccgagta 1980
   gcggtggagt ccaaggctac tctgggccta ggtggggtga ttgttgtgct gggagcagtt 2040
   ctggctgcca tgggcttcta ctcctacctg ggtgtcccct cttctctggt tatcatccaa 2100
   gtggtacctt tcctggtgct agctgtggga gctgacaaca tcttcatctt tgttcttgag 2160
   taccagaggc tacctaggat gcctggggaa cagcgagagg ctcacattgg ccgcaccctg 2220
   ggcagtgtgg cccccagcat gctgctgtgc agcctctctg aggccatctg cttctttcta 2280
   ggggccctga cccccatgcc agctgtgagg accttcgcct tgacctctgg cttagcaatt 2340
   atcctcgact tcctgctcca gatgactgcc tttgtggccc tgctctccct ggatagcaag 2400
   aggcaggagg cctctcgccc ggatgtctta tgctgctttt caacccggaa gctgccccca 2460
   cctaaagaaa aagaaggcct cttactccgc ttcttccgca agatatacgc tcctttcctg 2520
   ctgcacagat tcatccgccc tgttgtgatg ctgctgtttc tgaccctgtt tggagcaaat 2580
   ctctacttaa tgtgcaacat caacgtgggg ctagaccagg agctggctct gcccaaggac 2640
   tcgtacttga tagactactt cctctttctg aaccgatacc ttgaagtggg gcctccagtg 2700
   tactttgtca ccacctcggg cttcaacttc tccagcgagg caggcatgaa cgccacttgc 2760
   tctagcgcag gctgtaagag cttctcccta acccagaaaa tccagtatgc cagtgaattc 2820
   cctgaccagt cttacgtggc tattgctgca tcctcctggg tagatgactt catcgactgg 2880
   ctgaccccgt cctcctcctg ctgtcgcctt tatatacgtg gcccccataa ggatgagttc 2940
   tgtccctcaa cggatacttc cttcaactgc ttaaaaaact gcatgaaccg cactctgggt 3000
   cctgtgaggc ccacagcgga acagtttcat aagtacctgc cctggttcct gaatgatccg 3060
   cccaatatca gatgtcccaa agggggtcta gcagcgtata gaacgtctgt gaatttgagc 3120
   tcagatggcc aggttatagc ctcccagttc atggcctacc acaagccctt aaggaactca 3180
   caggacttca cagaagctct ccgggcgtcc cggttgctag cagccaacat cacagctgac 3240
   ctacggaagg tgcctgggac agatccaaac tttgaggtct tcccttacac gatctccaac 3300
   gtgttctacc agcaatacct gacggtcctt cctgagggaa tcttcaccct tgctctttgc 3360
   tttgtgccca cctttgttgt ctgctacctc ctactgggcc tggacatgtg ctcagggatc 3420
   ctcaacctac tctccatcat tatgattctc gtggacacca ttggcctcat ggctgtgtgg 3480
   ggtatcagct ataatgcggt atccctcatc aaccttgtca cggcagtggg catgtctgtg 3540
   gagtttgtgt cccacatcac tcggtccttt gctgtaagca ccaagcctac ccggctggag 3600
   agggctaaag atgctactgt cttcatgggc agtgcggtgt ttgctggagt ggccatgacc 3660
   aacttcccag gcatcctcat cttgggcttt gcccaagccc agcttattca gatcttcttc 3720
   ttccgcctca accttctgat caccttgctg ggtctgctgc atggcctggt cttcctgccg 3780
   gttgtcctca gctatctggg accagatgtt aaccaagctc tggtacagga ggagaaacta 3840
   gccagcgagg cagcagtggc cccagagcct tcttgcccac agtacccctc ccctgctgat 3900
   gcggatgcca atgttaacta cggctttgcc ccagaacttg cccacggagc taatgctgct 3960
   agaagctctt tgcccaaaag tgaccaaaag ttctaatgga gtaggagctt gtccatgctt 4020
   cttgctgatg agggatcatg aaggtcttcc ctctggttgt cctcaaggcc tggggggagg 4080
   ttgtttcaga gaaaaatggc tggcattcct gccacgaggc aaccggcagc attggcactg 4140
   acctccttgc tctcataggt ccctaaggcc ttggtcagat tacctcctcc atggagagac 4200
   tatcttaagt atcttaagta tcgtatggga tgcatcgcct gtcaattaaa aaggctatgg 4260
   cctatggctc aggcagggcc atccggaaga agagaggatt ctgggataaa gccaggtggg 4320
   agattcgcct ggggaaaatg tgacaatggt tcctgagcat gggcaatcag ccatgtggca 4380
   gaatgtaaat taatataaat gggttgtctt aagttatgat tctagctggg gaggagccta 4440
   gctgtgtagc caagatattt gtaaatataa aaaaaaaaaa aaaa 4484

<210> 10

<211> 3993
<212> DNA

<213> Rattus sp.

<220>

<221> misc_feature

<222> (1)..(3993)

<223> n is g or a or t or c

<400> 10
   atggcngcng cntggytngg ntggytnytn tgggcnytny tnytnwsngc ngcncarggn 60
   garytntaya cnccnaarca ygargcnggn gtntgyacnt tytaygarga rtgyggnaar 120
   aayccngary tnwsnggngg nytnacnwsn ytnwsnaayg tnwsntgyyt nwsnaayacn 180
   ccngcnmgnc aygtnacngg ngarcayytn gcnytnytnc armgnathtg yccnmgnytn 240
   tayaayggnc cnaayacnac nttygcntgy tgywsnacna arcarytnyt nwsnytngar 300
   wsnwsnatgw snathacnaa rgcnytnytn acnmgntgyc cngcntgyws ngayaaytty 360
   gtnwsnytnc aytgycayaa yacntgywsn ccngaycarw snytnttyat haaygtnacn 420
   mgngtngtng armgnggngc nggngarccn ccngcngtng tngcntayga rgcnttytay 480
   carmgnwsnt tygcngaraa rgcntaygar wsntgywsnc argtnmgnat hccngcngcn 540
   gcnwsnytng cngtnggnws natgtgyggn gtntayggnw sngcnytntg yaaygcncar 600
   mgntggytna ayttycargg ngayacnggn aayggnytng cnccnytnga yathacntty 660
   cayytnytng arccnggnca rgcnytnccn gayggnathc arccnytnaa yggnaarath 720
   gcnccntgya aygarwsnca rggngaygay wsngcngtnt gywsntgyca rgaytgygcn 780
   gcnwsntgyc cngtnathcc nccnccngar gcnytnmgnc cnwsnttyta yatgggnmgn 840
   atgccnggnt ggytngcnyt nathathath ttyacngcng tnttygtnyt nytnwsngcn 900
   gtnytngtnm gnytnmgngt ngtnwsnaay mgnaayaara ayaargcnga rggnccncar 960
   gargcnccna arytnccnca yaarcayaar ytnwsnccnc ayacnathyt nggnmgntty 1020
   ttycaraayt ggggnacnmg ngtngcnwsn tggccnytna cngtnytngc nytnwsntty 1080
   athgtngtna thgcnytngc ngcnggnytn acnttyathg arytnacnac ngayccngtn 1140
   garytntggw sngcnccnaa rwsncargcn mgnaargara arwsnttyca ygaygarcay 1200
   ttyggnccnt tyttymgnac naaycarath ttygtnacng cnmgnaaymg nwsnwsntay 1260
   aartaygayw snytnytnyt nggnwsnaar aayttywsng gnathytnws nytngaytty 1320
   ytnytngary tnytngaryt ncargarmgn ytnmgncayy tncargtntg gwsnccngar 1380
   gcngarmgna ayathwsnyt ncargayath tgytaygcnc cnytnaaycc ntayaayacn 1440
   wsnytnwsng aytgytgygt naaywsnytn ytncartayt tycaraayaa ymgnacnytn 1500
   ytnatgytna cngcnaayca racnytnaay ggncaracnw snytngtnga ytggaargay 1560
   cayttyytnt aytgygcnaa ygcnccnytn acnttyaarg ayggnacnws nytngcnytn 1620
   wsntgyatgg cngaytaygg ngcnccngtn ttyccnttyy tngcngtngg nggntaycar 1680
   ggnacngayt aywsngargc ngargcnytn athathacnt tywsnytnaa yaaytayccn 1740
   gcngaygayc cnmgnatggc ncargcnaar ytntgggarg argcnttyyt naargaratg 1800
   garwsnttyc armgnaayac nwsngayaar ttycargtng cnttywsngc ngarmgnwsn 1860
   ytngargayg arathaaymg nacnacnath cargayytnc cngtnttygc ngtnwsntay 1920
   athathgtnt tyytntayat hwsnytngcn ytnggnwsnt aywsnmgntg ywsnmgngtn 1980
   gcngtngarw snaargcnac nytnggnytn ggnggngtna thgtngtnyt nggngcngtn 2040
   ytngcngcna tgggnttyta ywsntayytn ggngtnccnw snwsnytngt nathathcar 2100
   gtngtnccnt tyytngtnyt ngcngtnggn gcngayaaya thttyathtt ygtnytngar 2160
   taycarmgny tnccnmgnat gccnggngar carmgngarg cncayathgg nmgnacnytn 2220
   ggnwsngtng cnccnwsnat gytnytntgy wsnytnwsng argcnathtg yttyttyytn 2280
   ggngcnytna cnccnatgcc ngcngtnmgn acnttygcny tnacnwsngg nytngcnath 2340
   athytngayt tyytnytnca ratgacngcn ttygtngcny tnytnwsnyt ngaywsnaar 2400
   mgncargarg cnwsnmgncc ngaygtnytn tgytgyttyw snacnmgnaa rytnccnccn 2460
   ccnaargara argarggnyt nytnytnmgn ttyttymgna arathtaygc nccnttyytn 2520
   ytncaymgnt tyathmgncc ngtngtnatg ytnytnttyy tnacnytntt yggngcnaay 2580
   ytntayytna tgtgyaayat haaygtnggn ytngaycarg arytngcnyt nccnaargay 2640
   wsntayytna thgaytaytt yytnttyytn aaymgntayy tngargtngg nccnccngtn 2700
   tayttygtna cnacnwsngg nttyaaytty wsnwsngarg cnggnatgaa ygcnacntgy 2760
   wsnwsngcng gntgyaarws nttywsnytn acncaraara thcartaygc nwsngartty 2820
   ccngaycarw sntaygtngc nathgcngcn wsnwsntggg tngaygaytt yathgaytgg 2880
   ytnacnccnw snwsnwsntg ytgymgnytn tayathmgng gnccncayaa rgaygartty 2940
   tgyccnwsna cngayacnws nttyaaytgy ytnaaraayt gyatgaaymg nacnytnggn 3000
   ccngtnmgnc cnacngcnga rcarttycay aartayytnc cntggttyyt naaygayccn 3060
   ccnaayathm gntgyccnaa rggnggnytn gcngcntaym gnacnwsngt naayytnwsn 3120
   wsngayggnc argtnathgc nwsncartty atggcntayc ayaarccnyt nmgnaaywsn 3180
   cargayttya cngargcnyt nmgngcnwsn mgnytnytng cngcnaayat hacngcngay 3240
   ytnmgnaarg tnccnggnac ngayccnaay ttygargtnt tyccntayac nathwsnaay 3300
   gtnttytayc arcartayyt nacngtnytn ccngarggna thttyacnyt ngcnytntgy 3360
   ttygtnccna cnttygtngt ntgytayytn ytnytnggny tngayatgtg ywsnggnath 3420
   ytnaayytny tnwsnathat hatgathytn gtngayacna thggnytnat ggcngtntgg 3480
   ggnathwsnt ayaaygcngt nwsnytnath aayytngtna cngcngtngg natgwsngtn 3540
   garttygtnw sncayathac nmgnwsntty gcngtnwsna cnaarccnac nmgnytngar 3600
   mgngcnaarg aygcnacngt nttyatgggn wsngcngtnt tygcnggngt ngcnatgacn 3660
   aayttyccng gnathytnat hytnggntty gcncargcnc arytnathca rathttytty 3720
   ttymgnytna ayytnytnat hacnytnytn ggnytnytnc ayggnytngt nttyytnccn 3780
   gtngtnytnw sntayytngg nccngaygtn aaycargcny tngtncarga rgaraarytn 3840
   gcnwsngarg cngcngtngc nccngarccn wsntgyccnc artayccnws nccngcngay 3900
   gcngaygcna aygtnaayta yggnttygcn ccngarytng cncayggngc naaygcngcn 3960
   mgnwsnwsny tnccnaarws ngaycaraar tty 3993

<210> 11

<211> 4002

<212> DNA

<213> Mus sp.

<220>

<221> CDS

<222> (1)..(4002)

<223>

<400> 11
atg gca gct gcc tgg cag gga tgg ctg ctc tgg gcc ctg ctc ctg aat 48
Met Ala Ala Ala Trp Gln Gly Trp Leu Leu Trp Ala Leu Leu Leu Asn
1               5                   10                  15

tcg gcc cag ggt gag ctc tac aca ccc act cac aaa gct ggc ttc tgc 96
Ser Ala Gln Gly Glu Leu Tyr Thr Pro Thr His Lys Ala Gly Phe Cys
            20                  25                  30

acc ttt tat gaa gag tgt ggg aag aac cca gag ctt tct gga ggc ctc 144
Thr Phe Tyr Glu Glu Cys Gly Lys Asn Pro Glu Leu Ser Gly Gly Leu
        35                  40                  45

aca tca cta tcc aat atc tcc tgc ttg tct aat acc cca gcc cgc cat 192
Thr Ser Leu Ser Asn Ile Ser Cys Leu Ser Asn Thr Pro Ala Arg His
    50                  55                  60

gtc aca ggt gac cac ctg gct ctt ctc cag cgc gtc tgt ccc cgc cta 240
Val Thr Gly Asp His Leu Ala Leu Leu Gln Arg Val Cys Pro Arg Leu
65                  70                  75                  80

tac aat ggc ccc aat gac acc tat gcc tgt tgc tct acc aag cag ctg 288
Tyr Asn Gly Pro Asn Asp Thr Tyr Ala Cys Cys Ser Thr Lys Gln Leu
                85                  90                  95

gtg tca tta gac agt agc ctg tct atc acc aag gcc ctc ctt aca cgc 336
Val Ser Leu Asp Ser Ser Leu Ser Ile Thr Lys Ala Leu Leu Thr Arg
            100                 105                 110

tgc ccg gca tgc tct gaa aat ttt gtg agc ata cac tgt cat aat acc 384
Cys Pro Ala Cys Ser Glu Asn Phe Val Ser Ile His Cys His Asn Thr
        115                 120                 125

tgc agc cct gac cag agc ctc ttc atc aat gtt act cgc gtg gtt cag 432

Cys Ser Pro Asp Gln Ser Leu Phe Ile Asn Val Thr Arg Val Val Gln
    130                 135                 140

cgg gac cct gga cag ctt cct gct gtg gtg gcc tat gag gcc ttt tat      480
Arg Asp Pro Gly Gln Leu Pro Ala Val Val Ala Tyr Glu Ala Phe Tyr
145                 150                 155                 160

caa cgc agt ttt gca gag aag gcc tat gag tcc tgt agc cgg gtg cgc      528
Gln Arg Ser Phe Ala Glu Lys Ala Tyr Glu Ser Cys Ser Arg Val Arg
                165                 170                 175

atc cct gca gct gcc tcg ctg gct gtg ggc agc atg tgt gga gtg tat      576
Ile Pro Ala Ala Ala Ser Leu Ala Val Gly Ser Met Cys Gly Val Tyr
            180                 185                 190

ggc tct gcc ctc tgc aat gct cag cgc tgg ctc aac ttc caa gga gac      624
Gly Ser Ala Leu Cys Asn Ala Gln Arg Trp Leu Asn Phe Gln Gly Asp
        195                 200                 205

aca ggg aat ggc ctg gct ccg ctg gac atc acc ttc cac ctc ttg gag      672
Thr Gly Asn Gly Leu Ala Pro Leu Asp Ile Thr Phe His Leu Leu Glu
    210                 215                 220

cct ggc cag gcc ctg gca gat ggg atg aag cca ctg gat ggg aag atc      720
Pro Gly Gln Ala Leu Ala Asp Gly Met Lys Pro Leu Asp Gly Lys Ile
225                 230                 235                 240

aca ccc tgc aat gag tcc cag ggt gaa gac tcg gca gcc tgt tcc tgc      768
Thr Pro Cys Asn Glu Ser Gln Gly Glu Asp Ser Ala Ala Cys Ser Cys
                245                 250                 255

cag gac tgt gca gca tcc tgc cct gtc atc cct ccg ccc ccg gcc ctg      816
Gln Asp Cys Ala Ala Ser Cys Pro Val Ile Pro Pro Pro Pro Ala Leu
            260                 265                 270

cgc cct tct ttc tac atg ggt cga atg cca ggc tgg ctg gct ctc atc      864
Arg Pro Ser Phe Tyr Met Gly Arg Met Pro Gly Trp Leu Ala Leu Ile
        275                 280                 285

atc atc ttc act gct gtc ttt gta ttg ctc tct gtt gtc ctt gtg tat      912
Ile Ile Phe Thr Ala Val Phe Val Leu Leu Ser Val Val Leu Val Tyr
    290                 295                 300

ctc cga gtg gct tcc aac agg aac aag aac aag aca gca ggc tcc cag      960
Leu Arg Val Ala Ser Asn Arg Asn Lys Asn Lys Thr Ala Gly Ser Gln
305                 310                 315                 320

gaa gcc ccc aac ctc cct cgt aag cgc aga ttc tca cct cac act gtc     1008
Glu Ala Pro Asn Leu Pro Arg Lys Arg Arg Phe Ser Pro His Thr Val
                325                 330                 335

ctt ggc cgg ttc ttc gag agc tgg gga aca agg gtg gcc tca tgg cca     1056
Leu Gly Arg Phe Phe Glu Ser Trp Gly Thr Arg Val Ala Ser Trp Pro
            340                 345                 350

ctc act gtc ttg gca ctg tcc ttc ata gtt gtg ata gcc ttg tca gta     1104
Leu Thr Val Leu Ala Leu Ser Phe Ile Val Val Ile Ala Leu Ser Val
        355                 360                 365

ggc ctg acc ttt ata gaa ctc acc aca gac cct gtg gaa ctg tgg tcg     1152
Gly Leu Thr Phe Ile Glu Leu Thr Thr Asp Pro Val Glu Leu Trp Ser

370                 375                 380

gcc cct aaa agc caa gcc cgg aaa gaa aag gct ttc cat gac gag cat     1200
Ala Pro Lys Ser Gln Ala Arg Lys Glu Lys Ala Phe His Asp Glu His
385                 390                 395                 400

ttt ggc ccc ttc ttc cga acc aac cag att ttt gtg aca gct aag aac     1248
Phe Gly Pro Phe Phe Arg Thr Asn Gln Ile Phe Val Thr Ala Lys Asn
                405                 410                 415

agg tcc agc tac aag tac gac tcc ctg ctg cta ggg ccc aag aac ttc     1296
Arg Ser Ser Tyr Lys Tyr Asp Ser Leu Leu Leu Gly Pro Lys Asn Phe
            420                 425                 430

agt ggg atc cta tcc ctg gac ttg ctg cag gag ctg ttg gag cta cag     1344
Ser Gly Ile Leu Ser Leu Asp Leu Leu Gln Glu Leu Leu Glu Leu Gln
        435                 440                 445

gag aga ctt cga cac ctg caa gtg tgg tcc cat gag gca cag cgc aac     1392
Glu Arg Leu Arg His Leu Gln Val Trp Ser His Glu Ala Gln Arg Asn
    450                 455                 460

atc tcc ctc cag gac atc tgc tat gct ccc ctc aac ccg cat aac acc     1440
Ile Ser Leu Gln Asp Ile Cys Tyr Ala Pro Leu Asn Pro His Asn Thr
465                 470                 475                 480

agc ctc act gac tgc tgt gtc aac agc ctc ctt caa tac ttc cag aac     1488
Ser Leu Thr Asp Cys Cys Val Asn Ser Leu Leu Gln Tyr Phe Gln Asn
                485                 490                 495

aac cac aca ctc ctg ctg ctc aca gcc aat cag act ctg aat ggc cag     1536
Asn His Thr Leu Leu Leu Leu Thr Ala Asn Gln Thr Leu Asn Gly Gln
            500                 505                 510

acc tcc ctg gtg gac tgg aag gac cat ttc ctc tac tgt gcc aat gcc     1584
Thr Ser Leu Val Asp Trp Lys Asp His Phe Leu Tyr Cys Ala Asn Ala
        515                 520                 525

cct ctc acg tac aaa gat ggc aca gcc ctg gcc ctg agc tgc ata gct     1632
Pro Leu Thr Tyr Lys Asp Gly Thr Ala Leu Ala Leu Ser Cys Ile Ala
    530                 535                 540

gac tac ggg gca cct gtc ttc ccc ttc ctt gct gtt ggg ggc tac caa     1680
Asp Tyr Gly Ala Pro Val Phe Pro Phe Leu Ala Val Gly Gly Tyr Gln
545                 550                 555                 560

ggg acg gac tac tcg gag gca gaa gcc ctg atc ata acc ttc tct atc     1728
Gly Thr Asp Tyr Ser Glu Ala Glu Ala Leu Ile Ile Thr Phe Ser Ile
                565                 570                 575

aat aac tac ccc gct gat gat ccc cgc atg gcc cac gcc aag ctc tgg     1776
Asn Asn Tyr Pro Ala Asp Asp Pro Arg Met Ala His Ala Lys Leu Trp
            580                 585                 590

gag gag gct ttc ttg aag gaa atg caa tcc ttc cag aga agc aca gct     1824
Glu Glu Ala Phe Leu Lys Glu Met Gln Ser Phe Gln Arg Ser Thr Ala
        595                 600                 605

gac aag ttc cag att gcg ttc tca gct gag cgt tct ctg gag gac gag     1872
Asp Lys Phe Gln Ile Ala Phe Ser Ala Glu Arg Ser Leu Glu Asp Glu
    610                 615                 620

atc aat cgc act acc atc cag gac ctg cct gtc ttt gcc atc agc tac 1920
Ile Asn Arg Thr Thr Ile Gln Asp Leu Pro Val Phe Ala Ile Ser Tyr
625                 630                 635                 640

ctt atc gtc ttc ctg tac atc tcc ctg gcc ctg ggc agc tac tcc aga 1968
Leu Ile Val Phe Leu Tyr Ile Ser Leu Ala Leu Gly Ser Tyr Ser Arg
                645                 650                 655

tgg agc cga gtt gcg gtg gat tcc aag gct act ctg ggc cta ggt ggg 2016
Trp Ser Arg Val Ala Val Asp Ser Lys Ala Thr Leu Gly Leu Gly Gly
            660                 665                 670

gtg gct gtt gtg ctg gga gca gtc gtc gct gcc atg ggc ttc tac tcc 2064
Val Ala Val Val Leu Gly Ala Val Val Ala Ala Met Gly Phe Tyr Ser
        675                 680                 685

tac ctg ggt gtc ccc tcc tct ctg gtc atc att caa gtg gta cct ttc 2112
Tyr Leu Gly Val Pro Ser Ser Leu Val Ile Ile Gln Val Val Pro Phe
    690                 695                 700

ctg gtg ctg gct gtg gga gct gac aac atc ttc atc ttt gtt ctt gag 2160
Leu Val Leu Ala Val Gly Ala Asp Asn Ile Phe Ile Phe Val Leu Glu
705                 710                 715                 720

tac cag agg ctg cct agg atg ccc ggg gag cag cga gag gct cac att 2208
Tyr Gln Arg Leu Pro Arg Met Pro Gly Glu Gln Arg Glu Ala His Ile
                725                 730                 735

ggc cgc acc ctg ggt agt gtg gcc ccc agc atg ctg ctg tgc agc ctc 2256
Gly Arg Thr Leu Gly Ser Val Ala Pro Ser Met Leu Leu Cys Ser Leu
            740                 745                 750

tct gag gcc atc tgc ttc ttt cta ggg gcc ctg acc tcc atg cca gct 2304
Ser Glu Ala Ile Cys Phe Phe Leu Gly Ala Leu Thr Ser Met Pro Ala
        755                 760                 765

gtg agg acc ttt gcc ttg acc tct ggc tta gca atc atc ttt gac ttc 2352
Val Arg Thr Phe Ala Leu Thr Ser Gly Leu Ala Ile Ile Phe Asp Phe
    770                 775                 780

ctg ctc cag atg aca gcc ttt gtg gcc ctg ctc tcc ctg gat agc aag 2400
Leu Leu Gln Met Thr Ala Phe Val Ala Leu Leu Ser Leu Asp Ser Lys
785                 790                 795                 800

agg cag gag gcc tct cgc ccc gac gtc gtg tgc tgc ttt tca agc cga 2448
Arg Gln Glu Ala Ser Arg Pro Asp Val Val Cys Cys Phe Ser Ser Arg
                805                 810                 815

aat ctg ccc cca ccg aaa caa aaa gaa ggc ctc tta ctt tgc ttc ttc 2496
Asn Leu Pro Pro Pro Lys Gln Lys Glu Gly Leu Leu Leu Cys Phe Phe
            820                 825                 830

cgc aag ata tac act ccc ttc ctg ctg cac aga ttc atc cgc cct gtt 2544
Arg Lys Ile Tyr Thr Pro Phe Leu Leu His Arg Phe Ile Arg Pro Val
        835                 840                 845

gtg ctg ctg ctc ttt ctg gtc ctg ttt gga gca aac ctc tac tta atg 2592
Val Leu Leu Leu Phe Leu Val Leu Phe Gly Ala Asn Leu Tyr Leu Met
    850                 855                 860

tgc aac atc agc gtg ggg ctg gac cag gat ctg gct ctg ccc aag gat 2640
Cys Asn Ile Ser Val Gly Leu Asp Gln Asp Leu Ala Leu Pro Lys Asp
865                 870                 875                 880

tcc tac ctg ata gac tac ttc ctc ttt ctg aac cgg tac ttg gaa gtg 2688
Ser Tyr Leu Ile Asp Tyr Phe Leu Phe Leu Asn Arg Tyr Leu Glu Val
                885                 890                 895

ggg cct cca gtg tac ttt gac acc acc tca ggc tac aac ttt tcc acc 2736
Gly Pro Pro Val Tyr Phe Asp Thr Thr Ser Gly Tyr Asn Phe Ser Thr
            900                 905                 910

gag gca ggc atg aac gcc att tgc tct agt gca ggc tgt gag agc ttc 2784
Glu Ala Gly Met Asn Ala Ile Cys Ser Ser Ala Gly Cys Glu Ser Phe
        915                 920                 925

tcc cta acc cag aaa atc cag tat gcc agt gaa ttc cct aat cag tct 2832
Ser Leu Thr Gln Lys Ile Gln Tyr Ala Ser Glu Phe Pro Asn Gln Ser
    930                 935                 940

tat gtg gct att gct gca tcc tcc tgg gta gat gac ttc atc gac tgg 2880
Tyr Val Ala Ile Ala Ala Ser Ser Trp Val Asp Asp Phe Ile Asp Trp
945                 950                 955                 960

ctg acc cca tcc tcc tcc tgc tgc cgc att tat acc cgt ggc ccc cat 2928
Leu Thr Pro Ser Ser Ser Cys Cys Arg Ile Tyr Thr Arg Gly Pro His
                965                 970                 975

aaa gat gag ttc tgt ccc tca acg gat act tcc ttc aac tgt ctc aaa 2976
Lys Asp Glu Phe Cys Pro Ser Thr Asp Thr Ser Phe Asn Cys Leu Lys
            980                 985                 990

aac tgc atg aac cgc act ctg ggt ccc gtg aga ccc aca aca gaa cag 3024
Asn Cys Met Asn Arg Thr Leu Gly Pro Val Arg Pro Thr Thr Glu Gln
        995                 1000                1005

ttt cat aag tac ctg ccc tgg ttc ctg aat gat acg ccc aac atc 3069
Phe His Lys Tyr Leu Pro Trp Phe Leu Asn Asp Thr Pro Asn Ile
    1010                1015                1020

aga tgt cct aaa ggg ggc cta gca gcg tat aga acc tct gtg aat 3114
Arg Cys Pro Lys Gly Gly Leu Ala Ala Tyr Arg Thr Ser Val Asn
    1025                1030                1035

ttg agc tca gat ggc cag att ata gcc tcc cag ttc atg gcc tac 3159
Leu Ser Ser Asp Gly Gln Ile Ile Ala Ser Gln Phe Met Ala Tyr
    1040                1045                1050

cac aag ccc tta cgg aac tca cag gac ttt aca gaa gct ctc cgg 3204
His Lys Pro Leu Arg Asn Ser Gln Asp Phe Thr Glu Ala Leu Arg
    1055                1060                1065

gca tcc cgg ttg cta gca gcc aac atc aca gct gaa cta cgg aag 3249
Ala Ser Arg Leu Leu Ala Ala Asn Ile Thr Ala Glu Leu Arg Lys
    1070                1075                1080

gtg cct ggg aca gat ccc aac ttt gag gtc ttc cct tac acg atc 3294
Val Pro Gly Thr Asp Pro Asn Phe Glu Val Phe Pro Tyr Thr Ile
    1085                1090                1095

tcc aat gtg ttc tac cag caa tac ctg acg gtt ctc cct gag gga 3339

Ser Asn Val Phe Tyr Gln Gln Tyr Leu Thr Val Leu Pro Glu Gly
    1100                1105                1110

atc ttc act ctt gct ctc tgc ttc gtg ccc acc ttt gtg gtc tgc 3384
Ile Phe Thr Leu Ala Leu Cys Phe Val Pro Thr Phe Val Val Cys
    1115                1120                1125

tac ctc cta ctg ggc ctg gac ata cgc tca ggc atc ctc aac ctg 3429
Tyr Leu Leu Leu Gly Leu Asp Ile Arg Ser Gly Ile Leu Asn Leu
    1130                1135                1140

ctc tcc atc att atg atc ctc gtg gac acc atc ggc ctc atg gct 3474
Leu Ser Ile Ile Met Ile Leu Val Asp Thr Ile Gly Leu Met Ala
    1145                1150                1155

gtg tgg ggt atc agc tac aat gct gtg tcc ctc atc aac ctt gtc 3519
Val Trp Gly Ile Ser Tyr Asn Ala Val Ser Leu Ile Asn Leu Val
    1160                1165                1170

acg gca gtg ggc atg tct gtg gag ttc gtg tcc cac att acc cgg 3564
Thr Ala Val Gly Met Ser Val Glu Phe Val Ser His Ile Thr Arg
    1175                1180                1185

tcc ttt gct gta agc acc aag cct acc cgg ctg gag aga gcc aaa 3609
Ser Phe Ala Val Ser Thr Lys Pro Thr Arg Leu Glu Arg Ala Lys
    1190                1195                1200

gat gct act atc ttc atg ggc agt gcg gtg ttt gct gga gtg gcc 3654
Asp Ala Thr Ile Phe Met Gly Ser Ala Val Phe Ala Gly Val Ala
    1205                1210                1215

atg acc aac ttc ccg ggc atc ctc atc ctg ggc ttt gct cag gcc 3699
Met Thr Asn Phe Pro Gly Ile Leu Ile Leu Gly Phe Ala Gln Ala
    1220                1225                1230

cag ctt atc cag att ttc ttc ttc cgc ctc aac ctc ctg atc acc 3744
Gln Leu Ile Gln Ile Phe Phe Phe Arg Leu Asn Leu Leu Ile Thr
    1235                1240                1245

ttg ctg ggt ctg cta cac ggc ctg gtc ttc ctg ccc gtt gtc ctc 3789
Leu Leu Gly Leu Leu His Gly Leu Val Phe Leu Pro Val Val Leu
    1250                1255                1260

agc tat ctg ggg cca gat gtt aac caa gct ctg gta ctg gag gag 3834
Ser Tyr Leu Gly Pro Asp Val Asn Gln Ala Leu Val Leu Glu Glu
    1265                1270                1275

aaa cta gcc act gag gca gcc atg gtc tca gag cct tct tgc cca 3879
Lys Leu Ala Thr Glu Ala Ala Met Val Ser Glu Pro Ser Cys Pro
    1280                1285                1290

cag tac ccc ttc ccg gct gat gca aac acc agt gac tat gtt aac 3924
Gln Tyr Pro Phe Pro Ala Asp Ala Asn Thr Ser Asp Tyr Val Asn
    1295                1300                1305

tac ggc ttt aat cca gaa ttt atc cct gaa att aat gct gct agc 3969
Tyr Gly Phe Asn Pro Glu Phe Ile Pro Glu Ile Asn Ala Ala Ser
    1310                1315                1320

agc tct ctg ccc aaa agt gac caa aag ttc taa 4002
Ser Ser Leu Pro Lys Ser Asp Gln Lys Phe

1325                    1330

<210>  12

<211>  1333

<212>  PRT

<213>  Mus sp.

<400>  12

Met Ala Ala Ala Trp Gln Gly Trp Leu Leu Trp Ala Leu Leu Leu Asn
1               5                   10                  15

Ser Ala Gln Gly Glu Leu Tyr Thr Pro Thr His Lys Ala Gly Phe Cys
            20                  25                  30

Thr Phe Tyr Glu Glu Cys Gly Lys Asn Pro Glu Leu Ser Gly Gly Leu
        35                  40                  45

Thr Ser Leu Ser Asn Ile Ser Cys Leu Ser Asn Thr Pro Ala Arg His
    50                  55                  60

Val Thr Gly Asp His Leu Ala Leu Leu Gln Arg Val Cys Pro Arg Leu
65                  70                  75                  80

Tyr Asn Gly Pro Asn Asp Thr Tyr Ala Cys Cys Ser Thr Lys Gln Leu
                85                  90                  95

Val Ser Leu Asp Ser Ser Leu Ser Ile Thr Lys Ala Leu Leu Thr Arg
            100                 105                 110

Cys Pro Ala Cys Ser Glu Asn Phe Val Ser Ile His Cys His Asn Thr
        115                 120                 125

Cys Ser Pro Asp Gln Ser Leu Phe Ile Asn Val Thr Arg Val Val Gln
    130                 135                 140

Arg Asp Pro Gly Gln Leu Pro Ala Val Val Ala Tyr Glu Ala Phe Tyr
145                 150                 155                 160

Gln Arg Ser Phe Ala Glu Lys Ala Tyr Glu Ser Cys Ser Arg Val Arg
                165                 170                 175

Ile Pro Ala Ala Ala Ser Leu Ala Val Gly Ser Met Cys Gly Val Tyr
            180                 185                 190

Gly Ser Ala Leu Cys Asn Ala Gln Arg Trp Leu Asn Phe Gln Gly Asp
        195                 200                 205

Thr Gly Asn Gly Leu Ala Pro Leu Asp Ile Thr Phe His Leu Leu Glu
    210                 215                 220

Pro Gly Gln Ala Leu Ala Asp Gly Met Lys Pro Leu Asp Gly Lys Ile
225                 230                 235                 240

Thr Pro Cys Asn Glu Ser Gln Gly Glu Asp Ser Ala Ala Cys Ser Cys
                245                 250                 255

Gln Asp Cys Ala Ala Ser Cys Pro Val Ile Pro Pro Pro Pro Ala Leu
            260                 265                 270

Arg Pro Ser Phe Tyr Met Gly Arg Met Pro Gly Trp Leu Ala Leu Ile
        275                 280                 285

Ile Ile Phe Thr Ala Val Phe Val Leu Leu Ser Val Val Leu Val Tyr
    290                 295                 300

Leu Arg Val Ala Ser Asn Arg Asn Lys Asn Lys Thr Ala Gly Ser Gln
305                 310                 315                 320

Glu Ala Pro Asn Leu Pro Arg Lys Arg Arg Phe Ser Pro His Thr Val
                325                 330                 335

Leu Gly Arg Phe Phe Glu Ser Trp Gly Thr Arg Val Ala Ser Trp Pro
            340                 345                 350

Leu Thr Val Leu Ala Leu Ser Phe Ile Val Val Ile Ala Leu Ser Val
        355                 360                 365

Gly Leu Thr Phe Ile Glu Leu Thr Thr Asp Pro Val Glu Leu Trp Ser
    370                 375                 380

Ala Pro Lys Ser Gln Ala Arg Lys Glu Lys Ala Phe His Asp Glu His
385                 390                 395                 400

Phe Gly Pro Phe Phe Arg Thr Asn Gln Ile Phe Val Thr Ala Lys Asn
                405                 410                 415

Arg Ser Ser Tyr Lys Tyr Asp Ser Leu Leu Leu Gly Pro Lys Asn Phe
            420                 425                 430

Ser Gly Ile Leu Ser Leu Asp Leu Leu Gln Glu Leu Leu Glu Leu Gln
        435                 440                 445

Glu Arg Leu Arg His Leu Gln Val Trp Ser His Glu Ala Gln Arg Asn
    450                 455                 460

Ile Ser Leu Gln Asp Ile Cys Tyr Ala Pro Leu Asn Pro His Asn Thr
465                 470                 475                 480

Ser Leu Thr Asp Cys Cys Val Asn Ser Leu Leu Gln Tyr Phe Gln Asn
                485                 490                 495

Asn His Thr Leu Leu Leu Leu Thr Ala Asn Gln Thr Leu Asn Gly Gln
            500                 505                 510

Thr Ser Leu Val Asp Trp Lys Asp His Phe Leu Tyr Cys Ala Asn Ala
        515                 520                 525

Pro Leu Thr Tyr Lys Asp Gly Thr Ala Leu Ala Leu Ser Cys Ile Ala
    530                 535                 540

Asp Tyr Gly Ala Pro Val Phe Pro Phe Leu Ala Val Gly Gly Tyr Gln
545                 550                 555                 560

Gly Thr Asp Tyr Ser Glu Ala Glu Ala Leu Ile Ile Thr Phe Ser Ile
                565                 570                 575

Asn Asn Tyr Pro Ala Asp Asp Pro Arg Met Ala His Ala Lys Leu Trp
            580                 585                 590

Glu Glu Ala Phe Leu Lys Glu Met Gln Ser Phe Gln Arg Ser Thr Ala
        595                 600                 605

Asp Lys Phe Gln Ile Ala Phe Ser Ala Glu Arg Ser Leu Glu Asp Glu
    610                 615                 620

Ile Asn Arg Thr Thr Ile Gln Asp Leu Pro Val Phe Ala Ile Ser Tyr
625                 630                 635                 640

Leu Ile Val Phe Leu Tyr Ile Ser Leu Ala Leu Gly Ser Tyr Ser Arg
                645                 650                 655

Trp Ser Arg Val Ala Val Asp Ser Lys Ala Thr Leu Gly Leu Gly Gly
            660                 665                 670

Val Ala Val Val Leu Gly Ala Val Val Ala Ala Met Gly Phe Tyr Ser
        675                 680                 685

Tyr Leu Gly Val Pro Ser Ser Leu Val Ile Ile Gln Val Val Pro Phe
    690                 695                 700

Leu Val Leu Ala Val Gly Ala Asp Asn Ile Phe Ile Phe Val Leu Glu
705                 710                 715                 720

Tyr Gln Arg Leu Pro Arg Met Pro Gly Glu Gln Arg Glu Ala His Ile
                725                 730                 735

Gly Arg Thr Leu Gly Ser Val Ala Pro Ser Met Leu Leu Cys Ser Leu
            740                 745                 750

Ser Glu Ala Ile Cys Phe Phe Leu Gly Ala Leu Thr Ser Met Pro Ala
        755                 760                 765

Val Arg Thr Phe Ala Leu Thr Ser Gly Leu Ala Ile Ile Phe Asp Phe
    770                 775                 780

Leu Leu Gln Met Thr Ala Phe Val Ala Leu Leu Ser Leu Asp Ser Lys
785                 790                 795                 800

Arg Gln Glu Ala Ser Arg Pro Asp Val Val Cys Cys Phe Ser Ser Arg
                805                 810                 815

Asn Leu Pro Pro Pro Lys Gln Lys Glu Gly Leu Leu Leu Cys Phe Phe
            820                 825                 830

Arg Lys Ile Tyr Thr Pro Phe Leu Leu His Arg Phe Ile Arg Pro Val
        835                 840                 845

Val Leu Leu Leu Phe Leu Val Leu Phe Gly Ala Asn Leu Tyr Leu Met
    850                 855                 860

Cys Asn Ile Ser Val Gly Leu Asp Gln Asp Leu Ala Leu Pro Lys Asp
865                 870                 875                 880

Ser Tyr Leu Ile Asp Tyr Phe Leu Phe Leu Asn Arg Tyr Leu Glu Val
                885                 890                 895

Gly Pro Pro Val Tyr Phe Asp Thr Thr Ser Gly Tyr Asn Phe Ser Thr
            900                 905                 910

Glu Ala Gly Met Asn Ala Ile Cys Ser Ser Ala Gly Cys Glu Ser Phe

        915                 920                 925

Ser Leu Thr Gln Lys Ile Gln Tyr Ala Ser Glu Phe Pro Asn Gln Ser
    930                 935                 940

Tyr Val Ala Ile Ala Ala Ser Ser Trp Val Asp Asp Phe Ile Asp Trp
945                 950                 955                 960

Leu Thr Pro Ser Ser Ser Cys Cys Arg Ile Tyr Thr Arg Gly Pro His
                965                 970                 975

Lys Asp Glu Phe Cys Pro Ser Thr Asp Thr Ser Phe Asn Cys Leu Lys
            980                 985                 990

Asn Cys Met Asn Arg Thr Leu Gly Pro Val Arg Pro Thr Thr Glu Gln
        995                 1000                1005

Phe His Lys Tyr Leu Pro Trp Phe Leu Asn Asp Thr Pro Asn Ile
    1010                1015                1020

Arg Cys Pro Lys Gly Gly Leu Ala Ala Tyr Arg Thr Ser Val Asn
    1025                1030                1035

Leu Ser Ser Asp Gly Gln Ile Ile Ala Ser Gln Phe Met Ala Tyr
    1040                1045                1050

His Lys Pro Leu Arg Asn Ser Gln Asp Phe Thr Glu Ala Leu Arg
    1055                1060                1065

Ala Ser Arg Leu Leu Ala Ala Asn Ile Thr Ala Glu Leu Arg Lys
    1070                1075                1080

Val Pro Gly Thr Asp Pro Asn Phe Glu Val Phe Pro Tyr Thr Ile
    1085                1090                1095

Ser Asn Val Phe Tyr Gln Gln Tyr Leu Thr Val Leu Pro Glu Gly
    1100                1105                1110

Ile Phe Thr Leu Ala Leu Cys Phe Val Pro Thr Phe Val Val Cys
    1115                1120                1125

Tyr Leu Leu Leu Gly Leu Asp Ile Arg Ser Gly Ile Leu Asn Leu
    1130                1135                1140

Leu Ser Ile Ile Met Ile Leu Val Asp Thr Ile Gly Leu Met Ala
    1145                1150                1155

Val Trp Gly Ile Ser Tyr Asn Ala Val Ser Leu Ile Asn Leu Val
    1160                    1165                    1170

Thr Ala Val Gly Met Ser Val Glu Phe Val Ser His Ile Thr Arg
    1175                    1180                    1185

Ser Phe Ala Val Ser Thr Lys Pro Thr Arg Leu Glu Arg Ala Lys
    1190                    1195                    1200

Asp Ala Thr Ile Phe Met Gly Ser Ala Val Phe Ala Gly Val Ala
    1205                    1210                    1215

Met Thr Asn Phe Pro Gly Ile Leu Ile Leu Gly Phe Ala Gln Ala
    1220                    1225                    1230

Gln Leu Ile Gln Ile Phe Phe Phe Arg Leu Asn Leu Leu Ile Thr
    1235                    1240                    1245

Leu Leu Gly Leu Leu His Gly Leu Val Phe Leu Pro Val Val Leu
    1250                    1255                    1260

Ser Tyr Leu Gly Pro Asp Val Asn Gln Ala Leu Val Leu Glu Glu
    1265                    1270                    1275

Lys Leu Ala Thr Glu Ala Ala Met Val Ser Glu Pro Ser Cys Pro
    1280                    1285                    1290

Gln Tyr Pro Phe Pro Ala Asp Ala Asn Thr Ser Asp Tyr Val Asn
    1295                    1300                    1305

Tyr Gly Phe Asn Pro Glu Phe Ile Pro Glu Ile Asn Ala Ala Ser
    1310                    1315                    1320

Ser Ser Leu Pro Lys Ser Asp Gln Lys Phe
    1325                    1330

<210>  13

<211>  3999

<212>  DNA

<213>  Mus sp.

<220>

<221> misc_feature

<222> (1)..(3999)

<223> n is g or a or t or c

<400> 13
atggcngcng cntggcargg ntggytnytn tgggcnytny tnytnaayws ngcncarggn 60

garytntaya cnccnacnca yaargcnggn ttytgyacnt tytaygarga rtgyggnaar 120

aayccngary tnwsnggngg nytnacnwsn ytnwsnaaya thwsntgyyt nwsnaayacn 180

ccngcnmgnc aygtnacngg ngaycayytn gcnytnytnc armgngtntg yccnmgnytn 240

tayaayggnc cnaaygayac ntaygcntgy tgywsnacna arcarytngt nwsnytngay 300

wsnwsnytnw snathacnaa rgcnytnytn acnmgntgyc cngcntgyws ngaraaytty 360

gtnwsnathc aytgycayaa yacntgywsn ccngaycarw snytnttyat haaygtnacn 420

mgngtngtnc armgngaycc nggncarytn ccngcngtng tngcntayga rgcnttytay 480

carmgnwsnt tygcngaraa rgcntaygar wsntgywsnm gngtnmgnat hccngcngcn 540

gcnwsnytng cngtnggnws natgtgyggn gtntayggnw sngcnytntg yaaygcncar 600

mgntggytna ayttycargg ngayacnggn aayggnytng cnccnytnga yathacntty 660

cayytnytng arccnggnca rgcnytngcn gayggnatga arccnytnga yggnaarath 720

acnccntgya aygarwsnca rggngargay wsngcngcnt gywsntgyca rgaytgygcn 780

gcnwsntgyc cngtnathcc nccnccnccn gcnytnmgnc cnwsnttyta yatgggnmgn 840

atgccnggnt ggytngcnyt nathathath ttyacngcng tnttygtnyt nytnwsngtn 900

gtnytngtnt ayytnmgngt ngcnwsnaay mgnaayaara ayaaracngc nggnwsncar 960

gargcnccna ayytnccnmg naarmgnmgn ttywsnccnc ayacngtnyt nggnmgntty 1020

ttygarwsnt ggggnacnmg ngtngcnwsn tggccnytna cngtnytngc nytnwsntty 1080

athgtngtna thgcnytnws ngtnggnytn acnttyathg arytnacnac ngayccngtn 1140

garytntggw sngcnccnaa rwsncargcn mgnaargara argcnttyca ygaygarcay 1200

ttyggnccnt tyttymgnac naaycarath ttygtnacng cnaaraaymg nwsnwsntay 1260

aartaygayw snytnytnyt nggnccnaar aayttywsng gnathytnws nytngayytn 1320

ytncargary tnytngaryt ncargarmgn ytnmgncayy tncargtntg gwsncaygar 1380

gcncarmgna ayathwsnyt ncargayath tgytaygcnc cnytnaaycc ncayaayacn 1440

wsnytnacng aytgytgygt naaywsnytn ytncartayt tycaraayaa ycayacnytn 1500

ytnytnytna cngcnaayca racnytnaay ggncaracnw snytngtnga ytggaargay 1560

cayttyytnt aytgygcnaa ygcnccnytn acntayaarg ayggnacngc nytngcnytn 1620

wsntgyathg cngaytaygg ngcnccngtn ttyccnttyy tngcngtngg nggntaycar 1680

ggnacngayt aywsngargc ngargcnytn athathacnt tywsnathaa yaaytayccn 1740

gcngaygayc cnmgnatggc ncaygcnaar ytntgggarg argcnttyyt naargaratg 1800

carwsnttyc armgnwsnac ngcngayaar ttycarathg cnttywsngc ngarmgnwsn 1860

ytngargayg arathaaymg nacnacnath cargayytnc cngtnttygc nathwsntay 1920

ytnathgtnt tyytntayat hwsnytngcn ytnggnwsnt aywsnmgntg gwsnmgngtn 1980

gcngtngayw snaargcnac nytnggnytn ggnggngtng cngtngtnyt nggngcngtn 2040

gtngcngcna tgggnttyta ywsntayytn ggngtnccnw snwsnytngt nathathcar 2100

gtngtnccnt tyytngtnyt ngcngtnggn gcngayaaya thttyathtt ygtnytngar 2160

taycarmgny tnccnmgnat gccnggngar carmgngarg cncayathgg nmgnacnytn 2220

ggnwsngtng cnccnwsnat gytnytntgy wsnytnwsng argcnathtg yttyttyytn 2280

ggngcnytna cnwsnatgcc ngcngtnmgn acnttygcny tnacnwsngg nytngcnath 2340

athttygayt tyytnytnca ratgacngcn ttygtngcny tnytnwsnyt ngaywsnaar 2400

mgncargarg cnwsnmgncc ngaygtngtn tgytgyttyw snwsnmgnaa yytnccnccn 2460

ccnaarcara argarggnyt nytnytntgy ttyttymgna arathtayac nccnttyytn 2520

ytncaymgnt tyathmgncc ngtngtnytn ytnytnttyy tngtnytntt yggngcnaay 2580

ytntayytna tgtgyaayat hwsngtnggn ytngaycarg ayytngcnyt nccnaargay 2640

wsntayytna thgaytaytt yytnttyytn aaymgntayy tngargtngg nccnccngtn 2700

tayttygaya cnacnwsngg ntayaaytty wsnacngarg cnggnatgaa ygcnathtgy 2760

wsnwsngcng gntgygarws nttywsnytn acncaraara thcartaygc nwsngartty 2820

ccnaaycarw sntaygtngc nathgcngcn wsnwsntggg tngaygaytt yathgaytgg 2880

ytnacnccnw snwsnwsntg ytgymgnath tayacnmgng gnccncayaa rgaygartty 2940

tgyccnwsna cngayacnws nttyaaytgy ytnaaraayt gyatgaaymg nacnytnggn 3000

ccngtnmgnc cnacnacnga rcarttycay aartayytnc cntggttyyt naaygayacn 3060

ccnaayathm gntgyccnaa rggnggnytn gcngcntaym gnacnwsngt naayytnwsn 3120

wsngayggnc arathathgc nwsncartty atggcntayc ayaarccnyt nmgnaaywsn 3180

cargayttya cngargcnyt nmgngcnwsn mgnytnytng cngcnaayat hacngcngar 3240

ytnmgnaarg tnccnggnac ngayccnaay ttygargtnt tyccntayac nathwsnaay 3300

gtnttytayc arcartayyt nacngtnytn ccngarggna thttyacnyt ngcnytntgy 3360

ttygtnccna cnttygtngt ntgytayytn ytnytnggny tngayathmg nwsnggnath 3420

ytnaayytny tnwsnathat hatgathytn gtngayacna thggnytnat ggcngtntgg 3480

ggnathwsnt ayaaygcngt nwsnytnath aayytngtna cngcngtngg natgwsngtn 3540

garttygtnw sncayathac nmgnwsntty gcngtnwsna cnaarccnac nmgnytngar 3600

mgngcnaarg aygcnacnat httyatgggn wsngcngtnt tygcnggngt ngcnatgacn 3660

aayttyccng gnathytnat hytnggntty gcncargcnc arytnathca rathttytty 3720

ttymgnytna ayytnytnat hacnytnytn ggnytnytnc ayggnytngt nttyytnccn 3780

gtngtnytnw sntayytngg nccngaygtn aaycargcny tngtnytnga rgaraarytn 3840

gcnacngarg cngcnatggt nwsngarccn wsntgyccnc artayccntt yccngcngay 3900

gcnaayacnw sngaytaygt naaytayggn ttyaayccng arttyathcc ngarathaay 3960

gcngcnwsnw snwsnytncc naarwsngay caraartty 3999

<210> 14

<211> 20

<212> DNA

<213> Artificial sequence

<220>

<223> primer

<400> 14
tcttcaccct tgctctttgc 20

<210> 15

<211> 24

<212> DNA

<213> Artificial Sequence

<220>

<223> primer

<400> 15
aatgatggag agtaggttga ggat 24

<210> 16

<211>  26

<212>  DNA

<213>  Artificial Sequence

<220>

<223>  primer

<400>  16
tgcccacctt tgttgtctgc taccta                                          26

<210>  17

<211>  21

<212>  DNA

<213>  Artificial Sequence

<220>

<223>  primer

<400>  17
atcgctgaca ggatgcagaa g                                               21

<210>  18

<211>  22

<212>  DNA

<213>  Artificial Sequence

<220>

<223>  primer

<400>  18
tcaggaggag caatgatctt ga                                              22

<210>  19

<211>  30

<212>  DNA

<213>  Artificial Sequence

<220>

<223>  primer

<400>  19
agattactgc cctggctcct agcaccatta                                        30

<210>  20

<211>  20

<212>  DNA

<213>  Artificial Sequence

<220>

<223>  primer

<400>  20
atcctcatcc tgggctttgc                                                   20

<210>  21

<211>  21

<212>  DNA

<213>  Artificial Sequence

<220>

<223>  primer

<400>  21
gcaaggtgat caggaggttg a                                                 21

<210>  22

<211>  29

<212>  DNA

<213>  Artificial Sequence

<220>

<223>  primer

<400>  22

cccagcttat ccagattttc ttcttccgc 29

<210> 23

<211> 20

<212> DNA

<213> Artificial Sequence

<220>

<223> primer

<400> 23
tcttcaccct tgctctttgc 20

<210> 24

<211> 24

<212> DNA

<213> Artificial Sequence

<220>

<223> primer

<400> 24
aatgatggag agtaggttga ggat 24

<210> 25

<211> 24

<212> DNA

<213> Artificial Sequence

<220>

<223> primer

<400> 25
tgcccacctt tgttgtctgc tacc 24

<210> 26

<211> 23

<212>  DNA

<213>  Artificial Sequence

<220>

<223>  primer

<400>  26
agcacctgtc cactgaagat ttc                                                23

<210>  27

<211>  21

<212>  DNA

<213>  Artificial Sequence

<220>

<223>  primer

<400>  27
tggacgctga gcttcagttc t                                                  21

<210>  28

<211>  24

<212>  DNA

<213>  Artificial Sequence

<220>

<223>  primer

<400>  28
cttctctgcg ctgcctcgat ggaa                                               24

<210>  29

<211>  21

<212>  DNA

<213>  Artificial Sequence

<220>

<223>  primer

<400>  29
agtaaaaagg gctcgcagga t                                                21

<210>  30

<211>  21

<212>  DNA

<213>  Artificial Sequence

<220>

<223>  primer

<400>  30
ggcagctggt gacatcagag a                                                21

<210>  31

<211>  25

<212>  DNA

<213>  Artificial Sequence

<220>

<223>  primer

<400>  31
aggaggccat gcaggcctac tctga                                            25

<210>  32

<211>  21

<212>  DNA

<213>  Artificial Sequence

<220>

<223>  primer

<400>  32
gagtccacgg tcagtccatg t                                                21

<210>  33

<211>  23

<212>  DNA

<213>  Artificial Sequence

<220>

<223>  primer

<400>  33
ttatgaacaa caatgccaag caa                                              23

<210>  34

<211>  34

<212>  DNA

<213>  Artificial Sequence

<220>

<223>  primer

<400>  34
agtccttagg tagtggctta gtccctggaa gctc                                  34

<210>  35

<211>  52

<212>  DNA

<213>  Artificial Sequence

<220>

<223>  probe

<400>  35
gtaatacgac tcactatagg gccctgacgg tccttcctga gggaatcttc ac              52

<210>  36

<211>  50

<212>  DNA

<213>  Artificial Sequence

<220>

<223>  probe

<400>  36
gtaatacgac tcactatagg gcctgggaag ttggtcatgg ccactccagc                    50

<210>  37

<211>  8

<212>  PRT

<213>  Artificial Sequence

<220>

<223>  FLAG tag

<400>  37

Asp Tyr Lys Asp Asp Asp Asp Lys
1               5

<210>  38

<211>  4

<212>  PRT

<213>  Artificial Sequence

<220>

<223>  motif

<400>  38

Tyr Gln Arg Leu
1

<210>  39

<211>  19

<212>  PRT

<213>  Artificial Sequence

<220>

<223>  antigen

<400>  39

Glu Gln Phe His Lys Tyr Leu Pro Trp Phe Leu Asn Asp Pro Pro Asn
1               5                   10                  15

Ile Arg Cys

<210>  40

<211>  16

<212>  PRT

<213>  Artificial Sequence

<220>

<223>  antigen

<400>  40

Glu Ala Phe Tyr Gln Arg Ser Phe Ala Glu Lys Ala Tyr Glu Ser Cys
1               5                   10                  15

<210>  41

<211>  15

<212>  PRT

<213>  Artificial Sequence

<220>

<223>  antigen

<400>  41

Gly Gln Thr Ser Leu Val Asp Trp Lys Asp His Phe Leu Tyr Cys
1               5                   10                  15

<210>  42

<211>  17

<212>  PRT

<213>  Artificial Sequence

<220>

<223>  antigen

<400>  42

Cys Ala Asn Ala Pro Leu Thr Phe Lys Asp Gly Thr Ala Leu Ala Leu
1               5                   10                  15

Ser

<210>  43

<211>  5092

<212>  DNA

<213>  Homo sapiens

<220>

<221>  CDS

<222>  (57)..(4136)

<223>

<400>  43
cttggctgtt cctgaggcct ggcctggctc cccgctgacc ccttcccaga cctggg atg      59
                                                              Met
                                                              1

gcg gag gcc ggc ctg agg ggc tgg ctg ctg tgg gcc ctg ctc ctg cgc      107
Ala Glu Ala Gly Leu Arg Gly Trp Leu Leu Trp Ala Leu Leu Leu Arg
            5                   10                  15

ttg gcc cag agt gag cct tac aca acc atc cac cag cct ggc tac tgc      155
Leu Ala Gln Ser Glu Pro Tyr Thr Thr Ile His Gln Pro Gly Tyr Cys
        20                  25                  30

gcc ttc tat gac gaa tgt ggg aag aac cca gag ctg tct gga agc ctc      203
Ala Phe Tyr Asp Glu Cys Gly Lys Asn Pro Glu Leu Ser Gly Ser Leu
    35                  40                  45

atg aca ctc tcc aac gtg tcc tgc ctg tcc aac acg ccg gcc cgc aag      251
Met Thr Leu Ser Asn Val Ser Cys Leu Ser Asn Thr Pro Ala Arg Lys
50                  55                  60                  65

atc aca ggt gat cac ctg atc cta tta cag aag atc tgc ccc cgc ctc      299
Ile Thr Gly Asp His Leu Ile Leu Leu Gln Lys Ile Cys Pro Arg Leu

                70                  75                  80

tac acc ggc ccc aac acc caa gcc tgc tgc tcc gcc aag cag ctg gta 347
Tyr Thr Gly Pro Asn Thr Gln Ala Cys Cys Ser Ala Lys Gln Leu Val
            85                  90                  95

tca ctg gaa gcg agt ctg tcg atc acc aag gcc ctc ctc acc cgc tgc 395
Ser Leu Glu Ala Ser Leu Ser Ile Thr Lys Ala Leu Leu Thr Arg Cys
        100                 105                 110

cca gcc tgc tct gac aat ttt gtg aac ctg cac tgc cac aac acg tgc 443
Pro Ala Cys Ser Asp Asn Phe Val Asn Leu His Cys His Asn Thr Cys
    115                 120                 125

agc ccc aat cag agc ctc ttc atc aat gtg acc cgc gtg gcc cag cta 491
Ser Pro Asn Gln Ser Leu Phe Ile Asn Val Thr Arg Val Ala Gln Leu
130                 135                 140                 145

ggg gct gga caa ctc cca gct gtg gtg gcc tat gag gcc ttc tac cag 539
Gly Ala Gly Gln Leu Pro Ala Val Val Ala Tyr Glu Ala Phe Tyr Gln
                150                 155                 160

cat agc ttt gcc gag cag agc tat gac tcc tgc agc cgt gtg cgc gtc 587
His Ser Phe Ala Glu Gln Ser Tyr Asp Ser Cys Ser Arg Val Arg Val
            165                 170                 175

cct gca gct gcc acg ctg gct gtg ggc acc atg tgt ggc gtg tat ggc 635
Pro Ala Ala Ala Thr Leu Ala Val Gly Thr Met Cys Gly Val Tyr Gly
        180                 185                 190

tct gcc ctt tgc aat gcc cag cgc tgg ctc aac ttc cag gga gac aca 683
Ser Ala Leu Cys Asn Ala Gln Arg Trp Leu Asn Phe Gln Gly Asp Thr
    195                 200                 205

ggc aat ggt ctg gcc cca ctg gac atc acc ttc cac ctc ttg gag cct 731
Gly Asn Gly Leu Ala Pro Leu Asp Ile Thr Phe His Leu Leu Glu Pro
210                 215                 220                 225

ggc cag gcc gtg ggg agt ggg att cag cct ctg aat gag ggg gtt gca 779
Gly Gln Ala Val Gly Ser Gly Ile Gln Pro Leu Asn Glu Gly Val Ala
                230                 235                 240

cgt tgc aat gag tcc caa ggt gac gac gtg gcg acc tgc tcc tgc caa 827
Arg Cys Asn Glu Ser Gln Gly Asp Asp Val Ala Thr Cys Ser Cys Gln
            245                 250                 255

gac tgt gct gca tcc tgt cct gcc ata gcc cgc ccc cag gcc ctc gac 875
Asp Cys Ala Ala Ser Cys Pro Ala Ile Ala Arg Pro Gln Ala Leu Asp
        260                 265                 270

tcc acc ttc tac ctg ggc cag atg ccg ggc agt ctg gtc ctc atc atc 923
Ser Thr Phe Tyr Leu Gly Gln Met Pro Gly Ser Leu Val Leu Ile Ile
    275                 280                 285

atc ctc tgc tct gtc ttc gct gtg gtc acc atc ctg ctt gtg gga ttc 971
Ile Leu Cys Ser Val Phe Ala Val Val Thr Ile Leu Leu Val Gly Phe
290                 295                 300                 305

cgt gtg gcc ccc gcc agg gac aaa agc aag atg gtg gac ccc aag aag 1019
Arg Val Ala Pro Ala Arg Asp Lys Ser Lys Met Val Asp Pro Lys Lys
                310                 315                 320

ggc acc agc ctc tct gac aag ctc agc ttc tcc acc cac acc ctc ctt 1067
Gly Thr Ser Leu Ser Asp Lys Leu Ser Phe Ser Thr His Thr Leu Leu
            325                 330                 335

ggc cag ttc ttc cag ggc tgg ggc acg tgg gtg gct tcg tgg cct ctg 1115
Gly Gln Phe Phe Gln Gly Trp Gly Thr Trp Val Ala Ser Trp Pro Leu
        340                 345                 350

acc atc ttg gtg cta tct gtc atc ccg gtg gtg gcc ttg gca gcg ggc 1163
Thr Ile Leu Val Leu Ser Val Ile Pro Val Val Ala Leu Ala Ala Gly
    355                 360                 365

ctg gtc ttt aca gaa ctc act acg gac ccc gtg gag ctg tgg tcg gcc 1211
Leu Val Phe Thr Glu Leu Thr Thr Asp Pro Val Glu Leu Trp Ser Ala
370                 375                 380                 385

ccc aac agc caa gcc cgg agt gag aaa gct ttc cat gac cag cat ttc 1259
Pro Asn Ser Gln Ala Arg Ser Glu Lys Ala Phe His Asp Gln His Phe
                390                 395                 400

ggc ccc ttc ttc cga acc aac cag gtg atc ctg acg gct cct aac cgg 1307
Gly Pro Phe Phe Arg Thr Asn Gln Val Ile Leu Thr Ala Pro Asn Arg
            405                 410                 415

tcc agc tac agg tat gac tct ctg ctg ctg ggg ccc aag aac ttc agc 1355
Ser Ser Tyr Arg Tyr Asp Ser Leu Leu Leu Gly Pro Lys Asn Phe Ser
        420                 425                 430

gga atc ctg gac ctg gac ttg ctg ctg gag ctg cta gag ctg cag gag 1403
Gly Ile Leu Asp Leu Asp Leu Leu Leu Glu Leu Leu Glu Leu Gln Glu
    435                 440                 445

agg ctg cgg cac ctc cag gta tgg tcg ccc gaa gca cag cgc aac atc 1451
Arg Leu Arg His Leu Gln Val Trp Ser Pro Glu Ala Gln Arg Asn Ile
450                 455                 460                 465

tcc ctg cag gac atc tgc tac gcc ccc ctc aat ccg gac aat acc agt 1499
Ser Leu Gln Asp Ile Cys Tyr Ala Pro Leu Asn Pro Asp Asn Thr Ser
                470                 475                 480

ctc tac gac tgc tgc atc aac agc ctc ctg cag tat ttc cag aac aac 1547
Leu Tyr Asp Cys Cys Ile Asn Ser Leu Leu Gln Tyr Phe Gln Asn Asn
            485                 490                 495

cgc acg ctc ctg ctg ctc aca gcc aac cag aca ctg atg ggg cag acc 1595
Arg Thr Leu Leu Leu Leu Thr Ala Asn Gln Thr Leu Met Gly Gln Thr
        500                 505                 510

tcc caa gtc gac tgg aag gac cat ttt ctg tac tgt gcc aat gcc ccg 1643
Ser Gln Val Asp Trp Lys Asp His Phe Leu Tyr Cys Ala Asn Ala Pro
    515                 520                 525

ctc acc ttc aag gat ggc aca gcc ctg gcc ctg agc tgc atg gct gac 1691
Leu Thr Phe Lys Asp Gly Thr Ala Leu Ala Leu Ser Cys Met Ala Asp
530                 535                 540                 545

tac ggg gcc cct gtc ttc ccc ttc ctt gcc att ggg ggg tac aaa gga 1739
Tyr Gly Ala Pro Val Phe Pro Phe Leu Ala Ile Gly Gly Tyr Lys Gly
                550                 555                 560

aag gac tat tct gag gca gag gcc ctg atc atg acg ttc tcc ctc aac 1787
Lys Asp Tyr Ser Glu Ala Glu Ala Leu Ile Met Thr Phe Ser Leu Asn
            565                 570                 575

aat tac cct gcc ggg gac ccc cgt ctg gcc cag gcc aag ctg tgg gag 1835
Asn Tyr Pro Ala Gly Asp Pro Arg Leu Ala Gln Ala Lys Leu Trp Glu
        580                 585                 590

gag gcc ttc tta gag gaa atg cga gcc ttc cag cgt cgg atg gct ggc 1883
Glu Ala Phe Leu Glu Glu Met Arg Ala Phe Gln Arg Arg Met Ala Gly
    595                 600                 605

atg ttc cag gtc acg ttc atg gct gag cgc tct ctg gaa gac gag atc 1931
Met Phe Gln Val Thr Phe Met Ala Glu Arg Ser Leu Glu Asp Glu Ile
610                 615                 620                 625

aat cgc acc aca gct gaa gac ctg ccc atc ttt gcc acc agc tac att 1979
Asn Arg Thr Thr Ala Glu Asp Leu Pro Ile Phe Ala Thr Ser Tyr Ile
                630                 635                 640

gtc ata ttc ctg tac atc tct ctg gcc ctg ggc agc tat tcc agc tgg 2027
Val Ile Phe Leu Tyr Ile Ser Leu Ala Leu Gly Ser Tyr Ser Ser Trp
            645                 650                 655

agc cga gtg atg gtg gac tcc aag gcc acg ctg ggc ctc ggc ggg gtg 2075
Ser Arg Val Met Val Asp Ser Lys Ala Thr Leu Gly Leu Gly Gly Val
        660                 665                 670

gcc gtg gtc ctg gga gca gtc atg gct gcc atg ggc ttc ttc tcc tac 2123
Ala Val Val Leu Gly Ala Val Met Ala Ala Met Gly Phe Phe Ser Tyr
    675                 680                 685

ttg ggt atc cgc tcc tcc ctg gtc atc ctg caa gtg gtt cct ttc ctg 2171
Leu Gly Ile Arg Ser Ser Leu Val Ile Leu Gln Val Val Pro Phe Leu
690                 695                 700                 705

gtg ctg tcc gtg ggg gct gat aac atc ttc atc ttt gtt ctc gag tac 2219
Val Leu Ser Val Gly Ala Asp Asn Ile Phe Ile Phe Val Leu Glu Tyr
                710                 715                 720

cag agg ctg ccc cgg agg cct ggg gag cca cga gag gtc cac att ggg 2267
Gln Arg Leu Pro Arg Arg Pro Gly Glu Pro Arg Glu Val His Ile Gly
            725                 730                 735

cga gcc cta ggc agg gtg gct ccc agc atg ctg ttg tgc agc ctc tct 2315
Arg Ala Leu Gly Arg Val Ala Pro Ser Met Leu Leu Cys Ser Leu Ser
        740                 745                 750

gag gcc atc tgc ttc ttc cta ggg gcc ctg acc ccc atg cca gct gtg 2363
Glu Ala Ile Cys Phe Phe Leu Gly Ala Leu Thr Pro Met Pro Ala Val
    755                 760                 765

cgg acc ttt gcc ctg acc tct ggc ctt gca gtg atc ctt gac ttc ctc 2411
Arg Thr Phe Ala Leu Thr Ser Gly Leu Ala Val Ile Leu Asp Phe Leu
770                 775                 780                 785

ctg cag atg tca gcc ttt gtg gcc ctg ctc tcc ctg gac agc aag agg 2459
Leu Gln Met Ser Ala Phe Val Ala Leu Leu Ser Leu Asp Ser Lys Arg
                790                 795                 800

cag gag gcc tcc cgg ttg gac gtc tgc tgc tgt gtc aag ccc cag gag 2507

Gln Glu Ala Ser Arg Leu Asp Val Cys Cys Cys Val Lys Pro Gln Glu
            805                 810                 815

ctg ccc ccg cct ggc cag gga gag ggg ctc ctg ctt ggc ttc ttc caa      2555
Leu Pro Pro Pro Gly Gln Gly Glu Gly Leu Leu Leu Gly Phe Phe Gln
        820                 825                 830

aag gct tat gcc ccc ttc ctg ctg cac tgg atc act cga ggt gtt gtg      2603
Lys Ala Tyr Ala Pro Phe Leu Leu His Trp Ile Thr Arg Gly Val Val
    835                 840                 845

ctg ctg ctg ttt ctc gcc ctg ttc gga gtg agc ctc tac tcc atg tgc      2651
Leu Leu Leu Phe Leu Ala Leu Phe Gly Val Ser Leu Tyr Ser Met Cys
850                 855                 860                 865

cac atc agc gtg gga ctg gac cag gag ctg gcc ctg ccc aag gac tcg      2699
His Ile Ser Val Gly Leu Asp Gln Glu Leu Ala Leu Pro Lys Asp Ser
                870                 875                 880

tac ctg ctt gac tat ttc ctc ttt ctg aac cgc tac ttc gag gtg ggg      2747
Tyr Leu Leu Asp Tyr Phe Leu Phe Leu Asn Arg Tyr Phe Glu Val Gly
            885                 890                 895

gcc ccg gtg tac ttt gtt acc acc ttg ggc tac aac ttc tcc agc gag      2795
Ala Pro Val Tyr Phe Val Thr Thr Leu Gly Tyr Asn Phe Ser Ser Glu
        900                 905                 910

gct ggg atg aat gcc atc tgc tcc agt gca ggc tgc aac aac ttc tcc      2843
Ala Gly Met Asn Ala Ile Cys Ser Ser Ala Gly Cys Asn Asn Phe Ser
    915                 920                 925

ttc acc cag aag atc cag tat gcc aca gag ttc cct gag cag tct tac      2891
Phe Thr Gln Lys Ile Gln Tyr Ala Thr Glu Phe Pro Glu Gln Ser Tyr
930                 935                 940                 945

ctg gcc atc cct gcc tcc tcc tgg gtg gat gac ttc att gac tgg ctg      2939
Leu Ala Ile Pro Ala Ser Ser Trp Val Asp Asp Phe Ile Asp Trp Leu
                950                 955                 960

acc ccg tcc tcc tgc tgc cgc ctt tat ata tct ggc ccc aat aag gac      2987
Thr Pro Ser Ser Cys Cys Arg Leu Tyr Ile Ser Gly Pro Asn Lys Asp
            965                 970                 975

aag ttc tgc ccc tcg acc gtc aac tct ctg aac tgc cta aag aac tgc      3035
Lys Phe Cys Pro Ser Thr Val Asn Ser Leu Asn Cys Leu Lys Asn Cys
        980                 985                 990

atg agc atc acg atg ggc tct  gtg agg ccc tcg gtg  gag cag ttc cat      3083
Met Ser Ile Thr Met Gly Ser  Val Arg Pro Ser Val  Glu Gln Phe His
    995                 1000                 1005

aag  tat ctt ccc tgg ttc  ctg aac gac cgg ccc  aac atc aaa tgt      3128
Lys  Tyr Leu Pro Trp Phe  Leu Asn Asp Arg Pro  Asn Ile Lys Cys
1010                 1015                 1020

ccc  aaa ggc ggc ctg gca  gca tac agc acc tct  gtg aac ttg act      3173
Pro  Lys Gly Gly Leu Ala  Ala Tyr Ser Thr Ser  Val Asn Leu Thr
1025                 1030                 1035

tca  gat ggc cag gtt tta  gac aca gtt gcc att  ctg tca ccc agg      3218
Ser  Asp Gly Gln Val Leu  Asp Thr Val Ala Ile  Leu Ser Pro Arg

1040                    1045                    1050

ctg gag tac agt ggc aca atc tcg gct cac tgc aac ctc tac ctc          3263
Leu Glu Tyr Ser Gly Thr Ile Ser Ala His Cys Asn Leu Tyr Leu
1055                    1060                    1065

ctg gat tca gcc tcc agg ttc atg gcc tat cac aag ccc ctg aaa          3308
Leu Asp Ser Ala Ser Arg Phe Met Ala Tyr His Lys Pro Leu Lys
1070                    1075                    1080

aac tca cag gat tac aca gaa gct ctg cgg gca gct cga gag ctg          3353
Asn Ser Gln Asp Tyr Thr Glu Ala Leu Arg Ala Ala Arg Glu Leu
1085                    1090                    1095

gca gcc aac atc act gct gac ctg cgg aaa gtg cct gga aca gac          3398
Ala Ala Asn Ile Thr Ala Asp Leu Arg Lys Val Pro Gly Thr Asp
1100                    1105                    1110

ccg gct ttt gag gtc ttc ccc tac acg atc acc aat gtg ttt tat          3443
Pro Ala Phe Glu Val Phe Pro Tyr Thr Ile Thr Asn Val Phe Tyr
1115                    1120                    1125

gag cag tac ctg acc atc ctc cct gag ggg ctc ttc atg ctc agc          3488
Glu Gln Tyr Leu Thr Ile Leu Pro Glu Gly Leu Phe Met Leu Ser
1130                    1135                    1140

ctc tgc ctt gtg ccc acc ttc gct gtc tcc tgc ctc ctg ctg ggc          3533
Leu Cys Leu Val Pro Thr Phe Ala Val Ser Cys Leu Leu Leu Gly
1145                    1150                    1155

ctg gac ctg cgc tcc ggc ctc ctc aac ctg ctc tcc att gtc atg          3578
Leu Asp Leu Arg Ser Gly Leu Leu Asn Leu Leu Ser Ile Val Met
1160                    1165                    1170

atc ctc gtg gac act gtc ggc ttc atg gcc ctg tgg ggc atc agt          3623
Ile Leu Val Asp Thr Val Gly Phe Met Ala Leu Trp Gly Ile Ser
1175                    1180                    1185

tac aat gct gtg tcc ctc atc aac ctg gtc tcg gcg gtg ggc atg          3668
Tyr Asn Ala Val Ser Leu Ile Asn Leu Val Ser Ala Val Gly Met
1190                    1195                    1200

tct gtg gag ttt gtg tcc cac att acc cgc tcc ttt gcc atc agc          3713
Ser Val Glu Phe Val Ser His Ile Thr Arg Ser Phe Ala Ile Ser
1205                    1210                    1215

acc aag ccc acc tgg ctg gag agg gcc aaa gag gcc acc atc tct          3758
Thr Lys Pro Thr Trp Leu Glu Arg Ala Lys Glu Ala Thr Ile Ser
1220                    1225                    1230

atg gga agt gcg gtg ttt gca ggt gtg gcc atg acc aac ctg cct          3803
Met Gly Ser Ala Val Phe Ala Gly Val Ala Met Thr Asn Leu Pro
1235                    1240                    1245

ggc atc ctt gtc ctg ggc ctc gcc aag gcc cag ctc att cag atc          3848
Gly Ile Leu Val Leu Gly Leu Ala Lys Ala Gln Leu Ile Gln Ile
1250                    1255                    1260

ttc ttc ttc cgc ctc aac ctc ctg atc act ctg ctg ggc ctg ctg          3893
Phe Phe Phe Arg Leu Asn Leu Leu Ile Thr Leu Leu Gly Leu Leu
1265                    1270                    1275

cat ggc ttg gtc ttc ctg ccc gtc atc ctc agc tac gtg ggg cct 3938
His Gly Leu Val Phe Leu Pro Val Ile Leu Ser Tyr Val Gly Pro
1280                1285                1290

gac gtt aac ccg gct ctg gca ctg gag cag aag cgg gct gag gag 3983
Asp Val Asn Pro Ala Leu Ala Leu Glu Gln Lys Arg Ala Glu Glu
1295                1300                1305

gcg gtg gca gca gtc atg gtg gcc tct tgc cca aat cac ccc tcc 4028
Ala Val Ala Ala Val Met Val Ala Ser Cys Pro Asn His Pro Ser
1310                1315                1320

cga gtc tcc aca gct gac aac atc tat gtc aac cac agc ttt gaa 4073
Arg Val Ser Thr Ala Asp Asn Ile Tyr Val Asn His Ser Phe Glu
1325                1330                1335

ggt tct atc aaa ggt gct ggt gcc atc agc aac ttc ttg ccc aac 4118
Gly Ser Ile Lys Gly Ala Gly Ala Ile Ser Asn Phe Leu Pro Asn
1340                1345                1350

aat ggg cgg cag ttc tga tacagccaga ggccctgtct aggctctatg 4166
Asn Gly Arg Gln Phe
1355

gccctgaacc aaagggttat ggggatcttc cttgtgactg ccccttgaca cacgccctcc 4226

tcaaatccta ggggaggcca ttcccatgag actgcctgtc actggaggat ggcctgctct 4286

tgaggtatcc aggcagcacc actgatggct cctctgctcc catagtgggt ccccagtttc 4346

caagtcacct aggccttggg cagtgcctcc tcctgggcct gggtctggaa gttggcagga 4406

acagacacac tccatgtttg tcccacactc actcactttc ctaggagccc acttctcatc 4466

caacttttcc cttctcagtt cctctctcga aagtcttaat tctgtgtcag taagtcttta 4526

acacgtagca gtgtccctga gaacacagac aatgaccact accctgggtg tgatatcaca 4586

ggaggccaga gagaggcaaa ggctcaggcc aagagccaac gctgtgggag gccggtcggc 4646

agccactccc tccagggcgc acctgcaggt ctgccatcca cggccttttc tggcaagaga 4706

agggcccagg aaggatgctc tcataaggcc caggaaggat gctctcataa gcaccttggt 4766

catggattag cccctcctgg aaaatggtgt tgggtttggt ctccagctcc aatacttatt 4826

aaggctgttg ctgccagtca aggccaccca ggagtctgaa ggctgggagc tcttggggct 4886

gggctggtcc tcccatcttc acctcgggcc tggatcccag gcctcaaacc agcccaaccc 4946

gagcttttgg acagctctcc agaagcatga actgcagtgg agatgaagat cctggctctg 5006

tgctgtgcac ataggtgttt aataaacatt tgttggcaga aaaaaaaaaa aaaaaaaaaa 5066

aaaaaaaaaa aaaaaaaaaa aaaaaa 5092

<210> 44

<211>  1359

<212>  PRT

<213>  Homo sapiens

<400>  44

Met Ala Glu Ala Gly Leu Arg Gly Trp Leu Leu Trp Ala Leu Leu Leu
1               5                   10                  15

Arg Leu Ala Gln Ser Glu Pro Tyr Thr Thr Ile His Gln Pro Gly Tyr
            20                  25                  30

Cys Ala Phe Tyr Asp Glu Cys Gly Lys Asn Pro Glu Leu Ser Gly Ser
        35                  40                  45

Leu Met Thr Leu Ser Asn Val Ser Cys Leu Ser Asn Thr Pro Ala Arg
    50                  55                  60

Lys Ile Thr Gly Asp His Leu Ile Leu Leu Gln Lys Ile Cys Pro Arg
65                  70                  75                  80

Leu Tyr Thr Gly Pro Asn Thr Gln Ala Cys Cys Ser Ala Lys Gln Leu
                85                  90                  95

Val Ser Leu Glu Ala Ser Leu Ser Ile Thr Lys Ala Leu Leu Thr Arg
            100                 105                 110

Cys Pro Ala Cys Ser Asp Asn Phe Val Asn Leu His Cys His Asn Thr
        115                 120                 125

Cys Ser Pro Asn Gln Ser Leu Phe Ile Asn Val Thr Arg Val Ala Gln
    130                 135                 140

Leu Gly Ala Gly Gln Leu Pro Ala Val Val Ala Tyr Glu Ala Phe Tyr
145                 150                 155                 160

Gln His Ser Phe Ala Glu Gln Ser Tyr Asp Ser Cys Ser Arg Val Arg
                165                 170                 175

Val Pro Ala Ala Ala Thr Leu Ala Val Gly Thr Met Cys Gly Val Tyr
            180                 185                 190

Gly Ser Ala Leu Cys Asn Ala Gln Arg Trp Leu Asn Phe Gln Gly Asp
        195                 200                 205

Thr Gly Asn Gly Leu Ala Pro Leu Asp Ile Thr Phe His Leu Leu Glu
    210                 215                 220

Pro Gly Gln Ala Val Gly Ser Gly Ile Gln Pro Leu Asn Glu Gly Val
225                 230                 235                 240

Ala Arg Cys Asn Glu Ser Gln Gly Asp Asp Val Ala Thr Cys Ser Cys
                245                 250                 255

Gln Asp Cys Ala Ala Ser Cys Pro Ala Ile Ala Arg Pro Gln Ala Leu
            260                 265                 270

Asp Ser Thr Phe Tyr Leu Gly Gln Met Pro Gly Ser Leu Val Leu Ile
        275                 280                 285

Ile Ile Leu Cys Ser Val Phe Ala Val Val Thr Ile Leu Leu Val Gly
    290                 295                 300

Phe Arg Val Ala Pro Ala Arg Asp Lys Ser Lys Met Val Asp Pro Lys
305                 310                 315                 320

Lys Gly Thr Ser Leu Ser Asp Lys Leu Ser Phe Ser Thr His Thr Leu
                325                 330                 335

Leu Gly Gln Phe Phe Gln Gly Trp Gly Thr Trp Val Ala Ser Trp Pro
            340                 345                 350

Leu Thr Ile Leu Val Leu Ser Val Ile Pro Val Val Ala Leu Ala Ala
        355                 360                 365

Gly Leu Val Phe Thr Glu Leu Thr Thr Asp Pro Val Glu Leu Trp Ser
    370                 375                 380

Ala Pro Asn Ser Gln Ala Arg Ser Glu Lys Ala Phe His Asp Gln His
385                 390                 395                 400

Phe Gly Pro Phe Phe Arg Thr Asn Gln Val Ile Leu Thr Ala Pro Asn
                405                 410                 415

Arg Ser Ser Tyr Arg Tyr Asp Ser Leu Leu Leu Gly Pro Lys Asn Phe
            420                 425                 430

Ser Gly Ile Leu Asp Leu Asp Leu Leu Leu Glu Leu Leu Glu Leu Gln
        435                 440                 445

Glu Arg Leu Arg His Leu Gln Val Trp Ser Pro Glu Ala Gln Arg Asn
    450                 455                 460

Ile Ser Leu Gln Asp Ile Cys Tyr Ala Pro Leu Asn Pro Asp Asn Thr
465                 470                 475                 480

Ser Leu Tyr Asp Cys Cys Ile Asn Ser Leu Leu Gln Tyr Phe Gln Asn
                485                 490                 495

Asn Arg Thr Leu Leu Leu Leu Thr Ala Asn Gln Thr Leu Met Gly Gln
            500                 505                 510

Thr Ser Gln Val Asp Trp Lys Asp His Phe Leu Tyr Cys Ala Asn Ala
        515                 520                 525

Pro Leu Thr Phe Lys Asp Gly Thr Ala Leu Ala Leu Ser Cys Met Ala
    530                 535                 540

Asp Tyr Gly Ala Pro Val Phe Pro Phe Leu Ala Ile Gly Gly Tyr Lys
545                 550                 555                 560

Gly Lys Asp Tyr Ser Glu Ala Glu Ala Leu Ile Met Thr Phe Ser Leu
                565                 570                 575

Asn Asn Tyr Pro Ala Gly Asp Pro Arg Leu Ala Gln Ala Lys Leu Trp
            580                 585                 590

Glu Glu Ala Phe Leu Glu Glu Met Arg Ala Phe Gln Arg Arg Met Ala
        595                 600                 605

Gly Met Phe Gln Val Thr Phe Met Ala Glu Arg Ser Leu Glu Asp Glu
    610                 615                 620

Ile Asn Arg Thr Thr Ala Glu Asp Leu Pro Ile Phe Ala Thr Ser Tyr
625                 630                 635                 640

Ile Val Ile Phe Leu Tyr Ile Ser Leu Ala Leu Gly Ser Tyr Ser Ser
                645                 650                 655

Trp Ser Arg Val Met Val Asp Ser Lys Ala Thr Leu Gly Leu Gly Gly
            660                 665                 670

Val Ala Val Val Leu Gly Ala Val Met Ala Ala Met Gly Phe Phe Ser
        675                 680                 685

Tyr Leu Gly Ile Arg Ser Ser Leu Val Ile Leu Gln Val Val Pro Phe

690                 695                 700

Leu Val Leu Ser Val Gly Ala Asp Asn Ile Phe Ile Phe Val Leu Glu
705                 710                 715                 720

Tyr Gln Arg Leu Pro Arg Arg Pro Gly Glu Pro Arg Glu Val His Ile
                725                 730                 735

Gly Arg Ala Leu Gly Arg Val Ala Pro Ser Met Leu Leu Cys Ser Leu
            740                 745                 750

Ser Glu Ala Ile Cys Phe Phe Leu Gly Ala Leu Thr Pro Met Pro Ala
        755                 760                 765

Val Arg Thr Phe Ala Leu Thr Ser Gly Leu Ala Val Ile Leu Asp Phe
    770                 775                 780

Leu Leu Gln Met Ser Ala Phe Val Ala Leu Leu Ser Leu Asp Ser Lys
785                 790                 795                 800

Arg Gln Glu Ala Ser Arg Leu Asp Val Cys Cys Cys Val Lys Pro Gln
                805                 810                 815

Glu Leu Pro Pro Pro Gly Gln Gly Glu Gly Leu Leu Leu Gly Phe Phe
            820                 825                 830

Gln Lys Ala Tyr Ala Pro Phe Leu Leu His Trp Ile Thr Arg Gly Val
        835                 840                 845

Val Leu Leu Leu Phe Leu Ala Leu Phe Gly Val Ser Leu Tyr Ser Met
    850                 855                 860

Cys His Ile Ser Val Gly Leu Asp Gln Glu Leu Ala Leu Pro Lys Asp
865                 870                 875                 880

Ser Tyr Leu Leu Asp Tyr Phe Leu Phe Leu Asn Arg Tyr Phe Glu Val
                885                 890                 895

Gly Ala Pro Val Tyr Phe Val Thr Thr Leu Gly Tyr Asn Phe Ser Ser
            900                 905                 910

Glu Ala Gly Met Asn Ala Ile Cys Ser Ser Ala Gly Cys Asn Asn Phe
        915                 920                 925

Ser Phe Thr Gln Lys Ile Gln Tyr Ala Thr Glu Phe Pro Glu Gln Ser
    930                 935                 940

Tyr Leu Ala Ile Pro Ala Ser Ser Trp Val Asp Asp Phe Ile Asp Trp
945                 950                 955                 960

Leu Thr Pro Ser Ser Cys Cys Arg Leu Tyr Ile Ser Gly Pro Asn Lys
                965                 970                 975

Asp Lys Phe Cys Pro Ser Thr Val Asn Ser Leu Asn Cys Leu Lys Asn
            980                 985                 990

Cys Met Ser Ile Thr Met Gly Ser Val Arg Pro Ser Val Glu Gln Phe
        995                 1000                1005

His Lys Tyr Leu Pro Trp Phe Leu Asn Asp Arg Pro Asn Ile Lys
    1010                1015                1020

Cys Pro Lys Gly Gly Leu Ala Ala Tyr Ser Thr Ser Val Asn Leu
    1025                1030                1035

Thr Ser Asp Gly Gln Val Leu Asp Thr Val Ala Ile Leu Ser Pro
    1040                1045                1050

Arg Leu Glu Tyr Ser Gly Thr Ile Ser Ala His Cys Asn Leu Tyr
    1055                1060                1065

Leu Leu Asp Ser Ala Ser Arg Phe Met Ala Tyr His Lys Pro Leu
    1070                1075                1080

Lys Asn Ser Gln Asp Tyr Thr Glu Ala Leu Arg Ala Ala Arg Glu
    1085                1090                1095

Leu Ala Ala Asn Ile Thr Ala Asp Leu Arg Lys Val Pro Gly Thr
    1100                1105                1110

Asp Pro Ala Phe Glu Val Phe Pro Tyr Thr Ile Thr Asn Val Phe
    1115                1120                1125

Tyr Glu Gln Tyr Leu Thr Ile Leu Pro Glu Gly Leu Phe Met Leu
    1130                1135                1140

Ser Leu Cys Leu Val Pro Thr Phe Ala Val Ser Cys Leu Leu Leu
    1145                1150                1155

Gly Leu Asp Leu Arg Ser Gly Leu Leu Asn Leu Leu Ser Ile Val
    1160                1165                1170

Met Ile Leu Val Asp Thr Val Gly Phe Met Ala Leu Trp Gly Ile
    1175                1180                1185

Ser Tyr Asn Ala Val Ser Leu Ile Asn Leu Val Ser Ala Val Gly
    1190                1195                1200

Met Ser Val Glu Phe Val Ser His Ile Thr Arg Ser Phe Ala Ile
    1205                1210                1215

Ser Thr Lys Pro Thr Trp Leu Glu Arg Ala Lys Glu Ala Thr Ile
    1220                1225                1230

Ser Met Gly Ser Ala Val Phe Ala Gly Val Ala Met Thr Asn Leu
    1235                1240                1245

Pro Gly Ile Leu Val Leu Gly Leu Ala Lys Ala Gln Leu Ile Gln
    1250                1255                1260

Ile Phe Phe Phe Arg Leu Asn Leu Leu Ile Thr Leu Leu Gly Leu
    1265                1270                1275

Leu His Gly Leu Val Phe Leu Pro Val Ile Leu Ser Tyr Val Gly
    1280                1285                1290

Pro Asp Val Asn Pro Ala Leu Ala Leu Glu Gln Lys Arg Ala Glu
    1295                1300                1305

Glu Ala Val Ala Ala Val Met Val Ala Ser Cys Pro Asn His Pro
    1310                1315                1320

Ser Arg Val Ser Thr Ala Asp Asn Ile Tyr Val Asn His Ser Phe
    1325                1330                1335

Glu Gly Ser Ile Lys Gly Ala Gly Ala Ile Ser Asn Phe Leu Pro
    1340                1345                1350

Asn Asn Gly Arg Gln Phe
    1355

<210> 45

<211> 4471

<212> DNA

<213> Mus musculus

<400> 45
ggatcacttc ctggctctgg gatggcagct gcctggcagg gatggctgct ctgggccctg 60
ctcctgaatt cggcccaggg tgagctctac acacccactc acaaagctgg cttctgcacc 120
ttttatgaag agtgtgggaa gaacccagag ctttctggag gcctcacatc actatccaat 180
atctcctgct tgtctaatac cccagccccg ccatgtcaca ggtgaccacc tggctcttct 240
ccagcgcgtc tgtccccgcc tatacaatgg ccccaatgac acctatgcct gttgctctac 300
caagcagctg gtgtcattag acagtagcct gtctatcacc aaggccctcc ttacacgctg 360
cccggcatgc tctgaaaatt ttgtgagcat acactgtcat aatacctgca gccctgacca 420
gagcctcttc atcaatgtta ctcgcgtggt tcagcgggac cctggacagc ttcctgctgt 480
ggtggcctat gaggcctttt atcaacgcag ttttgcagag aaggcctatg agtcctgtag 540
ccgggtgcgc atccctgcag ctgcctcgct ggctgtgggc agcatgtgtg gagtgtatgg 600
ctctgccctc tgcaatgctc agcgcctggc tcaacttcca aggagacaca gggaatggcc 660
tggctccgct ggacatcacc ttccacctct tggagcctgg ccaggccctg gcagatggga 720
tgaagccact ggatgggaag atcaaaccct gcaatgagtc ccagggtgaa gactcggcag 780
cctgttcctg ccaggactgt gcagcatcct gccctgtcat ccctccgccc ccggccctgc 840
gcccttcttt ctacatgggt cgaatgccag gctggctggc tctcatcatc atcttcactg 900
ctgtctttgt attgctctct gttgtccttg tgtatctccg agtggcttcc aacaggaaca 960
agaacaagac agcaggctcc caggaagccc ccaacctccc tcgtaagcgc agattctcac 1020
ctcacactgt ccttggccgg ttcttcgaga gctggggaac aatggtggcc tcatggccac 1080
tcactgtctt ggcactgtcc ttcatagttg tgatagcctt gtcagtaggc ctgaccttta 1140
tagaactcac cacagaccct gtggaactgt ggtcggcccc taaaagccaa gcccggaaag 1200
aaaaggcttt ccatgacgag cattttggcc ccttcttccg aaccaaccag atttttgtga 1260
cagctaagaa caggtccagc tacaagtacg actccctgct gctagggccc aagaacttca 1320
gtgggatcct atccctggac ttgctgcagg agctgttgga gctacaggag agacttcgac 1380
acctgcaagt gtggtcccat gaggcacagc gcaacatctc cctccaggac atctgctatg 1440
ctcccctcaa accgcataac accagcctca ctgactgctg tgtcaacagc ctccttcaat 1500
acttccagaa caaccacaca ctcctgctgc tcacagccaa ccagactctg aatggccaga 1560
cctccctggt ggactggaag gaccatttcc tctactgtgc caatgcccct ctcacgtaca 1620
aagatggcac agccctggcc ctgagctgca tagctgacta cggggcgcct gtcttcccct 1680
tccttgctgt tgggggctac caagggacgg actactcgga ggcagaagcc ctgatcataa 1740

ccttctctat caataactac cccgctgatg atccccgcat ggcccacgcc aagctctggg 1800

aggaggcttt cttgaaggaa atgcaatcct tccagagaag cacagctgac aagttccaga 1860

ttgcgttctc agctgagcgt tctctggagg acgagatcaa tcgcactacc atccaggacc 1920

tgcctgtctt tgccatcagc taccttatcg tcttcctgta catctccctg gccctgggca 1980

gctactccag atggagccga gttgcggtgg attccaaggc tactctgggc ctaggtgggg 2040

tggctgttgt gctgggagca gtcgtggctg ccatgggctt ctactcctac ctgggtgtcc 2100

cctcctctct ggtcatcatt caagtggtac ctttcctggt gctggctgtg ggagctgaca 2160

acatcttcat ctttgttctt gagtaccaga ggctgcctag gatgcccggg gagcagcgag 2220

aggctcacat tggccgcacc ctgggtagtg tggcccccag catgctgctg tgcagcctct 2280

ctgaggccat ctgcttcttt ctaggggccc tgacctccat gccagctgtg aggacctttg 2340

ccttgacctc tggcttagca atcatctttg acttcctgct ccagatgaca gcctttgtgg 2400

ccctgctctc cctggatagc aagaggcagg aggcctctcg ccccgacgtc gtgtgctgct 2460

tttcaagccg aaatctgccc ccaccgaaac aaaaagaagg cctcttactt tgcttcttcc 2520

gcaagatata cactcccttc ctgctgcaca gattcatccg ccctgttgtg ctgctgctct 2580

ttctggtcct gtttggagca aacctctact taatgtgcaa catcagcgtg gggctggacc 2640

aggatctggc tctgcccaag gattcctacc tgatagacta cttcctcttt ctgaaccggt 2700

acttggaagt ggggcctcca gtgtactttg acaccacctc aggctacaac ttttccaccg 2760

aggcaggcat gaacgccatt tgctctagtg caggctgtga gagcttctcc ctaacccaga 2820

aaatccagta tgccagtgaa ttccctaatc agtcttatgt ggctattgct gcatcctcct 2880

gggtagatga cttcatcgac tggctgaccc catcctcctc ctgctgccgc atttataccc 2940

gtggccccca taaagatgag ttctgtccct caacggatac ttccttcaac tgtctcaaaa 3000

actgcatgaa ccgcactctg ggtcccgtga gacccacaac agaacagttt cataagtacc 3060

tgccctggtt cctgaatgat acgcccaaca tcagatgtct taaagggggc ctagcagcgt 3120

atagaacctc tgtgaatttg atctcagatg gccagattat agcctcccag ttcatggcct 3180

accacaagcc cttacggaac tcacaggact ttacagaagc tctccgggca tcccggttgc 3240

tagcagccaa catcacagct gaactacgga aggtgcctgg gacagatccc aactttgagg 3300

tcttccctta cacgatctcc aatgtgttct accagcaata cctgacggtt ctccctgagg 3360

gaatcttcac tcttgctctc tgcttcgtgc ccacctttgt ggtctgctac ctcctactgg 3420

gcctggacat acgctcaggc atcctcaacc tgctctccat cattatgatc ctcgtggaca 3480

ccatcggcct catggctgtg tggggtatca gctacaatgc tgtgtccctc atcaaccttg 3540

tcacggcagt gggcatgtct gtggagttcg tgtcccacat tacccggtcc tttgctgtaa 3600

gcaccaagcc tacccggctg gagagagcca aagatgctac tatcttcatg ggcagtgcgg 3660
tgtttgctgg agtggccatg accaacttcc cgggcatcct catcctgggc tttgctcagg 3720
cccagcttat ccagattttc ttcttccgcc tcaacctcct gatcaccttg ctgggtctgc 3780
tacacggcct ggtcttcctg cccgttgtcc tcagctatct ggggccagat gttaaccaag 3840
ctctggtact ggaggagaaa ctagccactg aggcagccat ggtctcagag ccttcttgcc 3900
cacagtaccc cttcccggct gatgcaaaca ccagtgacct atgttaacta aggctttaat 3960
ccagaattta tccctgaaat taatgctgct agcagctctc tgcccaaaag tgaccaaaag 4020
ttctaatgga gtaggagctt gtccaggctc catggttctt gctgataagg ggccacgagg 4080
gtcttccctc tggttgtttc caaggcctgg ggaaagttgt tccagaaaaa aattgctggc 4140
attcttgtcc tgaggcagcc agcactggcc actttgttgt cataggtccc cgaggccatg 4200
atcagattac ctcctctgta aagagaatat cttgagtatt gtatgggatg tatcacatgt 4260
caattaaaaa ggccatggcc tatggcttag gcaggaaata gggtgtggaa catccaggag 4320
aagaaaggat tctgggataa aggacacttg ggaacgtgtg gcagtggtac ctgagcacag 4380
gtaattagcc atgtggcgaa atgtagatta atataaatgc atatctaagt tatgattcta 4440
gtctagctat atggccaagg tatttataaa t 4471

<210> 46

<211> 25

<212> DNA

<213> Artificial sequence

<220>

<223> primer

<400> 46
atgttaggtg agtctgaacc taccc 25

<210> 47

<211> 25

<212> DNA

<213> Artificial sequence

<220>

<223> primer

<400> 47
ggattgcatt tccttcaaga aagcc 25

<210> 48

<211> 25

<212> DNA

<213> Artificial sequence

<220>

<223> primer

<400> 48
tatggctctg ccctctgcaa tgctc 25

<210> 49

<211> 28

<212> DNA

<213> Artificial sequence

<220>

<223> primer

<400> 49
tcagcagcct ctgttccaca tacacttc 28

<210> 50

<211> 25

<212> DNA

<213> Artificial sequence

<220>

<223> primer

<400> 50
gttccacagg gtctgtggtg agttc 25

<210> 51

<211> 3996

<212> DNA

<213> Homo sapiens

<220>

<221> misc_feature

<222> (1)..(3996)

<223> n is g or a or t or c

<400> 51
atggcngarg cnggnytnmg nggntggytn ytntgggcny tnytnytnmg nytngcncar 60

wsngarccnt ayacnacnat hcaycarccn ggntaytgyg cnttytayga ygartgyggn 120

aaraayccng arytnwsngg nwsnytnatg acnytnwsna aygtnwsntg yytnwsnaay 180

acnccngcnm gnaarathac nggngaycay ytnathytny tncaraarat htgyccnmgn 240

ytntayacng gnccnaayac ncargcntgy tgywsngcna arcarytngt nwsnytngar 300

gcnwsnytnw snathacnaa rgcnytnytn acnmgntgyc cngcntgyws ngayaaytty 360

gtnaayytnc aytgycayaa yacntgywsn ccnaaycarw snytnttyat haaygtnacn 420

mgngtngcnc arytnggngc nggncarytn ccngcngtng tngcntayga rgcnttytay 480

carcaywsnt tygcngarca rwsntaygay wsntgywsnm gngtnmgngt nccngcngcn 540

gcnacnytng cngtnggnac natgtgyggn gtntayggnw sngcnytntg yaaygcncar 600

mgntggytna ayttycargg ngayacnggn aayggnytng cnccnytnga yathacntty 660

cayytnytng arccnggnca rgcngtnggn wsnggnathc arccnytnaa ygarggngtn 720

gcnmgntgya aygarwsnca rggngaygay gtngcnacnt gywsntgyca rgaytgygcn 780

gcnwsntgyc cngcnathgc nmgnccncar gcnytngayw snacnttyta yytnggncar 840

atgccnggnw snytngtnyt nathathath ytntgywsng tnttygcngt ngtnacnath 900

ytnytngtng gnttymgngt ngcnccngcn mgngayaarw snaaratggt ngayccnaar 960

aarggnacnw snytnwsnga yaarytnwsn ttywsnacnc ayacnytnyt nggncartty 1020

ttycarggnt ggggnacntg ggtngcnwsn tggccnytna cnathytngt nytnwsngtn 1080

athccngtng tngcnytngc ngcnggnytn gtnttyacng arytnacnac ngayccngtn 1140

garytntggw sngcnccnaa ywsncargcn mgnwsngara argcnttyca ygaycarcay 1200

ttyggnccnt tyttymgnac naaycargtn athytnacng cnccnaaymg nwsnwsntay 1260

mgntaygayw snytnytnyt nggnccnaar aayttywsng gnathytnga yytngayytn 1320

ytnytngary tnytngaryt ncargarmgn ytnmgncayy tncargtntg gwsnccngar 1380

gcncarmgna ayathwsnyt ncargayath tgytaygcnc cnytnaaycc ngayaayacn 1440

wsnytntayg aytgytgyat haaywsnytn ytncartayt tycaraayaa ymgnacnytn 1500

ytnytnytna cngcnaayca racnytnatg ggncaracnw sncargtnga ytggaargay 1560

cayttyytnt aytgygcnaa ygcnccnytn acnttyaarg ayggnacngc nytngcnytn 1620

wsntgyatgg cngaytaygg ngcnccngtn ttyccnttyy tngcnathgg nggntayaar 1680

ggnaargayt aywsngargc ngargcnytn athatgacnt tywsnytnaa yaaytayccn 1740

gcnggngayc cnmgnytngc ncargcnaar ytntgggarg argcnttyyt ngargaratg 1800

mgngcnttyc armgnmgnat ggcnggnatg ttycargtna cnttyacngc ngarmgnwsn 1860

ytngargayg arathaaymg nacnacngcn gargayytnc cnathttygc nacnwsntay 1920

athgtnatht tyytntayat hwsnytngcn ytnggnwsnt aywsnwsntg gwsnmgngtn 1980

atggtngayw snaargcnac nytnggnytn ggnggngtng cngtngtnyt nggngcngtn 2040

atggcngcna tgggnttytt ywsntayytn ggnathmgnw snwsnytngt nathytncar 2100

gtngtnccnt tyytngtnyt nwsngtnggn gcngayaaya thttyathtt ygtnytngar 2160

taycarmgny tnccnmgnmg nccnggngar ccnmgngarg tncayathgg nmgngcnytn 2220

ggnmgngtng cnccnwsnat gytnytntgy wsnytnwsng argcnathtg yttyttyytn 2280

ggngcnytna cnccnatgcc ngcngtnmgn acnttygcny tnacnwsngg nytngcngtn 2340

athytngayt tyytnytnca ratgwsngcn ttygtngcny tnytnwsnyt ngaywsnaar 2400

mgncargarg cnwsnmgnyt ngaygtntgy tgytgygtna arccncarga rytnccnccn 2460

ccnggncarg gngarggnyt nytnytnggn ttyttycara argcntaygc nccnttyytn 2520

ytncaytgga thacnmgngg ngtngtnytn ytnytnttyy tngcnytntt yggngtnwsn 2580

ytntaywsna tgtgycayat hwsngtnggn ytngaycarg arytngcnyt nccnaargay 2640

wsntayytny tngaytaytt yytnttyytn aaymgntayt tygargtngg ngcnccngtn 2700

tayttygtna cnacnytngg ntayaaytty wsnwsngarg cnggnatgaa ygcnathtgy 2760

wsnwsngcng gntgyaayaa yttywsntty acncaraara thcartaygc nacngartty 2820

ccngarcarw sntayytngc nathccngcn wsnwsntggg tngaygaytt yathgaytgg 2880

ytnacnccnw snwsntgytg ymgnytntay athwsnggnc cnaayaarga yaarttytgy 2940

ccnwsnacng tnaaywsnyt naaytgyytn aaraaytgya tgwsnathac natgggnwsn 3000

gtnmgnccnw sngtngarca rttycayaar tayytnccnt ggttyytnaa ygaymgnccn 3060
aayathaart gyccnaargg nggnytngcn gcntaywsna cnwsngtnaa yytnacnwsn 3120
gayggncarg tnytngcnws nmgnttyatg gcntaycaya arccnytnaa raaywsncar 3180
gaytayacng argcnytnmg ngcngcnmgn garytngcng cnaayathac ngcngayytn 3240
mgnaargtnc cnggnacnga yccngcntty gargtnttyc cntayacnat hacnaaygtn 3300
ttytaygarc artayytnac nathytnccn garggnytnt tyatgytnws nytntgyytn 3360
gtnccnacnt tygcngtnws ntgyytnytn ytnggnytng ayytnmgnws nggnytnytn 3420
aayytnytnw snathgtnat gathytngtn gayacngtng gnttyatggc nytntgggay 3480
athwsntaya aygcngtnws nytnathaay ytngtnwsng cngtnggnat gwsngtngar 3540
ttygtnwsnc ayathacnmg nwsnttygcn athwsnacna arccnacntg gytngarmgn 3600
gcnaargarg cnacnathws natgggnwsn gcngtnttyg cnggngtngc natgacnaay 3660
ytnccnggna thytngtnyt nggnytngcn aargcncary tnathcarat httyttytty 3720
mgnytnaayy tnytnathac nytnytnggn ytnytncayg gnytngtntt yytnccngtn 3780
athytnwsnt aygtnggncc ngaygtnaay ccngcnytng cnytngarca raarmgngcn 3840
gargargcng tngcngcngt natggtngcn wsntgyccna aycayccnws nmgngtnwsn 3900
acngcngaya ayathtaygt naaycaywsn ttygarggnw snathaargg ngcnggngcn 3960
athwsnaayt tyytnccnaa yaayggnmgn cartty 3996

## Claims

**1.** A kit comprising:
(a) ezetimibe in a pharmaceutical dosage form; and
(b) information indicating that NPC1L1 is a target of ezetimibe.

**2.** The kit of claim 1 wherein the dosage form is a tablet comprising 10 mg ezetimibe.

**3.** The kit of claim 1 or 2 further comprising simvastatin in a pharmaceutical dosage form.

**4.** The kit of one of claims 1 to 3 wherein the simvastatin in pharmaceutical dosage form comprises 5 mg, 10 mg, 20 mg, 40 mg or 80mg simvastatin.

**5.** The kit of one of claims 1 to 4 wherein the simvastatin in pharmaceutical dosage form and the ezetimibe in pharmaceutical dosage form are associated in a single pill or tablet.

**6.** A method for inhibiting NPC1L1 mediated sterol or 5α-stanol uptake, in a subject, by administering, to the subject, a substance identified by a method for identifying an antagonist of NPC1L1 comprising the steps of:
(a) contacting a host cell expressing a polypeptide comprising an amino acid sequence selected from SEQ ID NOs: 2, 4 and 12 or a functional fragment thereof on a cell surface, in the presence of a known amount of detectably labeled ezetimibe, with a sample to be tested for the presence of the antagonist; and
(b) measuring the amount of detectably labeled ezetimibe specifically bound, directly or indirectly, to the polypeptide;
wherein an NPC1L1 antagonist in the sample is identified by measuring substantially reduced direct or indirect binding of the detectably labeled ezetimibe to the polypeptide, compared to what would be measured in the absence of such an antagonist.

**7.** A method for inhibiting NPC1L1 mediated sterol or 5α-stanol uptake, in a subject, by administering, to the subject, a substance identified by a method for identifying an antagonist of NPC1L1 comprising the steps of:
(a) placing, in an aqueous suspension, a plurality of support particles, impregnated with a fluorescer, to which a host cell expressing a polypeptide comprising an amino acid sequence selected from SEQ ID NOs: 2, 4 and 12 or a functional fragment thereof on a cell surface are attached;
(b) adding, to the suspension, radiolabeled ezetimibe and a sample to be tested for the presence of the antagonist, wherein the radiolabel emits radiation energy capable of activating the fluorescer upon direct or indirect binding of the ezetimibe to the polypeptide to produce light energy, whereas radiolabeled ezetimibe that does not directly or indirectly bind to the polypeptide is, generally, too far removed from the support particles to enable the radioactive energy to activate the fluorescer; and
(c) measuring the light energy emitted by the fluorescer in the suspension; wherein an NPC1L1 antagonist in the sample is identified by measuring substantially reduced light energy emission, compared to what would be measured in the absence of such an antagonist.

**8.** A method for identifying an antagonist of NPC1L1 comprising:
(a) contacting a host cell expressing a polypeptide comprising an amino acid sequence selected from SEQ ID NOs: 2, 4 and 12 or a functional fragment thereof on a cell surface, in the presence of a known amount of a detectably labeled substituted azetidinone, with a sample to be tested for the presence of the antagonist; and
(b) measuring the amount of detectably labeled substituted azetidinone specifically bound, directly or indirectly, to the polypeptide;
wherein an NPC1L1 antagonist in the sample is identified by measuring substantially reduced direct or indirect binding of the detectably labeled substituted azetidinone to the polypeptide, compared to what would be measured in the absence of such an antagonist.

**8.** A kit comprising:
(a) a substituted azetidinone in a pharmaceutical dosage form; and
(b) information indicating that NPC1L1 is a target of the substituted azetidinone.

**9.** A method for identifying an antagonist of NPC1L1 comprising:
(a) contacting a host cell expressing a polypeptide comprising an amino acid sequence selected from SEQ ID NOs: 2, 4 and 12 or a functional fragment thereof on a cell surface, in the presence of a known amount of detectably labeled ezetimibe, with a sample to be tested for the presence of the antagonist; and
(b) measuring the amount of detectably labeled ezetimibe specifically bound, directly or indirectly, to the polypeptide;
wherein an NPC1L1 antagonist in the sample is identified by measuring substantially reduced direct or indirect binding of the detectably labeled ezetimibe to the polypeptide, compared to what would be measured in the absence of such an antagonist.

**10.** A method for identifying an antagonist of NPC1L1 comprising:
(a) placing, in an aqueous suspension, a plurality of support particles, impregnated with a fluorescer, to which a host cell expressing a polypeptide comprising an amino acid sequence selected from SEQ ID NOs: 2, 4 and 12 or a functional fragment thereof on a cell surface are attached;
(b) adding, to the suspension, radiolabeled ezetimibe and a sample to be tested for the presence of the antagonist, wherein the radiolabel emits radiation energy capable of activating the fluorescer upon direct or indirect binding of the ezetimibe to the polypeptide to produce light energy, whereas radiolabeled ezetimibe that does not directly or indirectly bind to the polypeptide is, generally, too far removed from the support particles to enable the radioactive energy to activate the fluorescer; and
(c) measuring the light energy emitted by the fluorescer in the suspension;
wherein an NPC1L1 antagonist in the sample is identified by measuring substantially reduced light energy emission, compared to what would be measured in the absence of such an antagonist.

**11.** The method of claim 10 wherein the fluorescer is selected from yttrium silicate, yttrium oxide, diphenyloxazole and polyvinyltoluene.

**12.** A method of claim 9 wherein the ezetimibe is labeled with a radiolabel selected from ³H and ¹²⁵I.

**13.** A method of claim 10 wherein the ezetimibe is labeled with a radiolabel selected from ³H and ¹²⁵I.
